# EUROPÄISCHE PATENTSCHRIFT

(11) **EP 0 719 259 B1**
(45) Veröffentlichungstag und Bekanntmachung des Hinweises auf die Patenterteilung: **20.01.1999**
(21) Anmeldenummer: 94927552.3
(22) Anmeldetag: 02.09.1994
(51) Int. Cl.: C07D 239/52, C07D 239/42, C07D 239/46, C07D 239/34, C07D 239/88, C07D 239/94, A01N 43/54

(54) **SUBSTITUIERTE PYRIDINE UND PYRIMIDINE, VERFAHREN ZU IHRER HERSTELLUNG UND IHRE VERWENDUNG ALS SCHÄDLINGSBEKÄMPFUNGSMITTEL UND FUNGIZIDE**
SUBSTITUTED PYRIDINES AND PYRIMIDINES, PROCESS FOR PRODUCING THEM AND THEIR USE AS PESTICIDES AND FUNGICIDES
PYRIDINES ET PYRIMIDINES SUBSTITUEES, LEURS PROCEDES DE PREPARATION ET LEUR UTILISATION COMME AGENTS DE LUTTE CONTRE LES PARASITES ET COMME FONGICIDES

(30) Priorität: 14.09.1993 DE 4331178
(43) Veröffentlichungstag der Anmeldung: 03.07.1996
(73) Patentinhaber: Hoechst Schering AgrEvo GmbH, 13509 Berlin (DE)
(72) Erfinder: REUSCHLING, Dieter, Bernd, D-35510 Butzbach (DE); LINKIES, Adolf, Heinz, D-65929 Frankfurt am Main (DE); WEHNER, Volkmar, D-97657 Sandberg (DE); PREUSS, Rainer, D-65719 Hofheim am Taunus (DE); SCHAPER, Wolfgang, D-86420 Diedorf (DE); JAKOBI, Harald, D-60598 Frankfurt am Main (DE); BRAUN, Peter, D-55268 Nieder-Olm (DE); KNAUF, Werner, D-65817 Eppstein (DE); SACHSE, Burkhard, D-65779 Kelkheim (DE); WALTERSDORFER, Anna, D-60529 Frankfurt am Main (DE); KERN, Manfred, D-55296 Lörzweiler (DE); LÜMMEN, Peter, D-65527 Niedernhausen (DE); BONIN, Werner, D-65779 Kelkheim (DE)
(86) Internationale Anmeldenummer: EP9402934
(87) Internationale Veröffentlichungsnummer: WO9507894

(56) Entgegenhaltungen:
- EP-A- 0 452 002
- WO-A-93/19050

## Beschreibung

Die Erfindung betrifft neue substituierte Pyridine und Pyrimidine und davon abgeleitete kondensierte Systeme, Verfahren zu ihrer Herstellung und ihre Verwendung als Schädlingsbekämpfungsmittel, insbesondere als Insektizide, Akarizide und Fungizide. Die Verbindungen eignen sich weiterhin zur Bekämpfung von Nematoden, Helminthen und Mollusken, sowie zur Bekämpfung von Endo- und Ektoparasiten auf dem veterinärmedizinischen Gebiet.

Es ist bereits bekannt, daß bestimmte 4-Alkoxy- und 4-Aminopyrimidine gute fungizide, akarizide und insektizide Wirkung zeigen (vgl. EP-A-326 328, EP-A-414 368, EP-A-424 125, EP-A-453 137 und EP-A-467 760). Die biologische Wirkung dieser Verbindungen ist jedoch insbesondere bei niedrigen Aufwandmengen und Konzentrationen nicht in allen Anwendungsbereichen zufriedenstellend.

Es wurden neue substituierte Pyridine und Pyrimidine und davon abgeleitete kondensierte Systeme der allgemeinen Formel I gefunden, 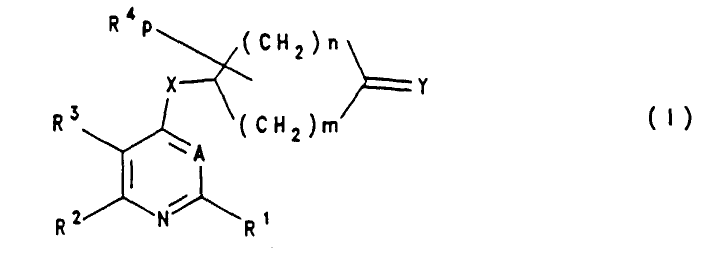 in welcher die Reste und Variablen wie unten definiert sind.

Die Erfindung betrifft daher Verbindungen der Formel I und deren Säureadditionssalze, worin
- A: die Bedeutung N oder CH hat,
- R¹: Wasserstoff, Halogen, (C₁-C₄)-Alkyl oder (C₃-C₆)-Cycloalkyl bedeutet,
- R²: Wasserstoff, Halogen, (C₁-C₄)-Alkyl, (C₁-C₄)-Halogenalkyl, (C₁-C₄)-Alkoxy, (C₁-C₄)-Halogenalkoxy, (C₁-C₄)-Alkoxy-(C₁-C₄)-alkyl, (C₁-C₄)-Halogenalkoxy-(C₁-C₄)-halogenalkyl, (C₁-C₄)-Alkylthio, (C₁-C₄)-Alkylthio-(C₁-C₄)-alkyl, (C₁-C₄)-Alkylamino oder Di-(C₁-C₄)-alkylamino bedeutet,
- R³: Wasserstoff, Halogen, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, Amino, (C₁-C₄)-Alkylamino oder Di-(C₁-C₄)-alkylamino bedeutet oder
- R² und R³: zusammen mit den Kohlenstoffatomen, an die sie gebunden sind, einen ungesättigten 5- oder 6-gliedrigen isocyclischen Ring bilden, der auch ein oder mehrere, vorzugsweise ein oder zwei Stickstoffatome enthalten kann und der, falls es sich um einen 5-Ring handelt, an Stelle von CH₂ ein Sauerstoff- oder Schwefelatom enthalten kann und der gegebenenfalls durch 1, 2 oder 3 gleiche oder verschiedene Reste substituiert ist und diese Reste (C₁-C₄)-Alkyl, (C₁-C₄)-Alkoxy, (C₁-C₄)-Halogenalkyl, (C₁-C₄)-Halogenalkoxy und/oder Halogen bedeuten,
oder
- R² und R³: zusammen mit den Kohlenstoffatomen, an die sie gebunden sind, einen gesättigten 5-, 6- oder 7-gliedrigen isocyclischen Ring bilden, der an Stelle von CH₂ ein oder mehrere Sauerstoff- oder Schwefelatome enthalten kann und der gegebenenfalls durch ein bis vier gleiche oder verschiedene Reste aus der Reihe (C₁-C₄)-Alkyl und Halogen substituiert ist,
- X: Sauerstoff, NH und S(O)_{q} bedeutet, wobei q 0, 1 oder 2 sein kann,
- R⁴: Halogen, (C₁-C₄)-Alkyl, (C₂-C₇)-Cyoloalkyl, (C₁-C₄)-Halogenalkyl, (C₁-C₄)-Alkoxy, (C₁-C₄)-Halogenalkoxy oder gegebenenfalls mit einem, zwei oder drei gleichen oder verschiedenen Substituenten aus der Reihe Halogen, (C₁-C₄)-Alkyl, (C₁-C₄)-Alkoxy, (C₁-C₄)-Halogenalkyl und (C₁-C₄)-Halogenalkoxy substituiertes Phenyl bedeutet;
- p: eine ganze Zahl von 0 bis 4,
- n: eine ganze Zahl von 0 bis 2 und
- m: eine ganze Zahl von 1 bis 3 sein kann,
- Y: die Bedeutungen 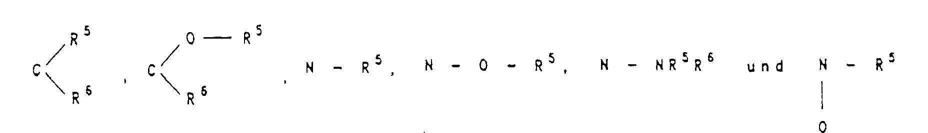 haben kann,
- R⁵: Wasserstoff, Halogen, (C₁-C₁₂)-Alkyl, (C₃-C₈)-Cycloalkyl, (C₃-C₈)-Cycloalkyl-(C₁-C₄)-alkyl, [(C₁-C₄)-Alkoxy]ₜ-(C₁-C₄)-alkyl, wobei t eine ganze Zahl von 1 bis 3 sein kann. (C₁-C₁₂)-Halogenalkyl, 2-(Tetrahydro-2H-pyran-2-yloxy)-(C₁-C₄)-alkyl, [(C₁-C₄)Halogenalkoxy]ₜ-(C₁-C₄)-halogenalkyl, [(C₁-C₄)Halogenalkoxy]ₜ-(C₁-C₄)-alkenyl, (C₁-C₈)-Alkylcarbonyl, (C₁-C₈)-Alkoxycarbonyl, (C₁-C₈)-Alkylcarbonyl-(C₁-C₄)-alkyl, (C₁-C₈)-Halogenalkylcarbonyl, (C₁-C₈)-Halogenalkoxycarbonyl, (C₃-C₈)-Cycloalkylcarbonyl, (C₃-C₈)-Cycloalkoxycarbonyl, gegebenenfalls substituiertes Benzoyl, gegebenenfalls substituiertes Benzyloxycarbonyl, (C₂-C₈)-Alkenyl, (C₂-C₈)-Alkinyl, Tri-(C₁-C₈)-alkylsilyl, vorzugsweise Dimethyl-(C₁-C₈)alkylsilyl oder Triethylsilyl, Di-(C₁-C₈)-alkyl-(C₃-C₈)-cycloalkylsilyl, vorzugsweise Dimethylcyclohexylsilyl, Di-(C₁-C₈)-alkyl-(phenyl-(C₁-C₄)-alkyl)-silyl, vorzugsweise Dimethyl-(phenyl-(C₁-C₄)-alkyl)-silyl, Di-(C₁-C₈)-alkyl-(C₁-C₄)-halogenalkylsilyl, Dimethylphenylsilyl, Heteroaryl, Phenyl, Phenyl-(C₂-C₄)-alkyl, Benzyl, Benzyloxy-(C₁-C₄)-alkyl bedeutet, wobei Phenyl oder Heteroaryl in den sechs letztgenannten Resten unsubstituiert oder ein oder mehrfach, vorzugsweise bis zu dreifach, substituiert sein können und diese Substituenten gleich oder verschieden sind und jeweils Halogen,(C₁-C₈)-Alkyl, (C₃-C₈)-Cycloalkyl, (C₁-C₄)-Halogenalkyl, (C₁-C₄)-Dialkylamino, (C₁-C₄)-Alkylthio, (C₁-C₈)-Alkoxy, (C₁-C₈)-Alkoxycarbonyl, (C₁-C₈)-Alkanoyloxy, (C₁-C₄)-Halogenalkoxy, (C₁-C₄)-Alkoxy-(C₁-C₄)-alkoxy, [(C₁-C₄)-Alkyl-O]ₜ-(C₂-C₄)-alkyl, 2-(Tetrahydro-2H-pyran-2-yloxy)-ethoxy, (C₂-C₈)-Alkenyl, (C₂-C₈)-Alkinyl, Phenoxy, Benzyloxy, welches im Phenylrest gegebenenfalls einen oder mehrere, vorzugsweise bis zu drei, gleiche oder verschiedene Substituenten aus der Reihe (C₁-C₄)-Alkyl, (C₁-C₄)-Halogenalkyl, (C₁-C₄)-Alkoxy, (C₁-C₄)-Halogenalkoxy und Halogen trägt,
- R⁶: Wasserstoff, Halogen, (C₁-C₈)-Alkyl, (C₃-C₈)-Cycloalkyl, (C₁-C₈)-Halogenalkyl, Phenyl und Benzyl bedeutet, wobei die Phenylringe wie oben bei R⁵ beschrieben substituiert sein können, oder
- R⁵ und R⁶: zusammen einen 3 - 7-gliedrigen Ring bilden können, der gegebenenfalls mit einem, zwei oder drei gleichen oder verschiedenen Resten aus der Reihe Halogen, (C₁-C₄)-Alkyl, (C₁-C₄)-Alkoxy, (C₁-C₄)-Halogenalkyl und (C₁-C₄)-Halogenalkoxy substituiert sein kann und in dem ein Kohlenstoffatom durch 0, S, NR⁷ ersetzt sein kann, wobei R⁷ die gleiche Bedeutung wie R⁴ hat, aber nicht Halogen sein darf.

Die Verbindungen liegen in den meisten Fällen als Enantiomere, Diastereomere, wie z.B. E/Z-lsomere oder deren Gemische vor. Die Erfindung umfaßt sowohl die reinen Isomeren als auch deren Gemische.

Bevorzugt sind solche Verbindungen der Formel I, für die
- A: die unter Formel I angebene Bedeutung hat,
- R¹: Wasserstoff oder Methyl ist,
- R²: Halogen, (C₁-C₄)-Alkyl, (C₁-C₄)-Halogenalkyl, (C₁-C₄)-Alkoxy oder (C₁-C₄)-Alkoxy-(C₁-C₄)-alkyl bedeutet,
- R³: Wasserstoff, Halogen, (C₁-C₃)-Alkyl, Methoxy oder Ethoxy bedeutet
oder
- R² und R³: zusammen mit den Kohlenstoffatomen, an die sie gebunden sind, einen ungesättigten 5- oder 6-gliedrigen Ring bilden, der ein bis zwei Stickstoffatome, ein Sauerstoff- oder Schwefelatom enthalten kann, oder
- R² und R³: zusammen mit den Kohlenstoffatomen, an die sie gebunden sind, einen gesättigten, gegebenenfalls substituierten, 5- oder 6-gliedrigen Ring bilden, der ein Schwefelatom oder 1 bis 2 Sauerstoffatome enthalten kann, und
- R⁴, R⁵, R⁶: und
- R⁷: sowie
- X, Y, m, n, p: die oben angegebenen Bedeutungen haben.

Besonders bevorzugt sind Verbindungen der Formel I, für die
- A: die unter der Formel I angegebene Bedeutung hat,
- R¹: Wasserstoff bedeutet,
- R²: (C₁-C₄)-Alkyl oder Methoxymethyl bedeutet,
- R³: Methyl, Ethyl, Methoxy, Chlor oder Brom bedeutet, oder
- R² und R³: zusammen mit den Kohlenstoffatomen, an die sie gebunden sind, das Chinolin- oder Chinazolin-System bilden, das mit Fluor, Chlor, Brom oder Methyl substituiert sein kann oder
- R² und R³: zusammen mit den Kohlestoffatomen, an die sie gebunden sind, ein ungesättigten 6-Ring bilden, der ein Stickstoffatom enthält, oder
- R² und R³: zusammen mit den Kohlenstoffatomen, an die sie gebunden sind, einen gesättigten 6-gliedrigen Ring bilden, der ein oder zwei Sauerstoffatome enthalten kann,
- m, n, p und X: die oben angegebenen Bedeutungen haben,
- Y: 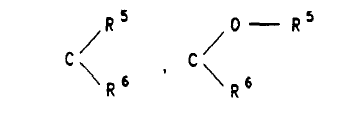 oder N - O - R⁵ sein kann
und
- R⁵, R⁶ und R⁷: ebenfalls die bereits genannten Bedeutungen haben.

Besonders bevorzugt sind weiterhin solche Verbindungen der Formel I, worin
- A und R¹ bis R⁷: die oben angegebenen Bedeutungen haben, und
- m und n: 2 sind,
- p: 0 ist, und
- X: die Bedeutung NH oder Sauerstoffatom hat.

Ganz besonders bevorzugt sind solche Verbindungen der Formel I, worin
- A: die unter Formel I angegebene Bedeutung hat,
- R¹: Wasserstoff bedeutet,
- R²: (C₁-C₄)-Alkyl oder Methoxymethyl bedeutet,
- R³: Chlor, Brom oder Methoxy bedeutet oder
- R² und R³: zusammen mit den Kohlenstoffatomen, an die sie gebunden sind, das Chinolin oder Chinazolin-System bilden, das mit Fluor, Chlor, Brom oder Methyl substituiert sein kann oder
- R² und R³: zusammen mit dem Pyrimidin-Ring das 5,6,7,8-Tetrahydrochinazolin-System oder das 5,6-Dihydro-7H-pyrano [2,3-d]pyrimidin-System bilden,
- m und n: die Zahl zwei bedeuten, und
- p: gleich null ist,
- X: die Bedeutung NH oder Sauerstoffatom hat,
- Y: 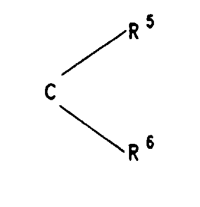 oder N - O - R⁵ bedeutet,
- R⁵, R⁶ und R⁷: die bereits genannten Bedeutungen haben.

Ganz besonders bevorzugt sind weiterhin solche Verbindungen der Formel I, worin
- A: die unter Formel I angegebene Bedeutung hat,
- R¹: Wasserstoff bedeutet,
- R²: Methoxymethyl und R³ Methoxy oder
- R²: (C₁-C₄)-Alkyl und R³ Chlor oder Brom bedeuten oder
- R² und R³: zusammen mit dem Kohlenstoffatom, an das sie gebunden sind, ein Chinolin oder Chinazolin-System bilden, das mit Fluor, Chlor oder Methyl substituiert ist oder ein 5,6,7,8-Tetrahydrochinazolin-, oder das 5,6-Dihydro-7H-pyrano[2,3-d]pyrimidin-System bilden,
- m, n, p, X sowie Y: die oben genannten Bedeutungen haben,
- R⁵: (C₂-C₈)-Alkyl, Cyclopentyl, Cyclohexyl, (C₁-C₈)-Alkylcarbonyl, (C₁-C₈)-Alkoxycarbonyl, Phenyl, Benzyl, Benzoyl bedeutet, wobei die letztgenannten drei Reste unsubstituiert oder mit einem oder zwei Substituierten versehen sein können die gleich oder verschieden sein können und diese Fluor, Chlor, Brom, (C₁-C₄)-Alkyl, (C₁-C₄)-Alkoxy, Trifluormethyl, (C₁-C₂)-Halogenalkoxy, Cyclohexyl, 2-Ethoxyethoxy, Methylthio oder Dimethylamino bedeuten und
- R⁶: H, Halogen oder (C₁-C₃)-Alkyl sein kann.

In der obigen Formel I ist unter "Halogen" ein Fluor-, Chlor-, Brom- oder Iodatom vorzugsweise ein Fluor-, Chlor- oder Bromatom zu verstehen, unter dem Ausdruck "(C₁-C₄)-Alkyl" ein unverzweigter oder verzweigter Kohlenwasserstoffrest mit 1-4 Kohlenstoffatomen, wie z. B. der Methyl-, Ethyl-, Propyl-, 1-Methylethyl-, 1-Methylpropyl-, 2-Methylpropyl- oder 1,1-Dimethylethylrest, unter dem Ausdruck "(C₁-C₈)-Alkyl" die vorgenannten Alkylreste sowie z. B. der Pentyl, 2-Methylbutyl- oder der 1,1-Dimethylpropylrest, der Hexyl-, Heptyl-, Octylrest oder 1,1,3,3-Tetramethylbutylrest;
unter dem Ausdruck "(C₁-C₁₂)-Alkyl" die vorgenannten Alkylreste sowie z. B. der Nonyl-, Decyl-, Undecyl- oder Dodecylrest;
unter dem Ausdruck "(C₃-C₈)-Cycloalkyl" die Cyclopropyl-, Cyclobutyl-, Cyclopentyl-, Cyclohexyl-, Cycloheptyl- oder Cyclooctylgruppe;
unter dem Ausdruck "(C₁-C₄)-Alkoxy" eine Alkoxygruppe, deren Kohlenwasserstoffrest die unter dem Ausdruck "(C₁-C₄)-Alkyl" angegebene Bedeutung hat;
unter dem Ausdruck "(C₃-C₈)-Cycloalkoxy" eine Cycloalkoxygruppe, deren Kohlenwasserstoffrest die unter "(C₃-C₈)-Cycloalkyl" angegebene Bedeutung hat;
unter dem Ausdruck "(C₁-C₄)-Alkylthio" eine Alkylthiogruppe, deren Kohlenwasserstoffrest die unter dem Ausdruck "(C₁-C₄)-Alkyl" angegebene Bedeutung hat;
unter dem Ausdruck "(C₁-C₄)-Halogenalkoxy" eine Halogenalkoxygruppe, deren Halogen-Kohlenwasserstoffrest die unter dem Ausdruck "(C₁-C₄)-Halogenalkyl" angegebene Bedeutung hat;
unter dem Ausdruck "(C₁-C₄)-Alkoxy-(C₁-C₄)-alkyl" beispielsweise eine 1-Methoxyethylgrupe, eine 2-Methoxyethylgruppe, eine 2-Ethoxyethylgruppe, eine Methoxymethyl- oder Ethoxymethylgruppe, eine 3-Methoxypropylgruppe oder eine 4-Butoxybutylgruppe;
unter dem Ausdruck "(C₁-C₄)-Alkylthio-(C₁-C₄)-alkyl" beispielsweise Methylthio, Ethylthiomethyl, Propylthiomethyl, 2-Methylthioethyl, 2-Ethylthioethyl oder 3-Methylthiopropyl;
unter dem Ausdruck "(C₁-C₄)-Alkylamino" eine Alkylaminogruppe, deren Kohlenwasserstoffrest die unter dem Ausdruck "(C₁-C₄)-Alkyl" angegebene Bedeutung hat, bervorzugt die Ethyl- und Methylaminogruppe;
unter dem Ausdruck "Di-(C₁-C₄)-alkylamino" eine Dialkylaminogruppe, deren Kohlenwasserstoffreste die unter dem Ausdruck "(C₁-C₄)-Alkyl" angegebene Bedeutung haben, bevorzugt die Dimethyl- und Diethylaminogruppe;
unter dem Ausdruck "(C₁-C₄)-Halogenalkyl" eine unter dem Ausdruck "(C₁-C₄)-Alkyl" genannte Alkylgruppe, in der eines oder mehrere Wasserstoffatome durch die obengenannten Halogenatome, bevorzugt Chlor oder Fluor, ersetzt wird, wie beispielsweise die Trifluormethylgruppe, die 2,2,2-Trifluorethylgruppe, die Chlormethyl-, Fluormethylgruppe, die Difluormethylgruppe oder die 1,1,2,2-tetrafluorethylgruppe;
unter dem Ausdruck "(C₃-C₈)-Cycloalkyl-(C₂-C₄)-alkyl" eine der oben genannten (C₁-C₄)-Alkylgruppen, die mit einer der oben genannten (C₃-C₈)-Cycloalkylgruppen substituiert ist, beispielsweise Cyclohexylmethyl, Cyclohexylethyl, Cyclohexylpropyl, Cyclohexylbutyl oder 1-Cyclohexyl-1-methyl-ethyl;
unter dem Ausdruck "Phenyl-(C₁-C₄)-alkyl" eine der oben genannten (C₁-C₄)-Alkylgruppen, die mit einer Phenylgruppe subsituiert ist, beispielweise die Benzyl- die 2-Phenylethyl-, die 1-Phenylethyl-, die 1-Methyl-1-phenylethylgruppe, die 3-Phenylpropyl-, die 4-Phenylbutylgruppe oder die 2-Methyl-2-phenyl-ethylgruppe;
unter dem Ausdruck "Aryl" beispielsweise Phenyl, Naphthyl oder Biphenyl, vorzugsweise Phenyl;
unter dem Ausdruck "gegebenenfalls substituiertes Phenyl", wenn im einzelnen nicht anders definiert, einen Phenylrest, der einen, zwei oder drei gleiche oder verschiedene Substituenten aus der Reihe Halogen, (C₁-C₄)-Alkyl, (C₁-C₄)-Alkoxy, (C₁-C₄)-Halogenalkyl und (C₁-C₄)-Halogenalkoxy trägt;
unter dem Ausdruck "gegebenenfalls substituiertes Benzoyl" einen Rest, in welchem der Phenylteil wie "gegebenenfalls substituiertes Phenyl" substituiert ist;
unter dem Ausdruck "gegebenenfalls substituiertes Benzoylcarbonyl" einen Rest, in welchem der Phenylteil wie "gegebenenfalls substituiertes Phenyl" substituiert ist;
unter dem Ausdruck "Heteroaryl" einen Arylrest, worin mindestens eine CH-Gruppe durch N ersetzt ist und/oder mindestens zwei benachbarte CH-Gruppen durch S, NH oder O ersetzt sind; Beispiele solcher Reste sind Thienyl, Benzothienyl, Furyl, Benzofuryl, Pyrrolyl, Imidazolyl, Pyrazolyl, Pyridyl, Pyrazinyl, Pyrimidinyl, Pyridazinyl, Indolizinyl, Isoindolyl, Indolyl, Indazolyl, Purinyl, Chinolyl, Isochinolyl, Phthalazinyl, Naphthydrinyl, Chinoxalinyl, Chinazolinyl, Cinnolinyl, Pteridinyl, Oxazolyl, Isoxazolyl, Thiazolyl, Isothiazolyl, Tetrazolyl;
unter dem Ausdruck "Benzyloxy-(C₁-C₄)-alkyl" eine (C₁-C₄)-Alkylgruppe mit den oben angegebenen Bedeutungen die mit einer Benzyloxygruppe substituiert ist, z. B. die Benzyloxymethyl- oder die 2-(Benzyloxy)-ethyl-Gruppe;
unter dem Ausdruck "(C₃-C₈)-Cycloalkyl-(C₁-C₄)-alkoxy" eine (C₁-C₄)-Alkoxygruppe mit den oben angegebenen Bedeutungen, die mit einer (C₃-C₈)-Cycloalkylgruppe mit den oben angegeben Bedeutungen substituiert ist, z. B. die Cyclopropylmethyloxy- oder die Cyclohexylmethyloxygruppe;
unter dem Ausdruck "Tri-(C₁-C₄)-alkylsilyl" eine Trialkylsilylgruppe die vorzugsweise zwei Methylgruppen und eine (C₁-C₈)-Alkylgruppe mit den oben angegebenen Bedeutungen trägt, z. B. die Trimethylsilyl-, die Dimethylethylsilyl oder die Dimethyloctylsilylgruppe;
unter dem Ausdruck "Di-(C₁-C₈)-alkyl-(C₁-C₄)-halogenalkylsilyl" einen Silylrest, der vorzugsweise zwei Methylgruppen und einen (C₁-C₄)-Halogenalkylrest mit den unter dem Ausdruck (C₁-C₄)-Halogenalkyl angegebenen Bedeutungen trägt, z. B. der Dimethyl-3,3,3-trifluorpropylsilyl-Rest;
unter dem Ausdruck "(C₁-C₄)-Alkoxy-(C₁-C₄)-alkoxy" z B. die Ethoxymethoxy-, 2-Ethoxy-ethoxy-, 2- Butoxyethoxy- oder 2-Methoxyethoxy-Gruppe;
unter dem Ausdruck "(C₂-C₈)-Alkenyl" einen unverzweigten oder verzweigten Rest, wie die Vinyl-, Allyl-, 2-Butenyl-, 2-Pentenyl- oder 2-Hexenylgruppe;
unter dem Ausdruck "(C₂-C₈)-Alkinyl" z. B. die Propargyl-, 2-Butinyl- oder 2-Pentinylgruppe;
unter dem Ausdruck "(C₁-C₈)-Alkanoyl" einen unverzweigten oder verzweigten Rest, wie Formyl, Acetyl, Propionyl, Butyryl, Isobutyryl, Valeryl, Isovaleryl, Pivaloyl, Caproyl oder Octanoyl;
unter dem Ausdruck "Tri-(C₁-C₄)-alkylsilylmethoxy" ein Trialkylsilylmethoxyrest mit vorzugsweise 2 Methylgruppen, worin die (C₁-C₄)-Alkylgruppe die oben angegebenen Bedeutungen hat;
unter dem Ausdruck "Di-(C₁-C₈)-alkyl-phenyl-(C₁-C₄)-alkyls ein Trialkylsilylrest mit vorzugsweise zwei Methylgruppen, bei dem eine Alkylgruppe die oben für den Ausdruck "Phenyl-(C₁-C₄)-alkyl" angegebenen Bedeutungen hat, vorzugsweise die Dimethylbenzylsilyl-Gruppe.

Die oben gegebene Erläuterung gilt entsprechend für homologe Reste bzw. von den genannten Resten abgeleitete Reste.

Die vorliegende Erfindung betrifft die Verbindungen der Formel I in Form der freien Base oder eines Säureadditionssalzes. Säuren, die zur Salzbildung herangezogen werden können, sind anorganische Säuren, wie Salzsäure, Bromwasserstoffsäure, Salpetersäure, Schwefelsäure, Phosphorsäure oder organische Säuren wie Ameisensäure, Essigsäure, Propionsäure, Oxalsäure, Fumarsäure, Adipinsäure, Stearinsäure, Ölsäure, Methansulfonsäure, Benzolsulfonsäure oder Toluolsulfonsäure.

Die Verbindungen der Formel I weisen zum Teil ein oder mehrere Chiralitätselemente auf. Es können daher Racemate und Diastereomere auftreten. Die Erfindung umfaßt sowohl die reinen Isomeren als auch deren Gemische. Die Gemische von Diastereomeren können nach gebräuchlichen Methoden, z. B. durch selektive Kristallisation aus geeigneten Lösungsmitteln oder durch Chromatographie in die Komponenten aufgetrennt werden. Racemate können nach üblichen Methoden in die Enantiomeren aufgetrennt werden, so z. B. durch Salzbildung mit einer optisch aktiven Säure, Trennung der diasteromeren Salze und Freisetzung der reinen Enantiomeren mittels einer Base.

Die Erfindung betrifft weiterhin ein Verfahren zur Herstellung von Verbindungen der Formel I, das dadurch gekennzeichnet ist, daß man
a) eine Verbindung der Formel II 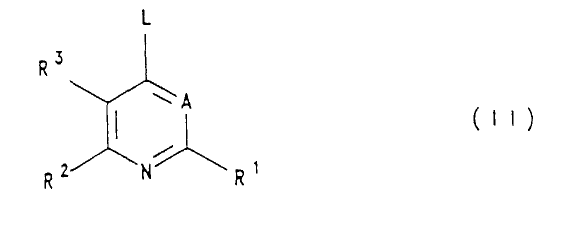 worin A, R¹, R² und R³ die unter Formel I angegebenen Bedeutungen haben und L eine Abgangsgruppe, beispielsweise Halogen, Alkylthio, Alkansulfonyloxy oder Arylsulfonyloxy, Alkylsulfonyl oder Arylsulfonyl bedeutet, mit einem Nucleophil der Formel III 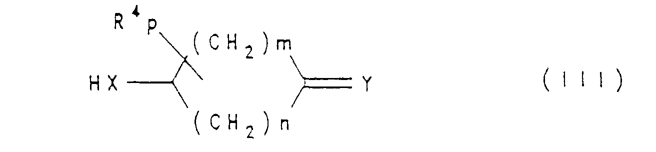 worin X, R⁴, n, m und Y die unter Formel I angegebenen Bedeutungen haben, umsetzt und die so erhaltenen Verbindungen der Formel I gegebenenfalls, falls R³ Wasserstoff und A Stickstoff bedeutet, an C⁵ des Pyrimidins chloriert oder bromiert,
   oder
b) zur Herstellung einer Verbindung der Formel I, worin Y für 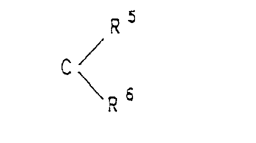 steht, eine Verbindung der Formel V 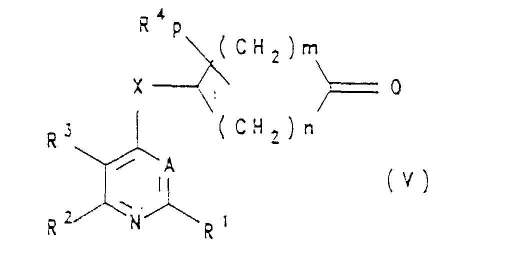 worin R¹, R², R³, R⁴, A, X, m, n und p die unter Formel I angegebenen Bedeutungen haben, in Gegenwart einer Base umsetzt mit einer Verbindung der Formel P⁺Ph₃CH-R⁵R⁶Z⁻, worin Ph einen aromatischen Rest und Z⁻ ein Anion bedeutet und R⁵ und R⁶ wie unter Formel I definiert sind, oder
c) zur Herstellung einer Verbindung, worin Y nicht für 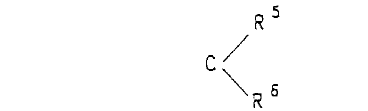 steht, eine Verbindung der unter b) definierten Formel V umsetzt mit H₂NOH oder einer Verbindung der Formel H-Q, worin Q für HNR⁵, HN-OR⁵, HN-NR⁵R⁶ oder N(OH)R^{a} steht und R⁵, R⁶ wie unter Formel I definiert sind,
   nach a) oder c) erhaltene Verbindungen der Formel I, worin Y für N-OH steht, gegebenenfalls in Gegenwart einer Base umsetzt, mit einer Verbindung der Formel L-R⁵, worin L eine Abgangsgruppe bedeutet und R⁵ wie unter Formel I definiert ist, aber nicht Wasserstoff bedeutet,
   und erhaltene Verbindungen gegebenenfalls in ihr Salz überführt.

Die oben beschriebene Substitutionsreaktion ist im Prinzip bekannt. Die Abgangsgruppe L ist in weiten Grenzen variierbar und kann beispielsweise ein Halogenatom wie Fluor, Chlor, Brom oder lod bedeuten oder Alkylthio wie Methyl- oder Ethylthio, oder Alkansulfonyloxy wie Methan-, Trifluormethan- oder Ethansulfonyloxy oder Arylsulfonyloxy, wie Benzolsulfonyloxy oder Toluolsulfonyloxy oder Alkylsulfonyl wie Methyl- oder Ethylsulfonyl oder Arylsulfonyl wie Phenyl- oder Toluolsulfonyl.

Die vorgenannte Reaktion wird in einem Temperaturbereich von 20 - 150 °C, zweckmäßig in Anwesenheit einer Base und gegebenenfalls in einem inerten organischen Lösungsmittel wie N,N-Dimethylformamid, N,N-Dimethylacetamid, Dimethylsulfoxid, N-Methylpyrrolidin-2-on, Dioxan, Tetrahydrofuran, 4-Methyl-2-pentanon, Methanol, Ethanol, Butanol, Ethylenglykol, Ethylenglykoldimethylether, Toluol, Chlorbenzol oder Xylol durchgeführt. Es können auch Gemische der genannten Lösungsmittel verwendet werden.

Geeignete Basen sind beispielsweise Alkali- oder Erdalkalimetallcarbonate, - hydrogencarbonate, -amid oder -hydride wie Natriumcarbonat,
Natriumhydrogencarbonat, Kaliumcarbonat, Natriumamid oder Natriumhydrid, lithiumorganische Verbindungen, wie n-Butyllithium.

Geeignete Basen für den Fall, daß X Sauerstoff bedeutet, sind beispielsweise Alkali- oder Erdalkalicarbonate, -hydrogencarbonate, -amide oder -hydride wie Natriumcarbonat, Natriumhydrogencarbonat, Kaliumcarbonat, Natriumamid oder Natriumhydrid, für den Fall, daß X NH bedeutet, sind dies beispielsweise Alkali-oder Erdalkalimetallcarbonate, -hydrogencarbonate, -hydroxide, -amide oder - hydride wie Natriumcarbonat, Natriumhydrogencarbonat, Kaliumcarbonat, Natriumhydroxid, Natriumamid oder Natriumhydrid oder organische Basen wie Triethylamin oder Pyridin. Auch ein zweites Äquivalent eines Amins III kann als Hilfsbase eingesetzt werden.

Die Erfindung betrifft weiterhin ein Verfahren zur Herstellung von Verbindungen der Formel V, das dadurch gekennzeichnet ist, daß man eine Verbindung der Formel IV 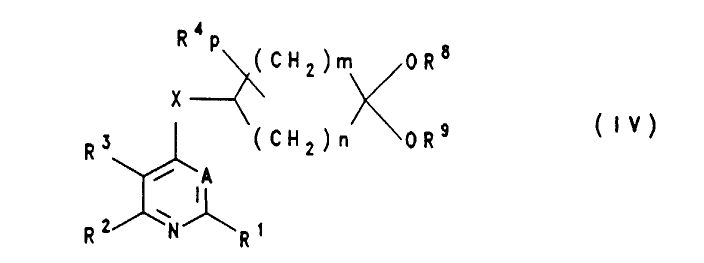 worin A, R¹, R², R³, R⁴, X, n, m und p die unter Formel I angegebene Bedeutung haben, und
R⁸ und R⁹ gleich oder verschieden sind, und Wasserstoff, (C₁-C₈)-Alkyl, (C₁-C₈)-Halogenalkyl bedeuten, oder
R⁸ und R⁹ zusammen einen gesättigten 5- ,6- oder 7-gliedrigen isocyclischen Ring bilden, der gegebenenfalls ein- oder mehrfach, vorzugsweise bis zu dreifach mit gleichen oder verschiedenen Resten, wie (C₁-C₄)-Alkyl, Benzyl oder Phenyl substituiert ist,
unter Verwendung einer anorganischen oder organischen Säure, wie zum Beispiel HCI, H₂SO₄, HNO₃, H₃PO₄, Oxalsäure, Essigsäure oder saurem Ionentauscher zu einer Verbindung der Formel V umgewandelt wird. 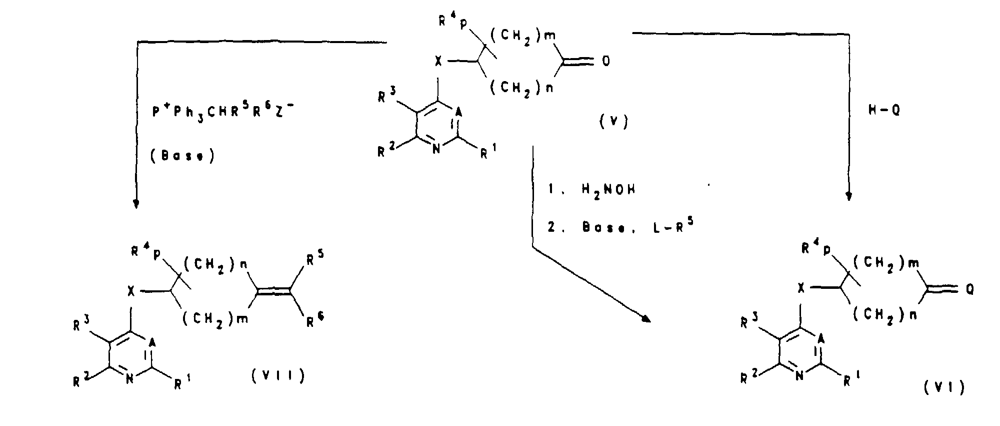 wobei die Reste A, R², R³, X, R⁵, R⁶, R⁷, n, m und p die unter Formel I angegebene Bedeutung haben, Q für HNR⁵, HNOR⁵, HN-NR⁵R⁶ oder N(OH)R⁵, Ph für einen aromatischen Rest, vorzugsweise Phenyl und Z⁻ für ein Anion, vorzugsweise Halogenid, wie Jodid stehen. Verbindungen der Formel IV sind bereits in der DOS 4116089 beschrieben. Für den Fall, daß R² und R³einen ungesättigten 5- oder 6-gliedrigen isocyclischen Ring bilden, der auch ein oder mehrere Stickstoffatome enthalten kann und der gegebenenfalls durch 1, 2 oder 3 gleiche oder verschiedene Reste substituiert ist und diese Reste (C₁-C₄)-Alkyl, (C₁-C₄)-Alkoxy, (C1-C4)-Halogenalkyl, (C₁-C₄)-Halogenalkoxy und/oder Halogen bedeuten, sind Verbindungen der Formel IV neu und sind Gegenstand der Erfindung.

Verbindungen der Formel V sind Ausgangsverbindungen zur Herstellung von Verbindungen der Formel VI und VII, die nach zu bekannten Verfahren analogen Verfahren hergestellt werden, wobei Z vorzugsweise ein Halogenatom ist, und insbesondere Cl, Br oder I bedeutet. Die Verbindungen der Formel V sind ebenfalls neu und Gegenstand der Erfindung.

H₂NOH und Verbindungen der Formel HQ können als freie Basen oder in Form ihrer Säureadditionssalze eingesetzt werden. Aus letzteren wird H₂NOH oder HQ durch Basenzugabe in situ freigesetzt. Hierfür geeignete Basen sind Alkali- oder Erdalkalihydroxide, -carbonate, -hydrogencarbonate, -acetate oder -alkoholate, wie Natriumhydroxid, Natriumcarbonat, Natriumacetat,
Natriumhydrogencarbonat oder Kaliumcarbonat oder organische Basen wie Triethylamin oder Pyridin.

Die vorgenannten, zu Verbindungen der Formel VI führenden Reaktionen werden in einem Temperaturbereich von -20 bis 150°C, und gegebenenfalls in einem inerten organischem Lösungsmittel wie N,N-Dimethylformamid, N,N-Dimethylacetamid, Dimethylsulfoxid, N-Methylpyrrolidin-2-on, Dioxan, Tetrahydrofuran, 4-Methyl-2-pentanon, Methanol, Ethanol, Butanol, Ethylenglykol, Ethylenglykoldimethylether, Toluol, Chlorbenzol oder Xylol durchgeführt. Es können auch Gemische der genannten Lösungsmittel verwendet werden.

Die vorgenannten, zu Verbindungen der Formel VII führenden Reaktionen werden in einem inerten organischen Lösungsmittel oder Lösungsmittelgemisch aus der Reihe der vorstehend genannten Lösungsmittel und Lösungsmittelgemische in Gegenwart einer Base bei einer Temperatur zwischen -80°C und dem Siedepunkt des Reaktionsgmeisches, vorzugsweise zwischen 10 und 70°C durchgeführt. Geeignete Basen sind beispielsweise Alkali- oder Erdalkalihydroxide, -alkoholate, -carbonate, -amide oder -hydride, wie Natriumhydroxid, Natriumethanolat, Natriumcarbonat, Kaliumcarbonat, Natriumamid oder Natriumhydrid, oder lithiumorganische Verbindungen, wie n-Butyllithium.

Die Ausgangsverbindungen der Formel II sind entweder bekannt oder sie können analog zu bekannten Verfahren hergestellt werden; siehe z. B.:
- Chinoline:: Org. Synth., Coll. Vol. 3, 272 (1955)
- 1,5-Naphthyridine:: J. Amer. Chem. Soc. 68, 1317 (1946) und
Britisches Patent 1147760

| | |
|---|---|
| 1,6-Naphthyridine | J. Chem. Soc. 1960, 1790 |
| 1,7-Naphthyridine | J. Org. Chem. 19, 2008 (1954) |
| 1,8-Naphthyridine | Synthesis 1974, 809 |
| Pyridopyrimidine | EP-A-414 386 |
| Pteridine | J. Chem. Soc. 1951, 474 |

Als Ausgangsprodukte dienen für den Fall, daß A ein Stickstoffatom ist, Acetessigester-Derivate, die über die entsprechenden Hydroxypyrimidine in die Halogenpyrimidine überführt werden: 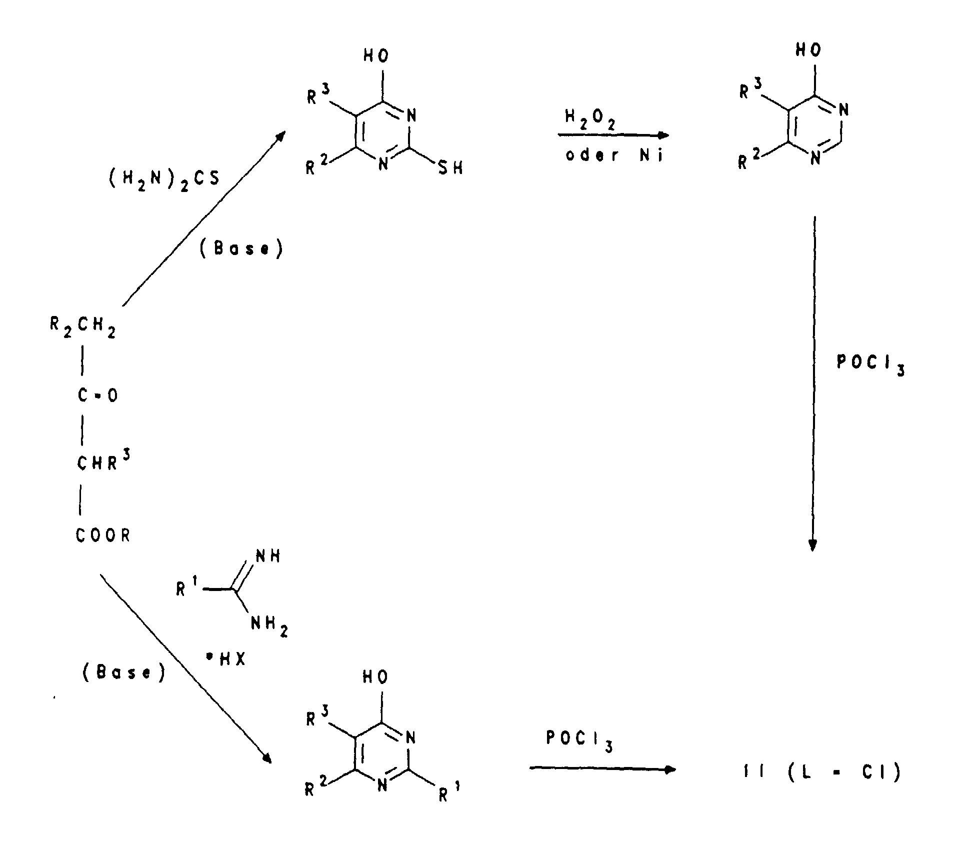 Für den Fall, daß R² und R³ zusammen mit den Kohlenstoffatomen, an die sie gebunden sind, einen gesättigten 5-, 6- oder 7-gliedrigen isocyclischen Ring bilden, der an Stelle von CH₂ ein oder mehrere Sauerstoff- oder Schwefelatome enthalten kann und der gegebenenfalls durch ein bis vier (C₁-C₄)-Alkylgruppen und/oder Halogen substituiert ist, sind die Verbindungen der Formel II neu und werden beansprucht.
Die Ausgangsverbindungen der Formel II können weiterhin in Analogie zu bekannten Verfahren aus Malonester-Derivaten erhalten werden:

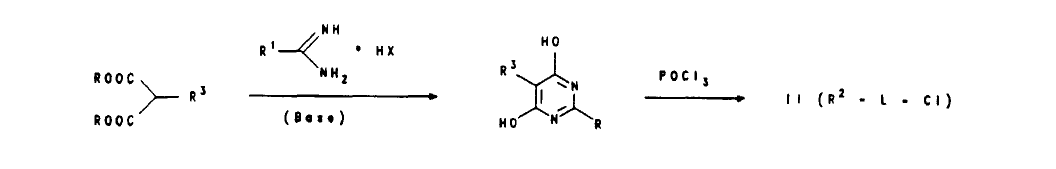

Die als Ausgangsprodukte benötigten Nucleophile der Formel III können für den Fall, daß X Sauerstoff bedeutet, nach bekannten Verfahren hergestellt werden, beispielsweise durch Reduktion einer Carbonylgruppe mit einem geeigneten Reduktionsmittel, beispielsweise einem komplexen Metallhydrid oder im Falle eines Aldehyds oder Ketons auch mit Wasserstoff und einem Hydrierkatalysator. Weitere Möglichkeiten sind die Umsetzung einer metallorganischen Verbindung mit einer Carbonylgruppe oder einem Oxiran.

Die als Ausgangsprodukte benötigten Nucleophile der Formel III können für den Fall, daß X NH bedeutet, ebenfalls nach bekannten Methoden hergestellt werden, beispielsweise Reduktion eines Oxims mit einem geeigneten Reduktionsmittel, beispielsweise einem komplexen Metallhydrid oder Wasserstoff in Gegenwart eines Hydrierkatalysators, reduktive Aminierung oder Leuckart-Wallach-Reaktion eines Aldehyds oder Ketons oder Gabriel-Reaktion eines Alkylhalogenids oder -Tosylats.

Die Verbindungen der Formel I, worin R³ Halogen bedeutet, können nach bekannten Verfahren halogeniert werden.

Die erfindungsgemäßen Verbindungen der Formel I zeichnen sich durch eine hervorragende fungizide Wirkung aus. Bereits in das pflanzliche Gewebe eingedrungene pilzliche Krankheitserreger lassen sich erfolgreich kurativ bekämpfen. Dies ist besonders wichtig und vorteilhaft bei solchem Pilzkrankheiten, die nach eingetretener Infektion mit den sonst üblichen Fungiziden nicht mehr wirksam bekämpft werden können. Das Wirkungsspektrum der beanspruchten Verbindungen erfaßt verschiedene wirtschaftlich bedeutende, phytopathogene Pilze, besonders aber Erysiphe graminis und Leptosphaeria nodorum.

Die erfindungsgemäßen Verbindungen eignen sich daneben auch für den Einsatz in technischen Bereichen, beispielsweise als Holzschutzmittel, als Konservierungsmittel in Anstrichfarben, in Kühlschmiermittel für die Metallbearbeitung oder als Konservierungsmittel in Bohr- und Schneidölen.

Gegenstand der Erfindung sind auch Mittel, die die Verbindungen der Formel I neben geeigneten Formulierungshilfsmitteln enthalten. Die erfindungsgemäßen Mittel enthalten die Wirkstoffe der Formel I im allgemeinen zu 1 bis 95 Gew.-%. Sie können auf verschiedene Art formuliert werden, je nachdem wie es durch die biologischen und/oder chemisch-physikalischen Parameter vorgegeben ist. Als Formulierungsmöglichkeiten kommen daher in Frage: Spritzpulver (WP), emulgierbare Konzentrate (EC), wäßrige Dispersionen auf Öl- oder Wasserbasis (SC, Suspoemulsionen (SC), Stäubemittel (DP), Beizmittel, Granulate in Form von wasserdispergierbaren Granulaten (WG), ULV-Formulierungen, Mikrokapseln, Wachse oder Köder.

Diese einzelnen Formulierungstypen sind im Prinzip bekannt und werden beispielsweise beschrieben in:
Winnacker-Küchler, "Chemische Technologie", Band 7, C. Hauser Verlag München, 4. Aufl. 1986; von Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekann und werden beispielsweise beschrieben in:
Watkins, "Handbook of Insecticide Dust Diluents and Carrier", 2nd Ed., Darland Books, Caldwell N.J.; H.v.Olphen, "Introduction to Clay Colloid Chemistry, 2nd Ed., J. Wiley & Sons, N.Y.; Marschen, "solvents Guide", 2nd Ed., Interscience, N.Y. 1950; McCutcheon's "Detergents and Emulsifiers Annual", MC Publ. Copr., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder
Wachstumregulatoren herstellen, z.B,. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch netzmittel, z.B. polyoxethylierte Alkylphenole, polyoxethylierte Fettalkohole, Alkyl- oder Alkylphenol-sulfonate und Dispergiermittel, z.B. ligninsulfonsaures Natrium, 2,2'-dinaphthylmethan-6,6'-disulfonsaures Natrium, dibutylnaphthalinsulfonsaures Natrium oder auch oleylmethyltaurinsaures Natrium enthalten. Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden:
Alkylarylsulfonsaure Calcium-Salze wie Ca-dodecylbenzolsulfonat oder nichtionische Emulgatoren wie Fettsäurepolyglykolester, Alkylarylpolyglykolether, Fettalkoholpolyglykolether, Polyoxyethylenpolyoxypropylen-Sorbitanfettsäureester, Polyoxyethylensorbitan-Fettsäureester oder Polyoxyethylensorbitester.

Stäubemittel erhält man durch Vermahlen des Wirkstoffes mit fein verteilten festen Stoffen, z.B. Talkum, natürlichen Tonen wie Kaolin, Bentonit, Pyrophyllit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemittel, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die herstellung von Düngemittelgranulaten üblichen Weise - gewünschtenfalls in Mischung mit Düngemitteln - granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbarten Konzentraten kann die Wirkstoffkonzentration etwa 5 bis 80 Gew.-% betragen. staubförmige Formulierungen enthalten meistens 5 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnte, z.B. bei Spritzpulvern, emulgierbaren Konzentraten, Dispersionen und teilweise aucvh bei Mikrogranulaten mittels Wasser.

Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Granzen schwanken, z.B. zwischen 0,005 und 10 kg/ha oder mehr Aktivsubstanz, vorzugsweise liegt sie jedoch zwischen 0,01 und 5 kg/ha.

Die erfindungsgemäßen Wirkstoffe können in ihren handelsüblichen Formulierungen entweder allein oder in Kombination mit weiteren, literaturbekannten Fungiziden angewendet werden.

Als literaturbekannte Fungizide, die erfindungsgemäße mit den Verbindungen der Formel I kombiniert werden können, sind z.B. folgende Produkte zu nennen: Aldimorph, Andoprim, Anilazine, BAS 480F, BAS 490F, Benalaxyl, Benodanil, Benomyl, Binapacryl, Bitertanol, Bromuconazol, Buthiobate, Captafol, Captan, Carbendazim, Carboxin, CGA 173506, Chlobenzthiazone, Chlorthalonil, Cymoxanil, Cyproconazole, Cyprofuram, Dichlofuanid, Dichlomezin, Diclobutrazol, Diethofencarb, Difenconazol (CGA 169374), Difluconazole, Dimethirimol, Dimethomorph, Diniconazole, Dinocap, Dithianon, Dodemorph, Dodine, Edifenfos, Ethirimol, Etridiazol, Fenarimol, Fenfuram, Fenpiclonil, Fenpropidin, Fenpropimorph, Fentinacetate, Fentinhydroxide, Ferimzone (TF 164), Fluazinam, Fluobenzimine, Fluquinconazole, Fluorimide, Flusilazole, Flutolanil, Flutriafol, Folpet, Fosetylaluminium, Fuberidazole, Fulsulfamide (MT-F651), Furalaxyl, furconazol, Furmecyclox, Guazatine, Hexaconazole, ICI A5504, Imazalil, Imibenconazole, Iprobenfos, Iprodione, Isoprothiolane, KNF 317, Kupferverbindungen wie Cu-oxychlorid, Oxine-Cu, Cu-oxide, Mancozeb, Maneb, Mepanipyrim (KIF 3535), Metconazole, Mepronil, Metalaxyl, Methasulfocarb, Methfuroxam, MON 24000, Myclobutanil, Namab, Nitrothalidopropyl, Nuarimol, Ofurace, Oxadixyl, Oxycarboxin, Penconazol, Pencycuron, PP 969, Probenazole, Propineb, Prochloraz, Procymidon, Propamocarb, Propiconazol, Prothiocarb, Pyracarbolid, Pyrazophos, Pyrifenox, Pyroquilon, Rabenzazole, RH7592, Schwefel,Tebuconazole, TF 167, Thiabendazole, Thicyofen, Thiophanatemethyl, Thiram, Tolclofos-methyl, Tolylfluanid, Triadimefon, Triadimenol, Tricyclazole, Tridemorph, Triflumizol, Triforine, Validamycin, Vinchlozolin, XRD 563, Zineb, Natriumdodecylsulfonate, Natriumdodecylsulfat, natrium-C13/C15-alkoholethersulfonat,
Natriumcetostearylphosphatester, Dioctyl-natrium-sulfosuccinat, Natriumisopropylnaphthalensulfonat, Natrium-methylenebisnaphthalenesulfonat, Cetyltrimethyl-ammoniumchlorid, Salze von langkettigen primären, sekundären oder tertiären Aminen, Alkyl-propyleneamine, Lauryl-pyrimidiniumbromid, ethoxylierte quarternierte Fettamine, Alkyl-dimethyl-benzyl-ammoniumchlorid und 1-Hydroxyethyl-2-alkyl-imidazolin.

Die oben genannten Kombinationspartner stellen bekannte Wirkstoffe dar, die zum großen Teil in Ch.R. Worthing, U.S.B. Walker, The Pesticide Manual, 7. Auflage (1983), British Crop Protection Council beschrieben sind.

Darüber hinaus kann der erfindungsgemäße Wirkstoff in seinen handelsüblichen Formulierungen sowie in den aus diesen Formulierungen bereiteten Anwendungsformen in Mischung mit anderen Wirkstoffen, wie Insektiziden, Lockstoffen, sterilantien, Akariziden, Nematiziden oder Herbiziden vorliegen. Zu den Insektiziden zählen beispielsweise Phosphorsäureester, Carbamate, Carbonsäureester, Formamidine, Zinnverbindungen, durch Mikroorganismen hergestellte Stoffe u.a. Bevorzugte Mischungspartner sind:
1. Aus der Gruppe der Phosphorverbindungen
   Acephate, Azamethiphos, Azinphos-ethyl, Azinphosmethyl, Bromophos, Bromophos-ethyl, Chlorfenvinphos, Chlormephos, Chlorpyrifos, Chlorpyrifos-methyl, Demeton, Demeton-S-methyl, Demeton-S-methyl sulfon, Dialifos, Diazinon, Dichlorvos, Dicrotophos, O,O-1,2,2,2-tetrachlorethylphosphorthioate (SD 208 304), Dimethoate, Disulfoton, EPN, Ethion, Ethoprophos, Etrimfos, Famphur, Fenamiphos, Fenitriothion, Fensulfothion, Fenthion, Fonofos, Formothion, Heptenophos, Isophos, Isothioate, Isoxathion, Malathion, Methacrifos, methamidophos, Methidation, Salithion, Mevinphos, Monocrotophos, Naled, Omethoate, Oxydemeton-methyl, Parathion, Parathion-methyl, Phenthoate, Phorate, Phosalone, Phosfolan, Phosmet, Phosphamidon, Phoxim, Pirimiphos-ethyl, Pirimiphos-methyl, Profenofos, Propaphos, Proetamphos, Prothiofos, Pyraclofos, Pyridapenthion, Quinalfos, Sulprofos, Temephos, Terbufos, Tetrachlorvinphos, Thiometon, Triazaphos, Trichlorphon, Vamidothion.
2. Aus der Gruppe der Carbamate
   Aldicarb, 2-sec.-Butylphenylmethylcarbamate (BPMC), CArbaryl, CArbofuran, Carbosulfan, Cloethocarb, Benfuracarb, Ethiofencarb, Furathiocarb, Isoprocarb, Methomyl, 5-Methyl-m-cu-menylbutyryl(methyl)carbamate, Oxamyl, Pirimicarb, Propoxur, Thiodicarb, Thiofanox, Ethyl 4, 6,9-triaza-4-benzyl-6,10-dimethyl-8-oxa-7-oxo-5,11-dithia-9-dodecenoate (OK 135), 1-Methylthio(ethylidenamino)-N-methyl-N-(morpholinothio)carbamate (UC 51717).
3. Aus der Gruppe der Carbonsäureester
   Allethrin, Alphametrin, 5-Benzyl-3-furylmethyl-(E)-(1R)-cis-2,2-di-methyl-3-(2-oxothiolan-3-ylidenmethyl)cyclopropan-carboxylate, Bioallethrin, Bioallethrin((S)-cyclopentylisomer), Bioresmethrin, Biphenate, (RS)-1-Cyano-1-(6-phenoxy-2-pyridyl)-methyl-(1RS)-trans-3-(4-ter.butylphenyl)-2,2-dimethylcyclopropanecarboxylate (NCI 85193), Cycloprothrin, cyhalothrin, Cypermethrin, Cyphenothrin, Deltamethrin, Empenthrin, Esfenvalerate, Fentfluthrin, Fenpropathrin, Fenvalerate, Flucythrinate, Flumethrin, Fluvalinate (D-isomer), Permethrin, Pheothrin ((R)-lsomer), d-Prallethrin, Pyrethrine (natürliche Produkte), Resmethrin, Tefluthrin, Tetramethrin, Tralomethrin.
4. Aus der Gruppe der Amidine
   Amitraz, Chlordimeform.
5. Aus der Gruppe der Zinnverbindungen
   Cyhexatin, Fenbutatinoxide.
6. Sonstige
   Abamectin, Bacillus thuringiensis, Bensultap, Binapacryl, Bromopropylate, Buprofezin, Camphechlor, Cartap, Chlorobenzilate, Chlorfluazuron, 2-(4-(Chlorphenyl)-4,5-diphenylthiophen (UBI-T 930), Chlorfentezine, Cyclopropancarbonsäure-(2-naphthylmethyl)ester (Rol 2-0470), Cyromazin, N-(3,5-Dichlor-4-(1,1,2,3,3,3-hexafluor-1-propyloxy)phenyl)carbamoyl)-2-chlorbenzcarboximidsäureethylester, DDT, Dicofol, N-(N-(3,5-Dichlor-4-(1,1,2,2-tetrafluorethoxy)phenylaminolcarbonyl)-2,6-difluorbenzamid (XRD 473), Diflubenzuron, N-(2,3-Dihydro-3-methyl-1,3-thiazol-2-ylidene)-2,4-xylidene, Dinobuton, Dinocap, Endosulfan, Ethofenprox, (4-Ethoxyphenyl)(dimethyl)(3-(3-phenoxyphenyl)propyl)silan, (4-Ethoxyphenyl)(3-(4-fluoro-3-phenoxyphenyl)propyl)dimethylsilan, Fenoxycarb, 2-Fluoro-5-(4-(4-ethoxyphenyl-4-methyl-1-pentyl)diphenylether (MTI 800), Granulose- und Kernpolyederviren, Fenthiocarb, Flubenzimine, Flucycloxuron, Flufenoxuron, Gamma-HCH, hexythiazox, Hydramethylnon (AC 217300), Ivermectin, 2-Nitromethyl-4,5-dihydro-6H-thiazin (SD 52618), 2-Nitromethyl-3,4-dihydrothiazol (SD 35651), 2-Nitromethylene-1,2-thiazinan-3-ylcarbamaldehyde (WL 108477), Propargite, Teflubenzuron, Tetradifon, Tetrasul, Thiocyclam, Triflumuron.

Der Wirkstoffgehalt der aus den handelsüblichen Formulierungen bereiteten Anwendungsformen kann in weiten Bereichen variieren, die Wirkstoffkonzentration der Anwendungsformen kann von 0,0001 bis zu 95 Gew.% Wirkstoff, vorzugsweise zwischen 0,001 und 1 Gew.-% liegen. Die Anwendung geschieht in einer den Anwendungsformen angepaßten üblichen Weise.

Die Wirkstoffe eignen sich bei guter Pflanzenverträglichkeit und günstiger Warmblütertoxizität zur Bekämpfung von tierischen Schädlingen, insbesondere Insekten, Spinnentieren, Helminthen und Mollusken, ganz besonders bevorzugt zur Bekämpfung von Insekten und Spinnentieren, die in der Landwirtschaft, bei der Tierzucht, in Forsten, im Vorrats- und Materialschutz sowie auf dem Hygienesektor vorkommen. Sie sind gegen normal sensible und resistente Arten sowie alle oder einzelne Entwicklungsstadien wirksam. Zu den oben erwähnten Schädlingen gehören:
Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Eotetranychus spp., Oligonychus spp., Eutetranychus spp..
Aus der Ordnung der Isopoda z. B. Oniscus aselus, Armadium vulgare, Porcellio scaber.
Aus der Ordnung der Diplopoda z. B. Blaniulus guttulatus.
Aus der Ordnung der Chilopoda z. B. Geophilus carpophagus, Scutigera spp..
Aus der Ordnung der Symphyla z. B. Scutigerella immaculata.
Aus der Ordnung der Thysanura z. B. Lepisma saccharina.
Aus der Ordnung der Collembola z. B. Onychiurus armatus.
Aus der Ordnung der Orthoptera z. B. Blatta orientalis, Periplaneta americana, Leucophaea maderae, Blatella germanica, Acheta domesticus, Gryllotalpa spp., Locusta migratoria migratorioides, Melanoplus differentialis, Schistocerca gregaria.
Aus der Ordnung des Isoptera z. B. Reticulitermes spp..
Aus der Ordnung der Anoplura z. B. Phylloxera vastatrix, Pemphigus spp., Pediculus humanus corporis, Haematopinus spp., Linognathus spp..
Aus der Ordnung der Mallophaga z. B. Trichodectes pp., Damalinea spp..
Aus der Ordnung der Thysanoptera z. B. Hercinothrips femoralis, Thrips tabaci.
Aus der Ordnung der Heteroptera z. B. Eurygaster spp., Dysdercus intermedius, Piesma quadrata, Cimex lectularius, Rhodnius prolixus, Triatoma spp..
Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelus bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp..
Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylloides chrysocephala, Epilachna varivestis, Atomaria spp., Oryzaephilus surinamensis, Anthonumus spp., Sitophilus spp., Otiorrhynchus sulcatus, Cosmopolites sordidus, Ceuthorrynchus assimilis, Hypera postica, Dermestes spp., Trogoderma, Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z. B. Diprion spp., Hoplocampa spp., Lasius spp., Monomorium pharaonis, Vespa spp..
Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hypobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z. B. Xenopsylla cheopsis, Ceratophyllus spp..
Aus der Ordnung der Arachnida z. B. Scorpio maurus, Latrodectus mactans.
Aus der Klasse der Helminthen z. B. Haemonchus, Trichostrongulus, Ostertagia, Cooperia, Chabertia, Strongyloides, Oesophagostomum, Hyostrongulus, Ancylostoma, Ascaris und Heterakis sowie Fasciola und pflanzenschädigende Nematoden z. B. solche der Gattungen Meloidogyne, Heterodera, Ditylenchus, Aphelenchoides, Radopholus, Globodera, Pratylenchus, Longidorus und Xiphinema.
Aus der Klasse der Gastropoda z. B. Deroceras spp., Arion spp., Lymnaea spp., Galba spp., Succinea spp., Biomphalaria spp., Bulinus spp., Oncomelania spp.. Aus der Klasse der Bivalva z. B. Dreissena spp..

Die Erfindung betrifft auch Mittel, insbesondere insektizide und akarizide Mittel, die die Verbindungen der Formel I neben geeigneten Formulierungshilfsmitteln enthalten.

Die erfindungsgemäßen Mittel enthalten die Wirkstoffe der Formel I im allgemeinen zu 1 bis 95 Gew.-%.

Sie können auf verschiedene Art formuliert werden, je nachdem wie es durch die biologischen und/oder chemisch-physikalischen Parameter vorgegeben ist.

Mit den äußeren Bedingungen wie Temperatur, Feuchtigkeit u. a. variiert die erforderliche Aufwandmenge. Sie kann innerhalb weiter Grenzen schwanken, z. B. zwischen 0,0005 und 10,0 kg/ha oder mehr Aktivsubstanz, vorzugsweise liegt sie jedoch zwischen 0,001 und 5 kg/ha.

Die erfindungsgemäßen Wirkstoffe können in ihren handelsüblichen Formulierungen sowie in den aus diesen Formulierungen bereiteten Anwendungsformen in Mischungen mit anderen Wirkstoffen, wie Insektiziden, Lockstoffen, Sterilantien, Akariziden, Nematiziden, Fungiziden, wachstumsregulierenden Stoffen oder Herbiziden vorliegen.

Zu den Schädlingsbekämpfungsmitteln zählen beispielsweise Phosphorsäureester, Carbamate, Carbonsäureester, Formamidine, Zinnverbindungen, durch Mikroorganismen hergestellte Stoffe u. a.. Bevorzugte Mischungspartner sind
1. aus der Gruppe der Phosphorverbindungen
   Acephate, Azamethiphos, Azinphos-ethyl-, Azinphosmethyl, Bromophos, Bromophos-ethyl, Chlorfenvinphos, Chlormephos, Chlorpyrifos, Chlorpyrifosmethyl, Demeton, Demeton-S-methyl, Demeton-S-methyl sulfon, Dialifos, Diazinon, Dichlorvos, Dicrotophos, O,O-1,2,2,2-Tetrachlorethylphosphorthioate (SD 208 304), Dimethoate, Disulfoton, EPN, Ethion, Ethoprophos, Etrimfos, Famphur, Fenamiphos, Fenitriothion, Fensulfothion, Fenthion, Fonofos, Formothion, Heptenophos, Isozophos, Isothioate, Isoxathion, Malathion, Methacrifos, Methamidophos, Methidathion, Salithion, Mevinphos, Monocrotophos, Naled, Omethoate, Oxydemeton-methyl, Parathion, Parathionmethyl, Phenthoate, Phorate, Phosalone, Phosfolan, Phosmet, Phosphamidon, Phoxim, Pirimiphos, Primiphos-ethyl, Pirimiphos-methyl, Profenofos, Propaphos, Proetamphos, Prothiofos, Pyraclofos, Pyridapenthion, Quinalphos, Sulprofos, Temephos, Terbufos, Tetrachlorvinphos, Thiometon, Triazaphos, Trichlorphon, Vamidothion;
2. aus der Gruppe der Carbamate
   Aldicarb, 2-sec.-Butylphenylmethylcarbamate (BPMC), Carbaryl, Carbofuran, Carbosulfan, Cloethocarb, Benfuracarb, Ethiofencarb, Furathiocarb, Isoprocarb, Methomyl, 5-Methyl-m-cumenylbutyryl(methyl)carbamate, Oxamyl, Pirimicarb, Propoxur, Thiodicarb, Thiofanox, Ethyl-4,6,9-triaza-4-benzyl-6, 10-dimethyl-8-oxa-7-oxo-5,11 -dithia-9-dodecenoate (OK 135), 1-Methylthio(ethylideneamino)-N-methyl-N-(morpholinothio)carbamate (UC 51717);
3. aus der Gruppe der Carbonsäureester
   Allethrin, Alphametrin, 5-Benzyl-3-furylmethyl-(E)-(1R)-cis, 2,2-di-methyl-3-(2-oxothiolan-3-ylidenemethyl)cyclopropanecarboxylate, Bioallethrin, Bioallethrin((S)-cyclopentylisomer), Bioresmethrin, Biphenate, (RS)-1-Cyano-1-(6-phenoxy-2-pyridyl)methyl-(1RS)-trans-3-(4-tert.butylphenyl)2,2-dimethylcyclopropanecarboxylate (NCI 85193), Cycloprothrin, Cyhalothrin, Cythithrin, Cypermethrin, Cyphenothrin, Deltamethrin, Empenthrin, Esfenvalerate, Fenfluthrin, Fenpropathrin, Fenvalerate, Flucythrinate, Flumethrin, Fluvalinate (D-Isomer), Permethrin, Pheothrin ((R)-Isomer), d-Pralethrin, Pyrethrine (natürliche Produkte), Resmethrin, Tefluthrin, Tetramethrin, Tralomethrin;
4. aus der Gruppe der Amidine
   Amitraz, Chlordimeform;
5. aus der Gruppe der Zinnverbindungen
   Cyhexatin, Fenbutatinoxide;
6. Sonstige
   Abamectin, Bacillus thuringiensis, Bensultap, Binapacryl, Bromopropylate, Buprofezin, Camphechlor, Cartap, Chlorobenzilate, Chlorfluazuron, 2-(4-Chlorphenyl)-4,5-diphenylthiophen (UBI-T 930), Chlorfentezine, Cyclopropancarbonsäure-(2-naphthylmethyl)ester (Ro12-0470), Cyromazin, N-(3,5-Dichlor-4-(1,1,2,3,3,3-hexafluor-1-propyloxy)phenyl)carbamoyl)-2-chlorbenzcarboximidsäureethylester, DDT, Dicofol, N-(N-(3,5-Di-chlor-4-(1,1,2,2-tetrafluorethoxy)phenylaminolcarbonyl)-2,6-difluorbenzamid (XRD 473), Diflubenzuron, N-(2,3-Dihydro-3-methyl-1,3-thiazol-2-ylidene)-2,4-xylidine, Dinobuton, Dinocap, Endosulfan, Ethofenprox, (4-Ethoxyphenyl)(dimethyl)(3-(3-phenoxyphenyl)propyl)silan, (4-Ethoxyphenyl)(3-(4-fluoro-3-phenoxyphenyl) propyl)dimethylsilan, Fenoxycarb, 2-Fluoro-5-(4-(4-ethoxyphenyl)-4-methyl-pentyl)diphenylether (MTI 800), Granulose- und Kernpolyederviren, Fenthiocarb, Flubenzimine, Flucycloxuron, Flufenoxuron, Gamma-HCH, Hexythiazox, Hydramethylnon (AC 217300), Ivermectin, 2-Nitromethyl-4,5-dihydro-6H-thiazin (DS 52618), 2-Nitromethyl-3,4-dihydrothiazol (SD 35651), 2-Nitromethylene-1,2-thiazinan-3-ylcarbamaldehyde (WL 108477), Propargite, Teflubenzuron, Tetradifon, Tetrasul, Thiocyclam, Triflumuron, Imidacloprid.

Der Wirkstoffgehalt der aus den handelsüblichen Formulierungen bereiteten Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Die erfindungsgemäßen Wirkstoffe eignen sich auch zur Bekämpfung von Endo-und Ektoparasiten auf dem veterinärmedizinischen Gebiet bzw. auf dem Gebiet der Tierhaltung.

Die Anwendung der erfindungsgemäßen Wirkstoffe geschieht hier in bekannter Weise wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießen (pour-on and spot-on) und des Einpuderns sowie durch parenterale Anwendung in Form beispielsweise der Injektion.

Die erfindungsgemäßen neuen Verbindungen der Formel I können demgemäß auch besonders vorteilhaft in der Viehhaltung (z. B. Rinder, Schafe, Schweine und Geflügel wie Hühner, Gänse usw.) eingesetzt werden. In einer bevorzugten Ausführungsform der Erfindung werden den Tieren die neuen Verbindungen, gegebenenfalls in geeigneten Formulierungen (vgl. oben) und gegebenenfalls mit dem Trinkwasser oder Futter oral verabreicht. Da eine Ausscheidung im Kot in wirksamer Weise erfolgt, läßt sich auf diese Weise sehr einfach die Entwicklung von Insekten im Kot der Tiere verhindern. Die jeweils geeigneten Dosierungen und Formulierungen sind insbesondere von der Art und dem Entwicklungsstadium der Nutztiere und auch vom Befallsdruck abhängig und lassen sich nach den üblichen Methoden leicht ermitteln und festlegen. Die neuen Verbindungen können bei Rindern z. B. in Dosierungen von 0,01 bis 1 mg/kg Körpergewicht eingesetzt werden.

Die folgenden Beispiele dienen zur Erläuterung der Erfindung, ohne daß diese darauf beschränkt wäre.

### A. Herstellungsbeispiele

### Beispiel a:

4-(4-lsobutyliden-cyclohexylaminol-5-methoxy-6-methoxymethyl-pyrimidin 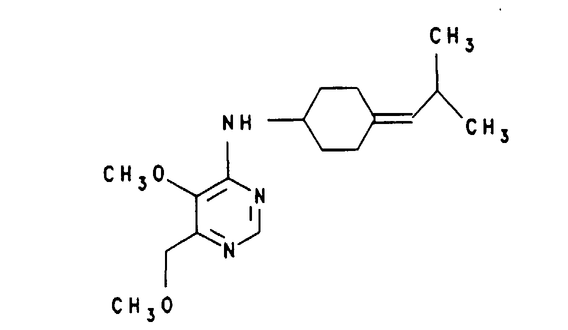 3,8 g (20 mmol) 4-Chlor-5-methoxy-6-methoxymethyl-pyrimidin (DOS 4 116 089), 3,1 g (20 mmol) 4-lsobutyliden-cyclohexylamin und 3,0 g (30 mmol) Triethylamin wurden 3 h ohne Lösungsmittel bei 80 - 90°C gerührt. Nach Abkühlen wurde mit Wasser/Toluol aufgenommen und die organische Phase getrocknet und einrotiert. Nach chromatographischer Reinigung (Kieselgel/Ethylacetat) wurden 2,2 g (36 % d.Th.) eines fast farblosen Öls erhalten.

### Herstellung des Ausgangsproduktes 4-lsobutyliden-cyclohexylamin

26,7 g (0,18 mol) 4-Isobutylidencyclohexanon (hergestellt aus Isobutyltriphenylphosphoniumbromid/Natriumhydrid und Cyclohexan-1,4-dionmonoethylenketal in DMSO und anschließende Verseifung des Ketals) wurde in 200 ml mit Ammoniak gesättigtem Methanol in Gegenwart von 6 g Raney Nickel bei 100°C und 100 bar Wasserstoffdruck reduktiv aminiert. Nach Abfiltrieren vom Katalysator und Abziehen des Methanols wurde das Rohprodukt am Dünnschichtverdampfer destilliert (135 °C/0,6 bar). Man erhielt 16,5 g (61 % d.Th.) eines schwach gelben Ols.

### Beispiel b:

4-(4-Hexyliden-cyclohexyloxy)-chinazolin

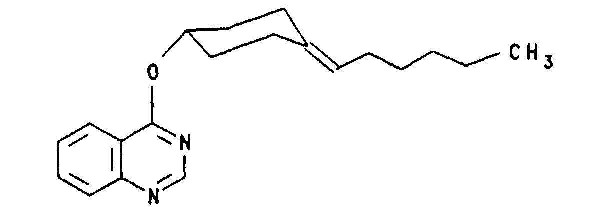

Zu einer Lösuing von 0,7 g (3,5 mmol) 4-Hexylidencyclohexanol in 20 ml abs. THF werden 130 mg (4,3 mmol) 80 %iges NaH gegeben und zur vollständigen Deprotonierung 1 Stunde refluxiert. Zur Reaktionslösung werden anschließend in der Siedehitze 0,6 g (3,6 mmol) 4-Chlorchinazolin zugegeben und 3 Stunden auf Rückfluß erhitzt. Zur Aufarbeitung gibt man 2ml Isopropanol hinzu. Nach Abkühlen wird mit wäßriger NH₄Cl/Ether aufgenommen und die organische Phase über M_{g}SO₄ getrocknet. Nach Einengen des Lösungsmittels erhält man 1,1 g (Rohprodukt, das durch Flash-Chromatographie (Kieselgel/Petrolether:Ethylacetat = 2:1) gereinigt wird).
Ausbeute: 0,75 g (65 % d.Th.) gelbliches Öl.

### Beispiel c:

4-[4-(n-Butoximino)-cyclohexyloxy]-5,6,7,8-tetrahydrochinazolin

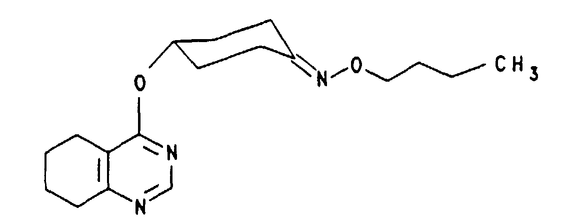

Zu einer Lösung von 1,13 g (3,8 mmol) 4-[4-(Hydroxylimino)cyclohexyloxy]-5,6,7,8-tetrahydrochinazolin in 30 ml abs. THF gibt man 140 mg (4,6 mmol) 80 %iges Natriumhydrid und rührt ca. 15 min bei RT bis keine Wasserstoffentwicklung mehr zu beobachten ist. Zur Reaktionslösung tropft man 0,9 ml (8,2 mmol) n-Butylbromid und refluxiert bis zur vollständigen Umsetzung.

Zur Aufarbeitung gibt man nach Abkühlen 1 - 2 ml Isopropanol zu, rührt nochmals 15 min. weiter und gießt die Reaktionsmischung auf 50 ml einer wäßrigen Ammoniumchloridlösung. Die wäßrige Phase wird mit Ether extrahiert und die vereinigten organischen Phasen über MgSO₄ getrocknet. Nach Einengen verbleiben 1,4 g Rohprodukt, die durch Flash-Chromatographie (Kieselgel,Ethylacetat) gereinigt werden. Ausbeute: 1.0 g (75 % d.Th.) gelbliches Öl.

### Herstellung des Ausgangsproduktes 4-[4-Hydroxylimino)cyclohexyloxy]-5,6,7,8-tetrahydrochinazolin

Zu einer Lösung von 3,8 g (15.5 mmol) 4-(Cyclohexanon-4-oxyl-5,6,7,8-tetrahydrochinazolin in 50 ml destilliertem Ethanol gibt man zuerst 2,9 g (34,2 mmol) Natriumacetat und anschließend 2,2 g (31,0 mmol) Hydroxylammoniumchlorid. Das Reaktionsgemisch wird 4 Stunden refluxiert und anschließend das auskristllisierte Produkt (1,8 g) abgesaugt.

Das Filtrat wird eingeengt, mit 50 ml Ethylacetat aufgenommen und mit verd. Ammoniumchloridlösung gewaschen. Die wäßrige Phase wird gründlich mit Ethylacetat gewaschen und die organische Phase über MgSO₄ getrocknet. Nach Einengen werden weitere 1,2 g sauberes Produkt erhalten. Ausbeute: 3,0 g (65 % d.Th.) gelbliche Kristalle, Smp. 182°C.

### Herstellung des Ausgangsproduktes 4-(Cyclohexanon-4-oxy)-5,6,7,8-tetrahydrochinazolin

Zu einer Lösung von 5,7 g (36,0 mmol) 4-Hydroxycyclohexanon-ethylenketal in 50 ml abs. THF gibt man 1,20 g (40,0 mmol) 80 % NaH und erhitzt 1 Stunde auf Rückfluß. Danach kühlt man die Reaktionslösung auf ca. 35°C und tropft 6,0 g (36,0 mmol) 4-Chlor-5,6,7,8-tetrahydrochinazolin, gelöst in 30 ml THF, zu. Die Reaktionslösung wird erneut 4 Stunden refluxiert.

Zur Aufarbeitung werden 10 ml Isopropanol zugetropft und die Reaktionsmischung auf 2N HCI gegossen und bis zur vollständigen Abspaltung der Schutzgruppe gerührt. Anschließend neutralisiert man mit NaHCO₃-Lösung und extrahiert die wäßrige Phase mit Et₂O. Die organische Phase wird über MgSO₄ getrocknet und anschließend einrotiert. Der Rückstand (7,2 g) wird durch Flash-Chromatographie (Kieselgel, Ethylacetat) gereinigt. Ausbeute: 4,8 g (56 % d.Th.) farbloses Öl.

### Herstellung des Ausgangsproduktes 4-Hydroxycyclohexanon-ethylenketal.

Zu einer Lösung von 50 g (0,25 mmol) 1,4-Cyclohexandionmonoethylenketal werden 3,6 g (95 mmol) NaBH₄ gegeben und bis zur vollständigen Umsetzung gerührt. Zur Aufarbeitung werden 30 ml Aceton zugegeben und weitere 30 min gerührt. Anschließend engt man bis zur Trockne ein und nimmt den Rückstand in 200 ml Ether auf, wäscht die organische Phase mit wäßriger NH₄Cl-Lösung und Wasser und trocknet die organische Phase über MgSO₄. Anschließend wird das Lösungsmittel einrotiert und der Rückstand getrocknet. Ausbeute: 46,0 g (91 % d.Th.) farbloses Öl

Die Verbindungen der Tabellen 2 bis 6 wurden in analoger Weise hergestellt. In Tabelle 1 sind Substituentenbedeutungen Tⁿ definiert.

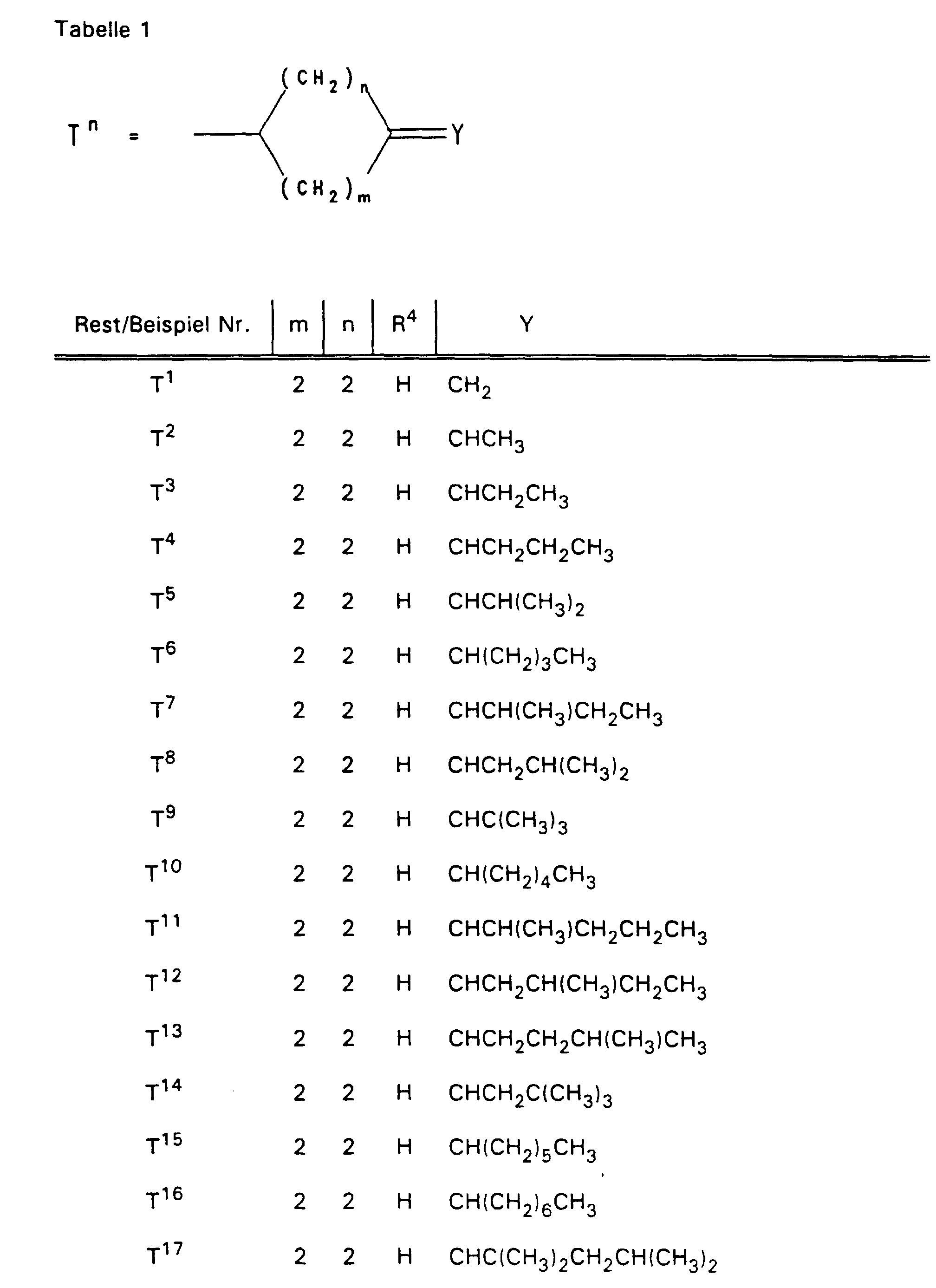

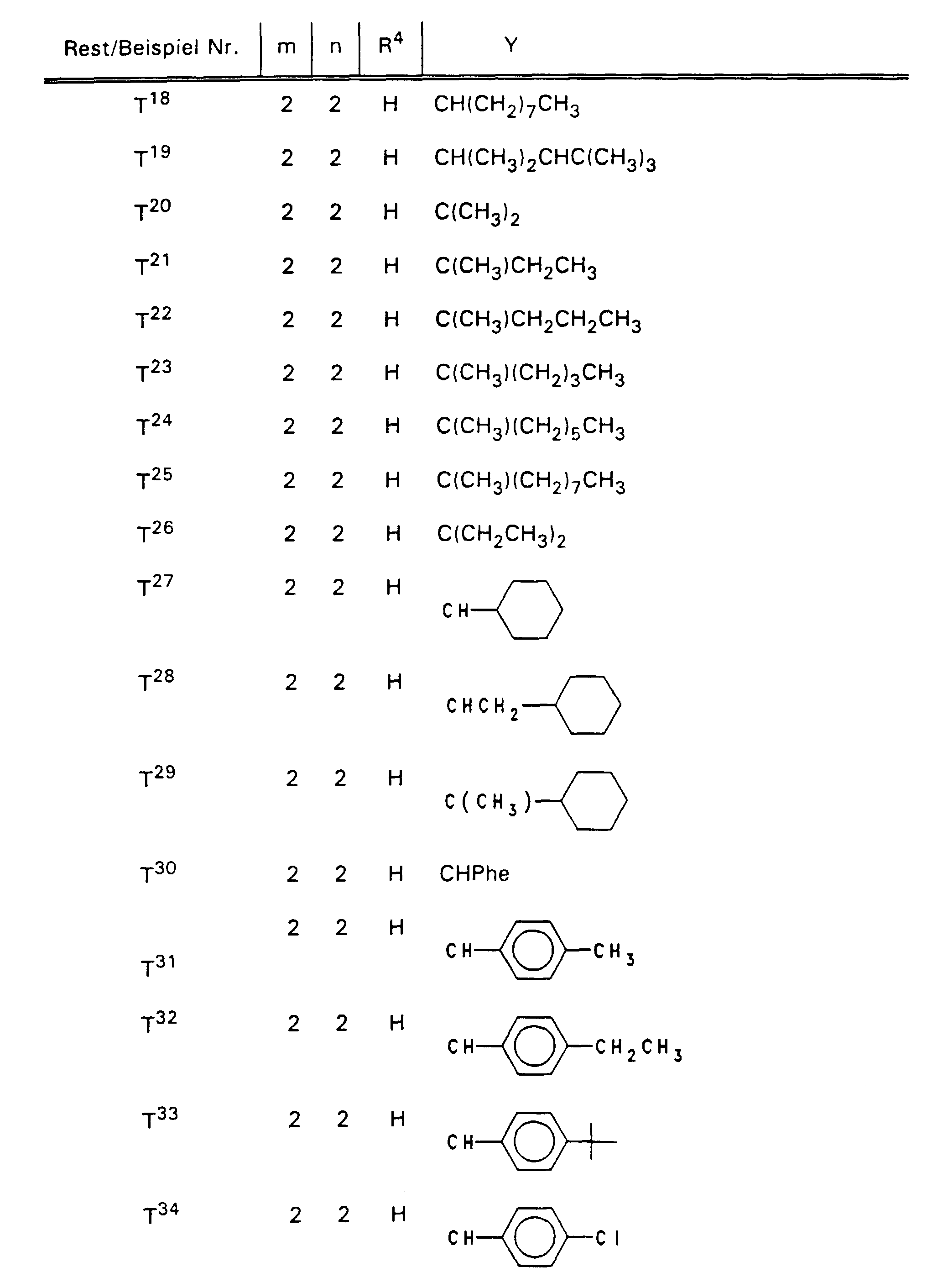

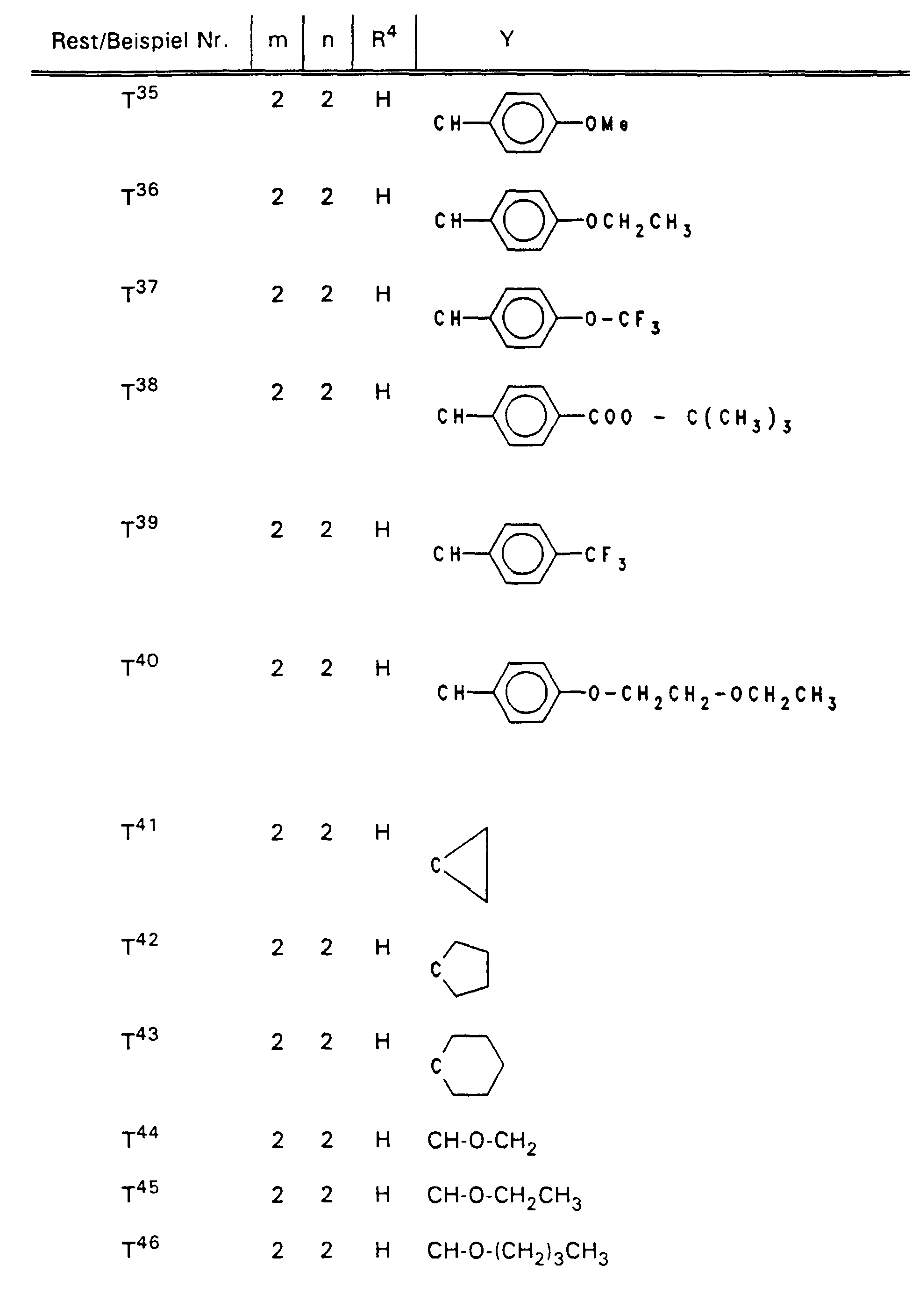

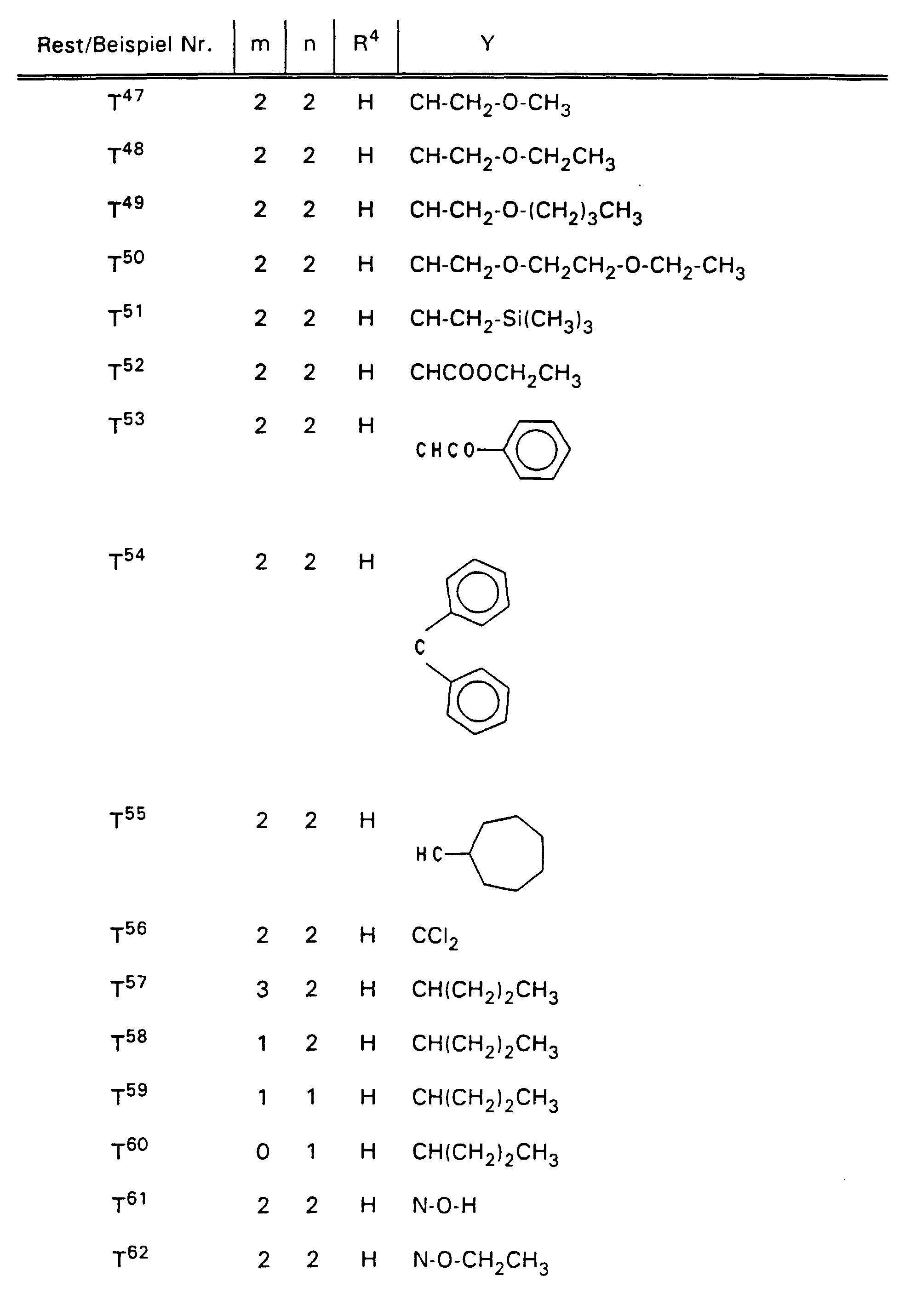

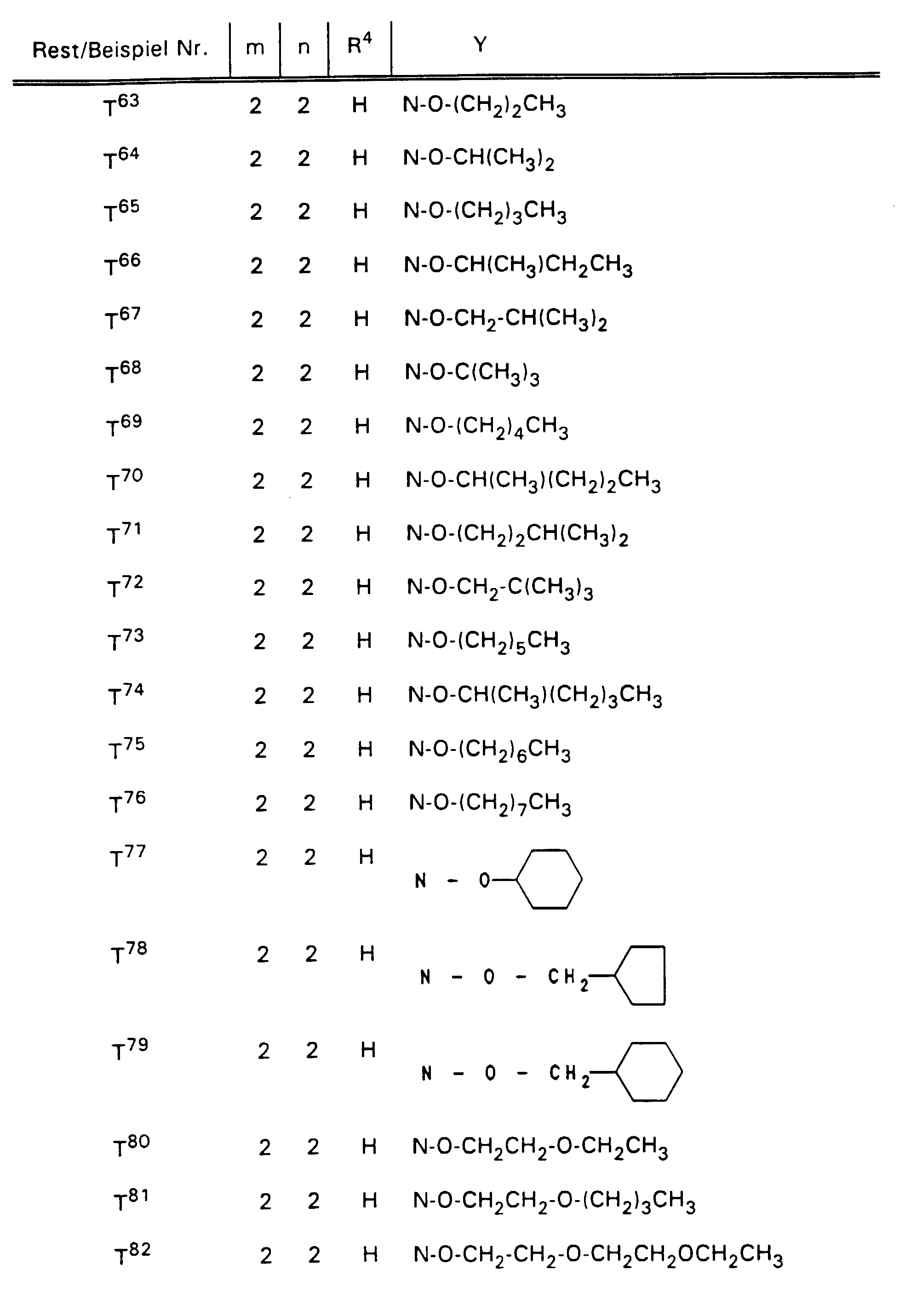

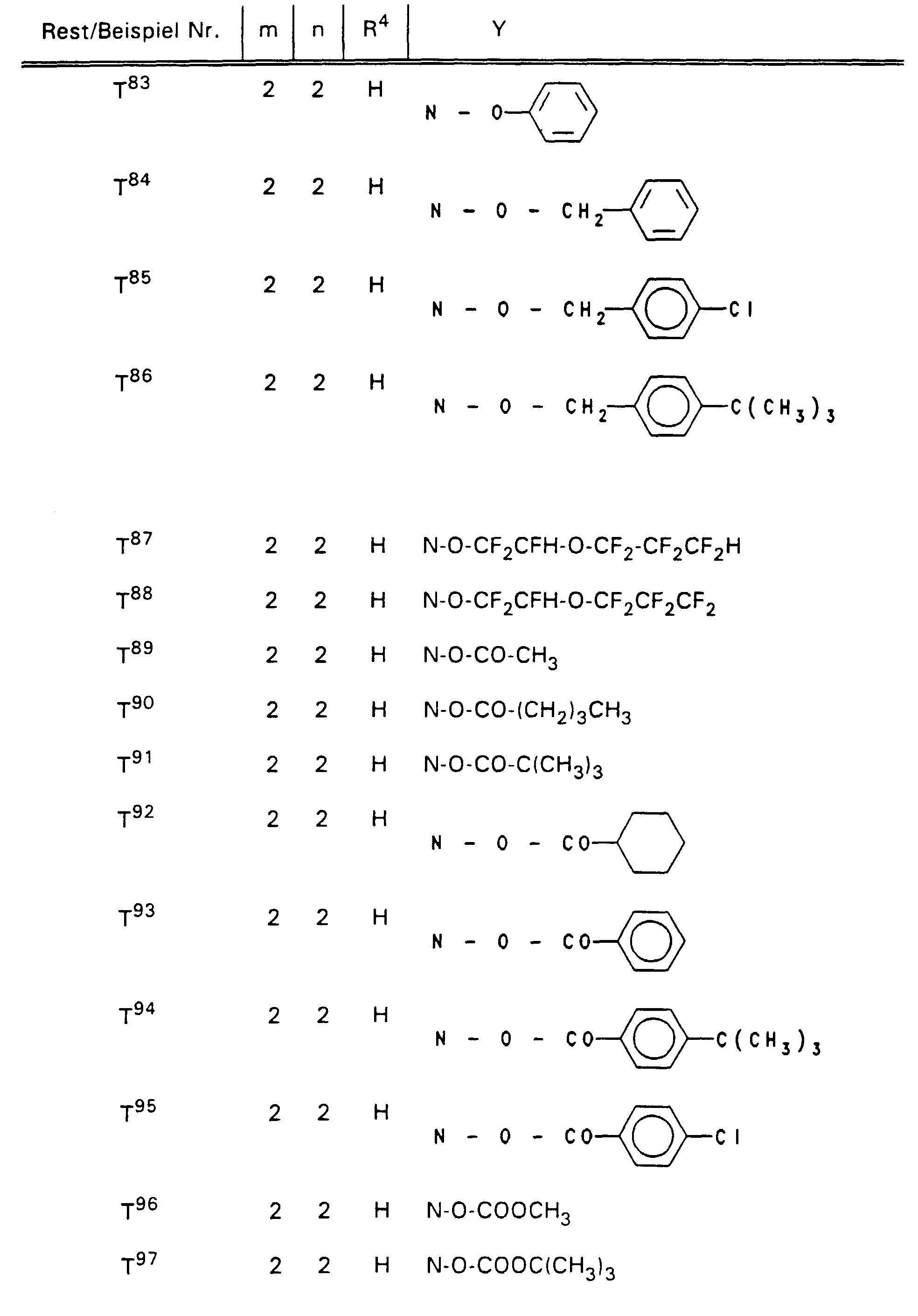

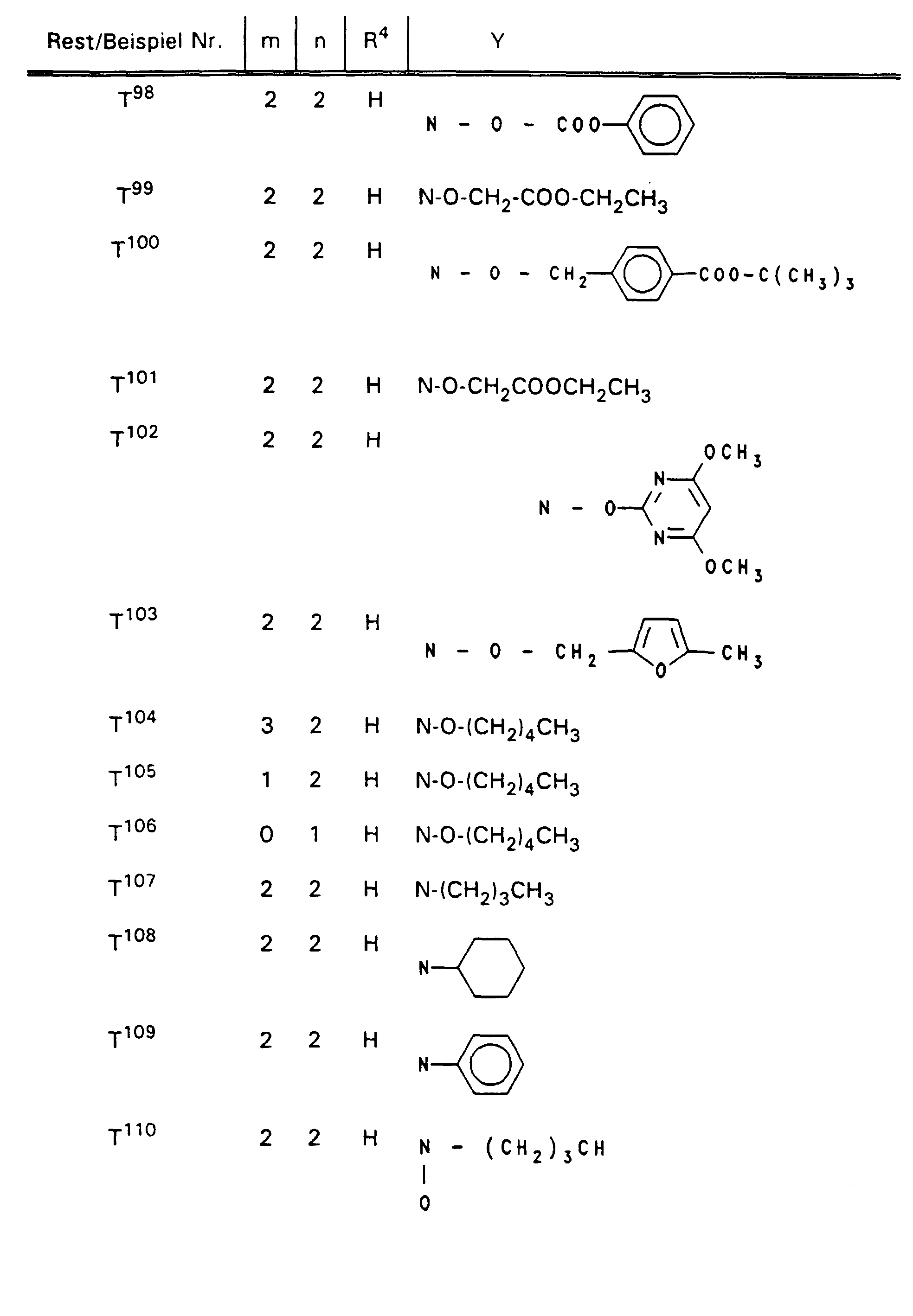

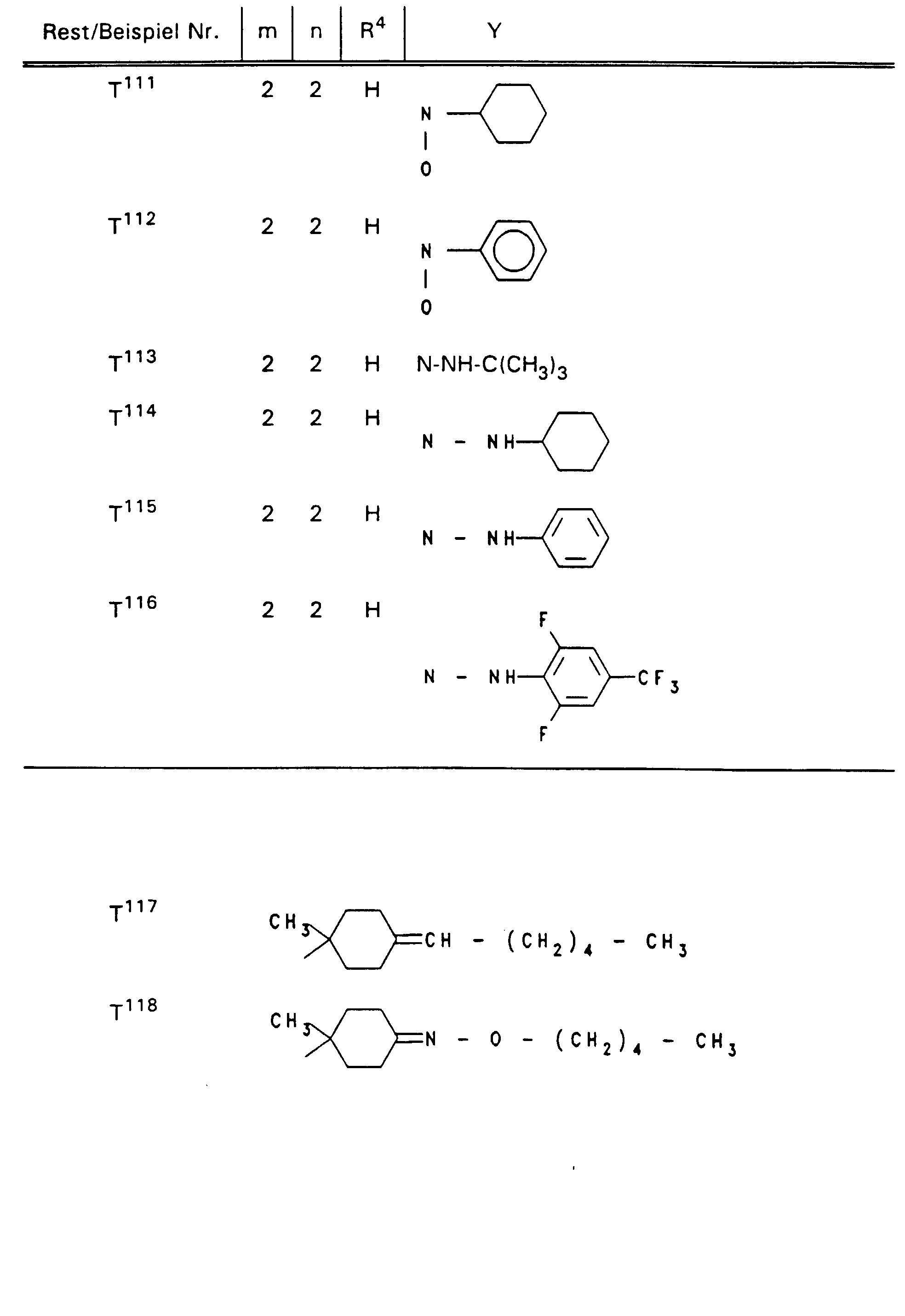

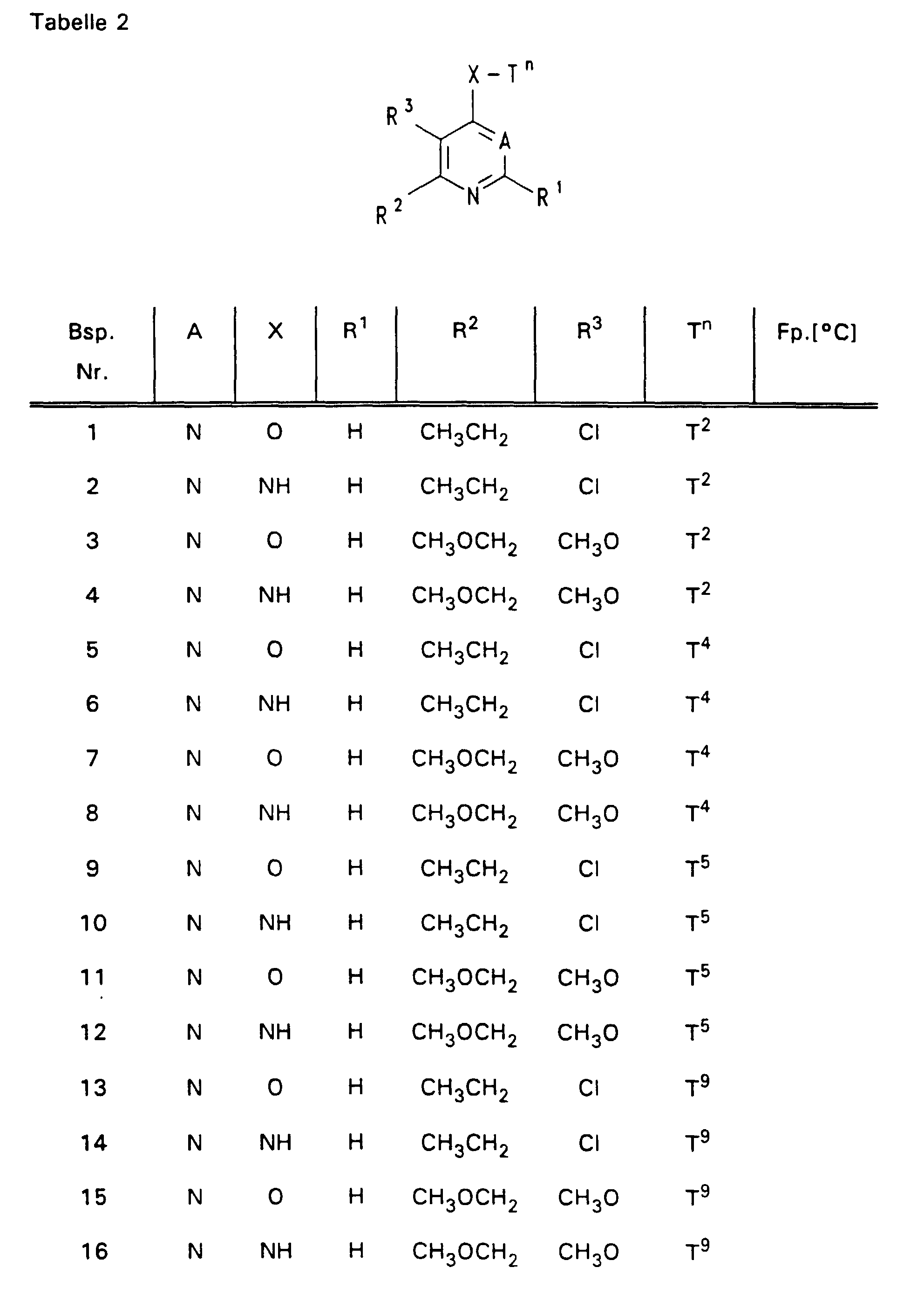

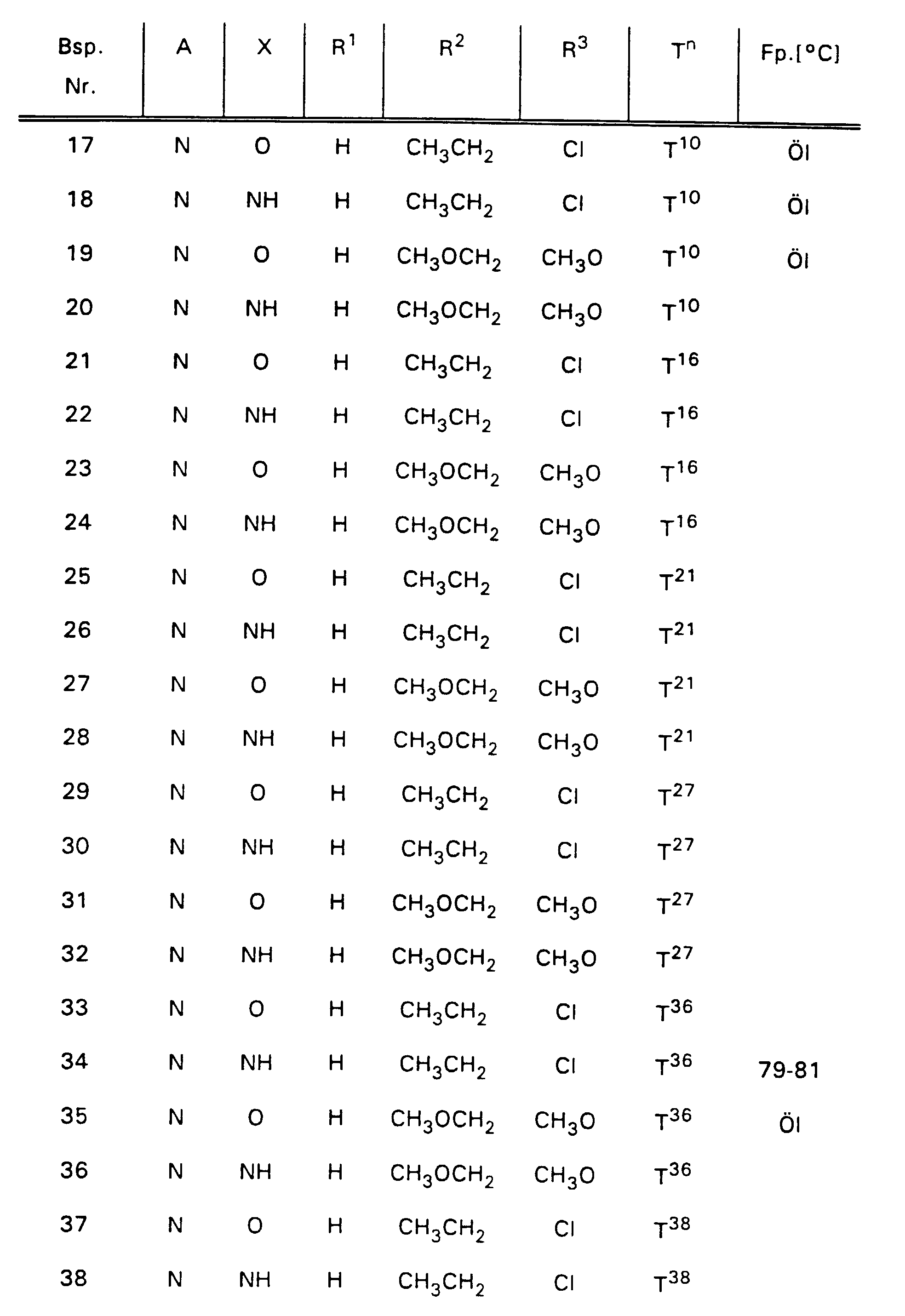

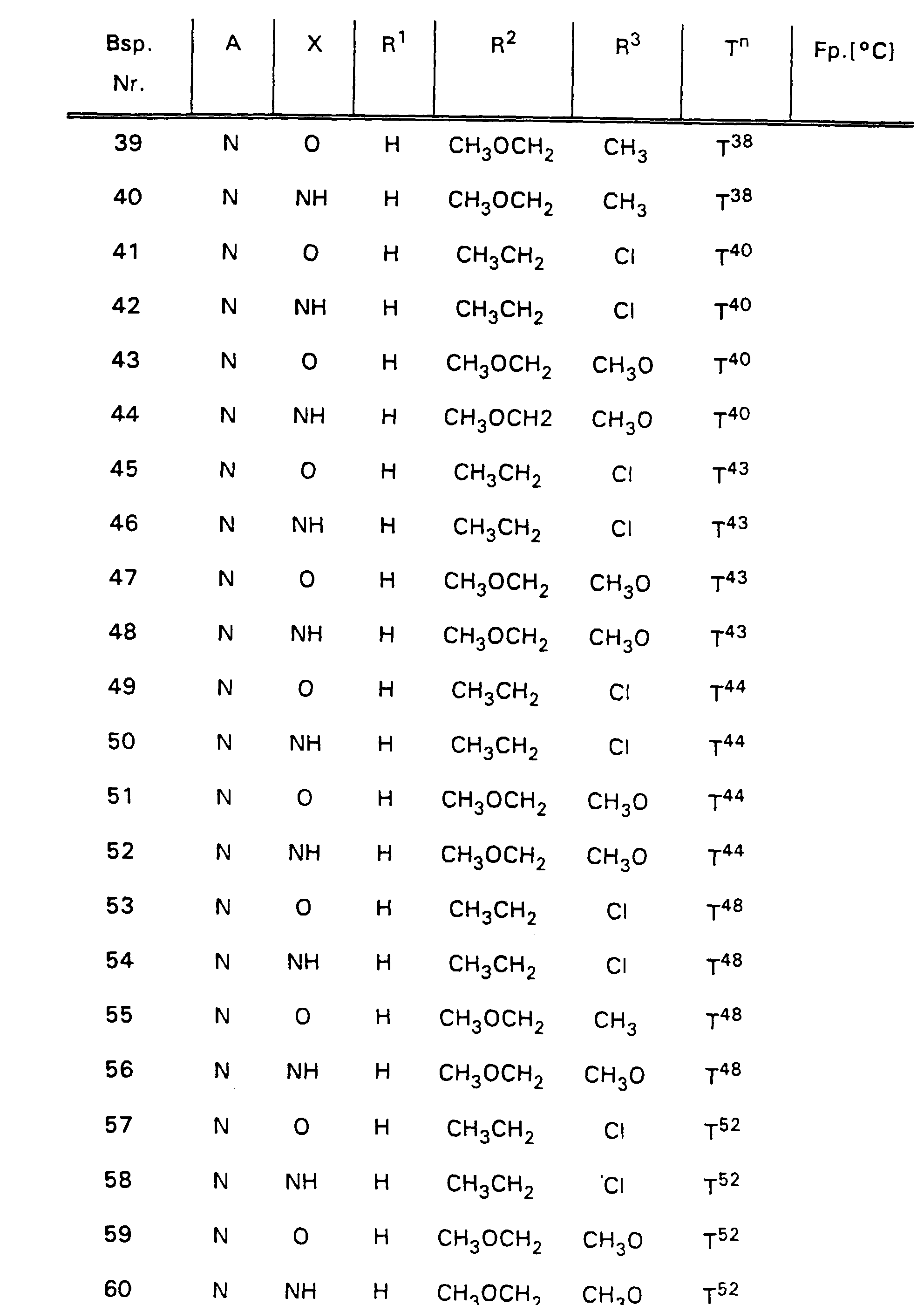

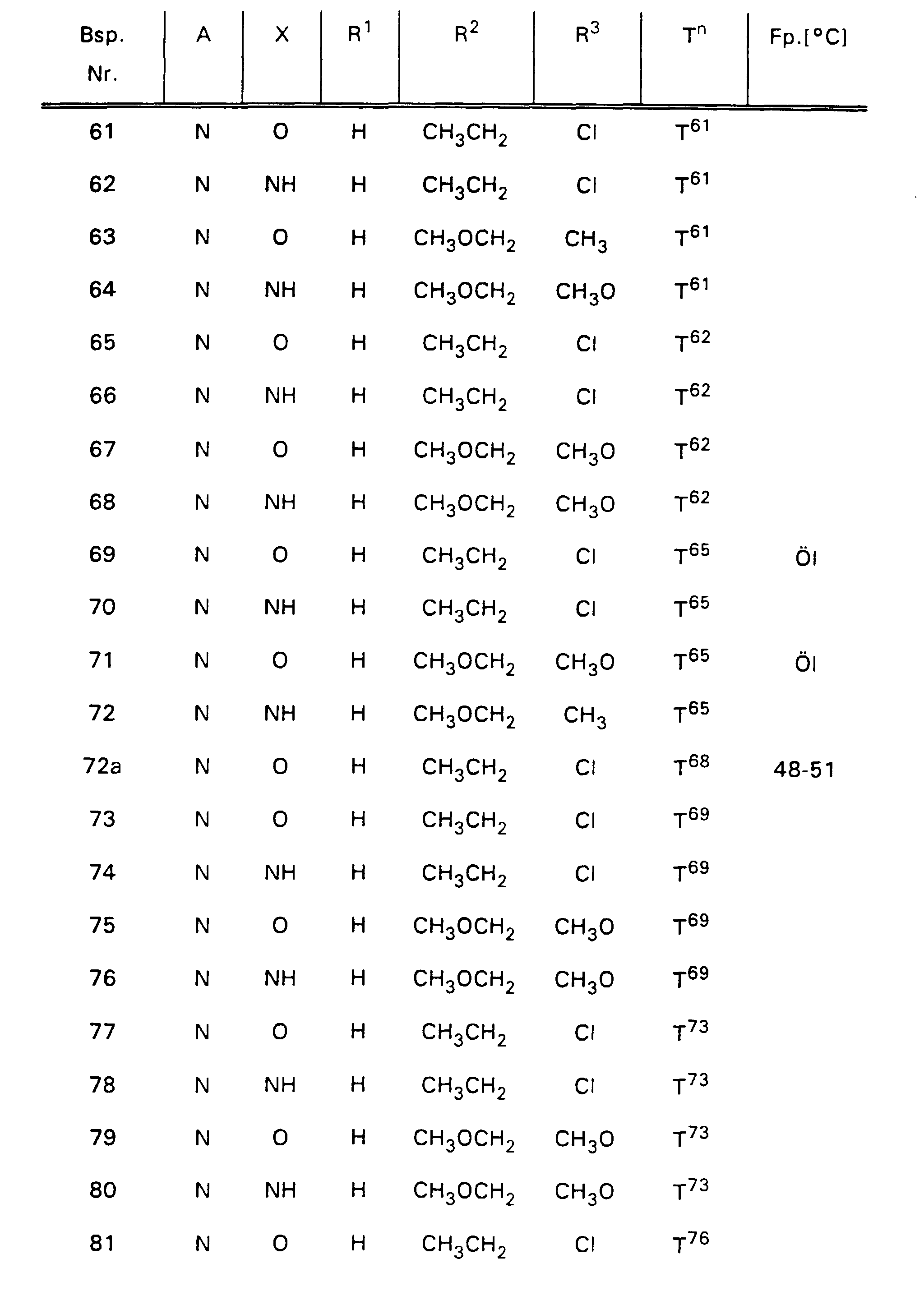

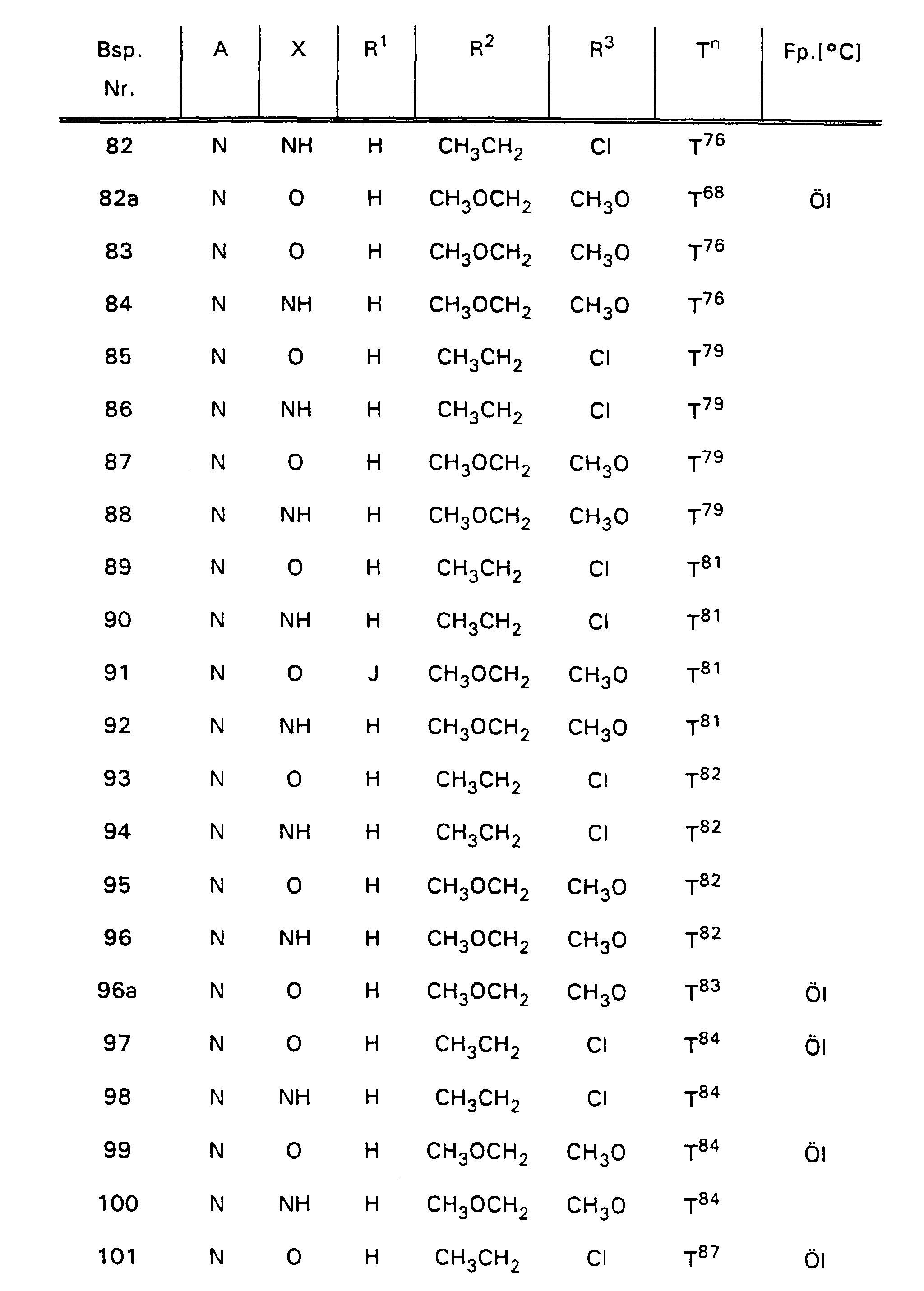

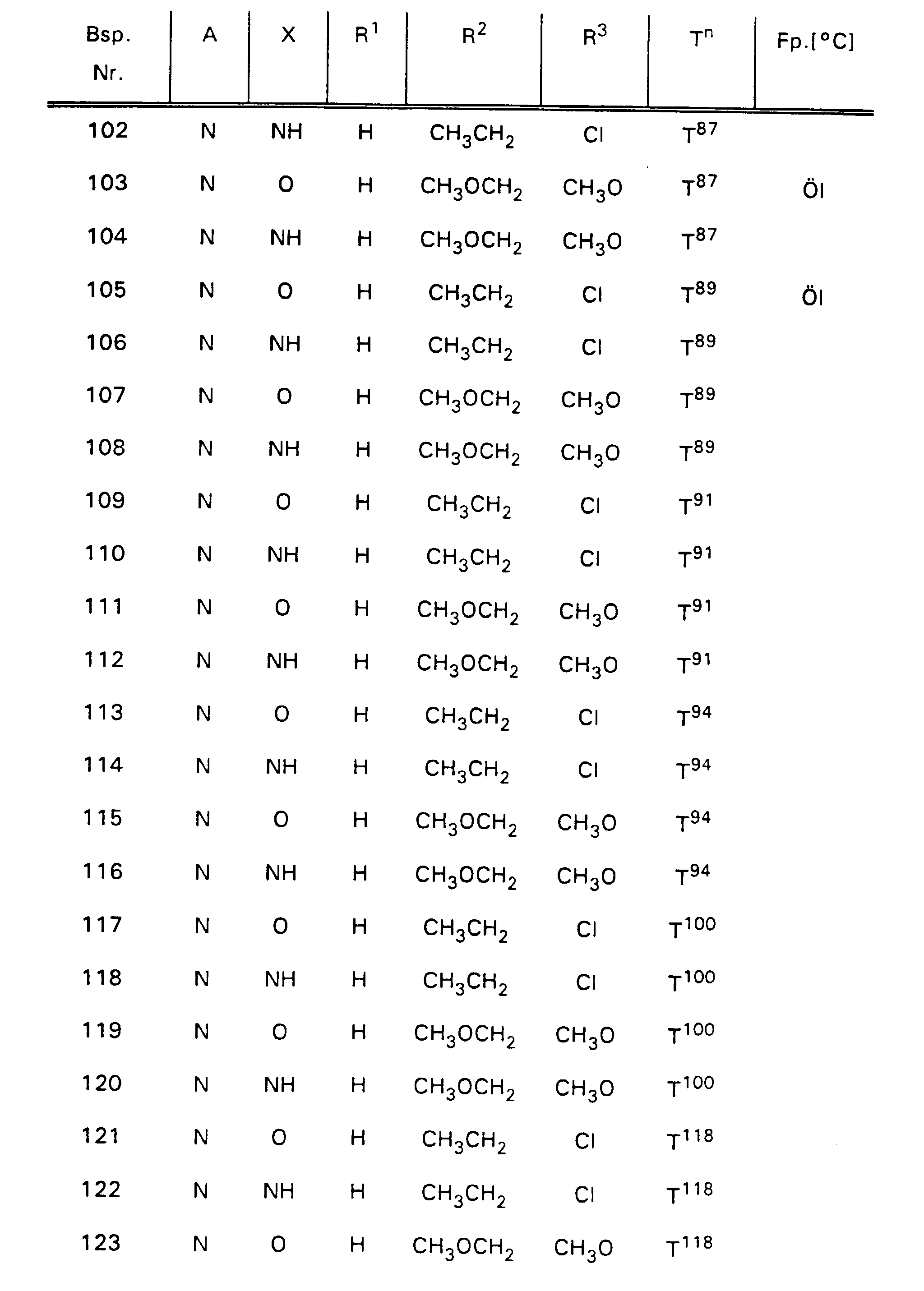

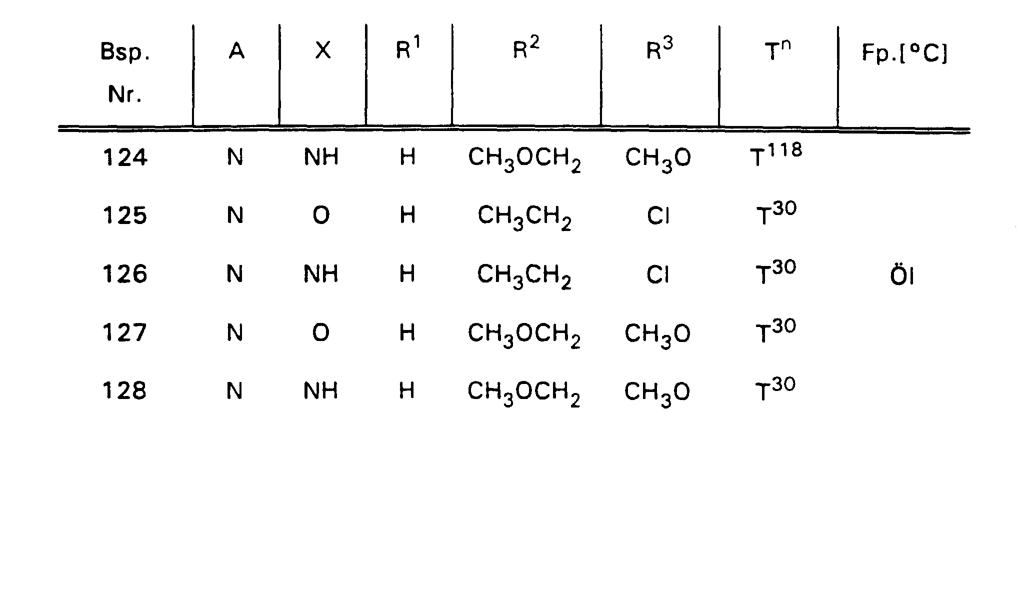

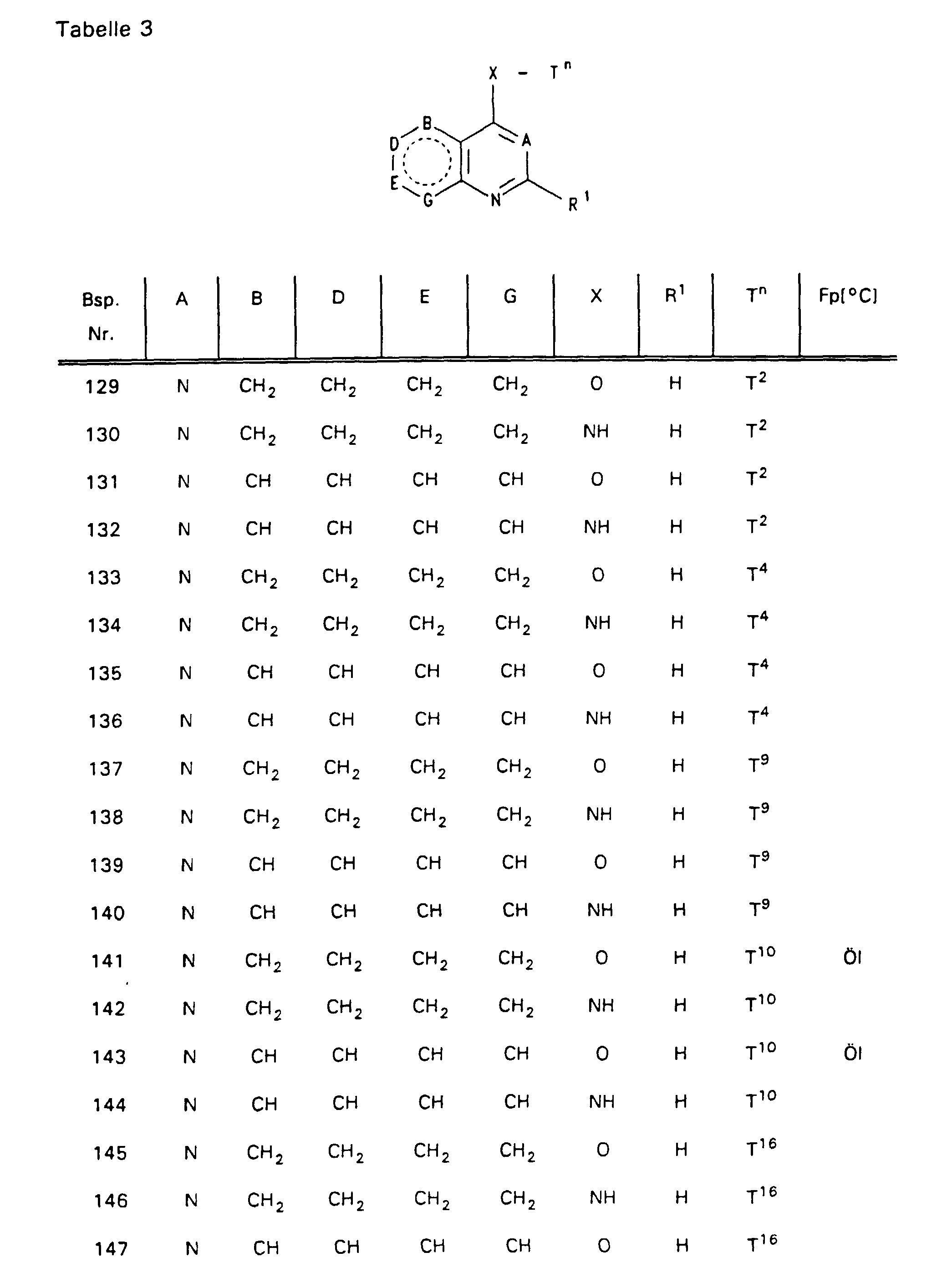

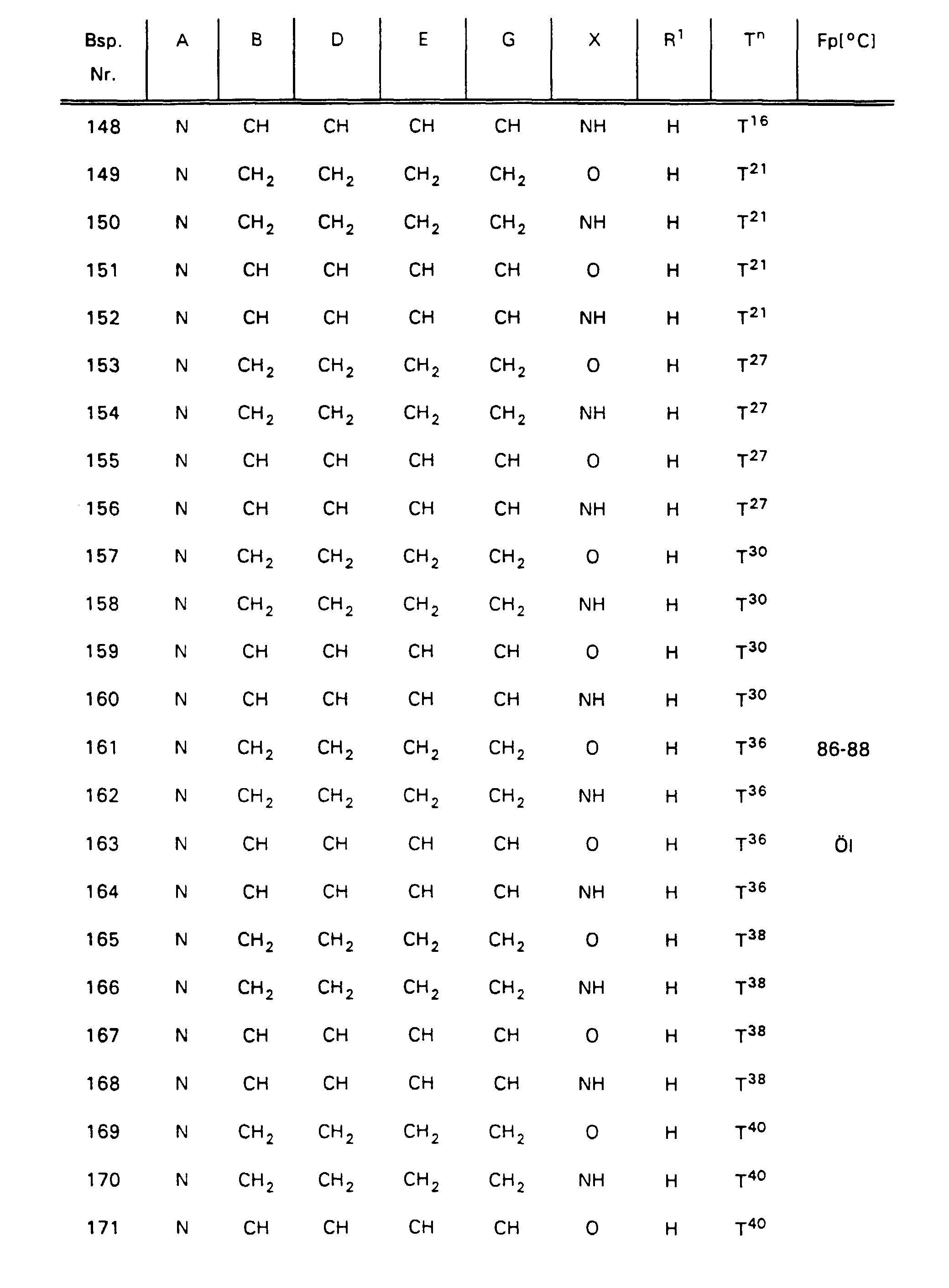

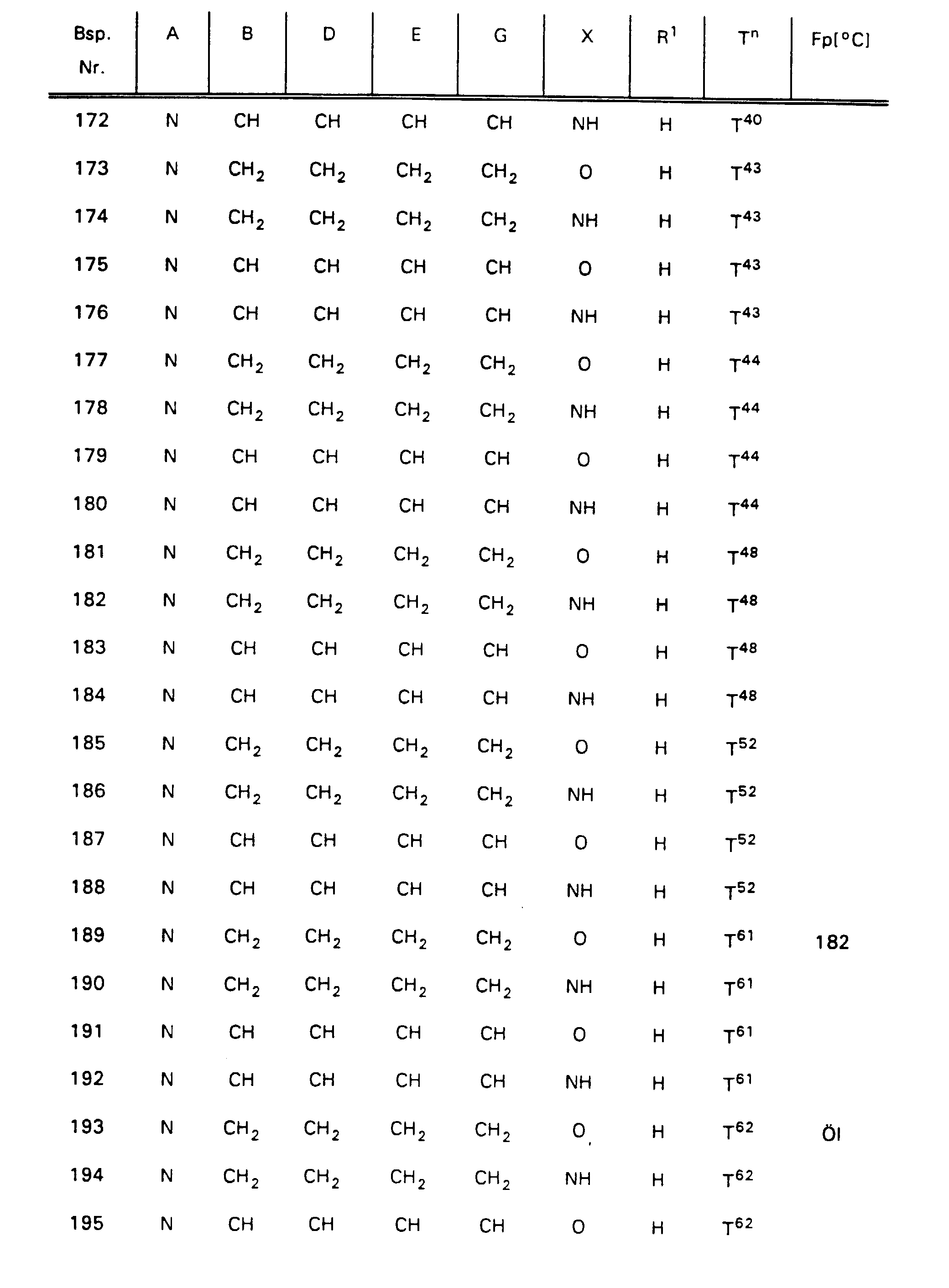

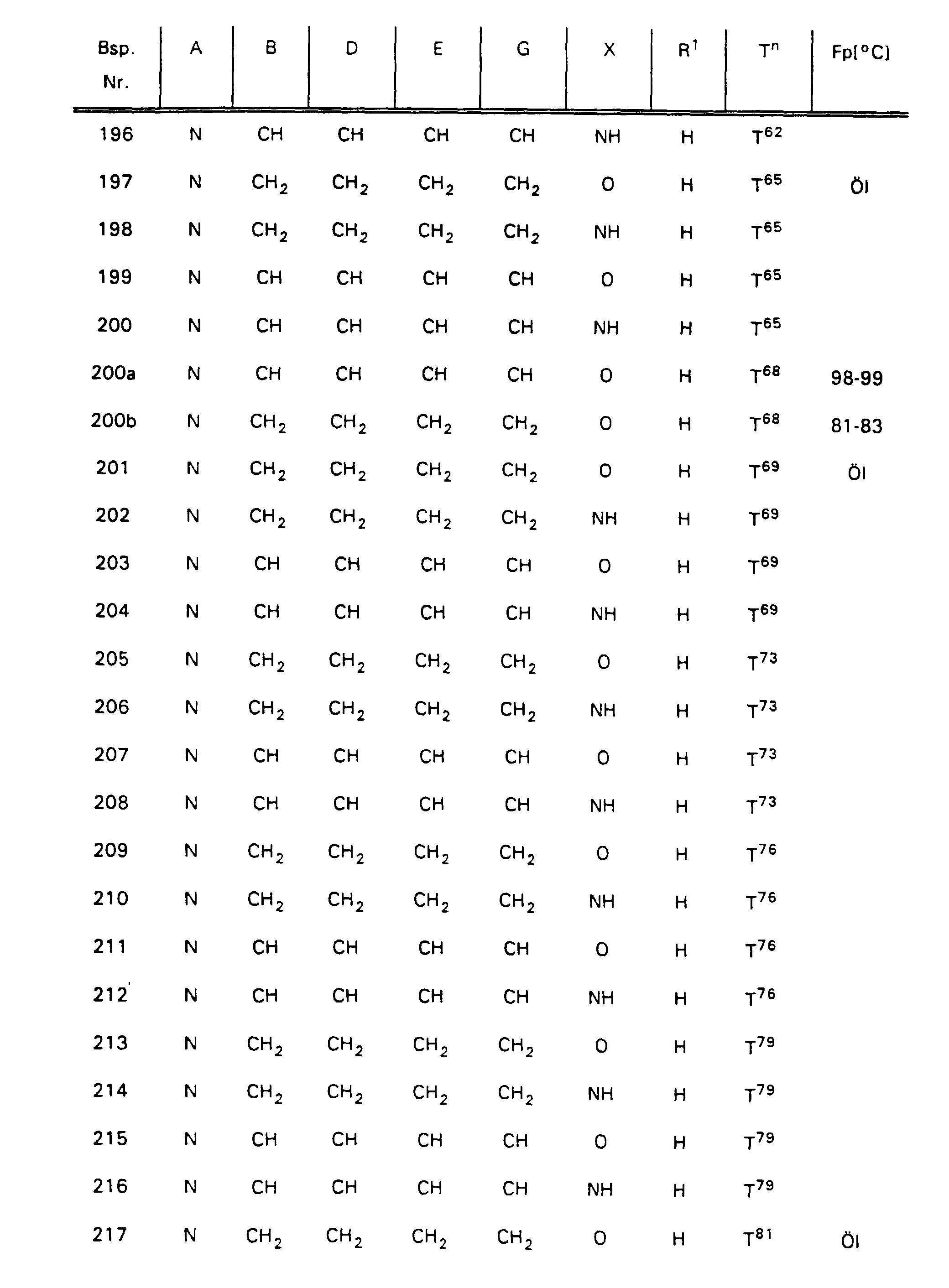

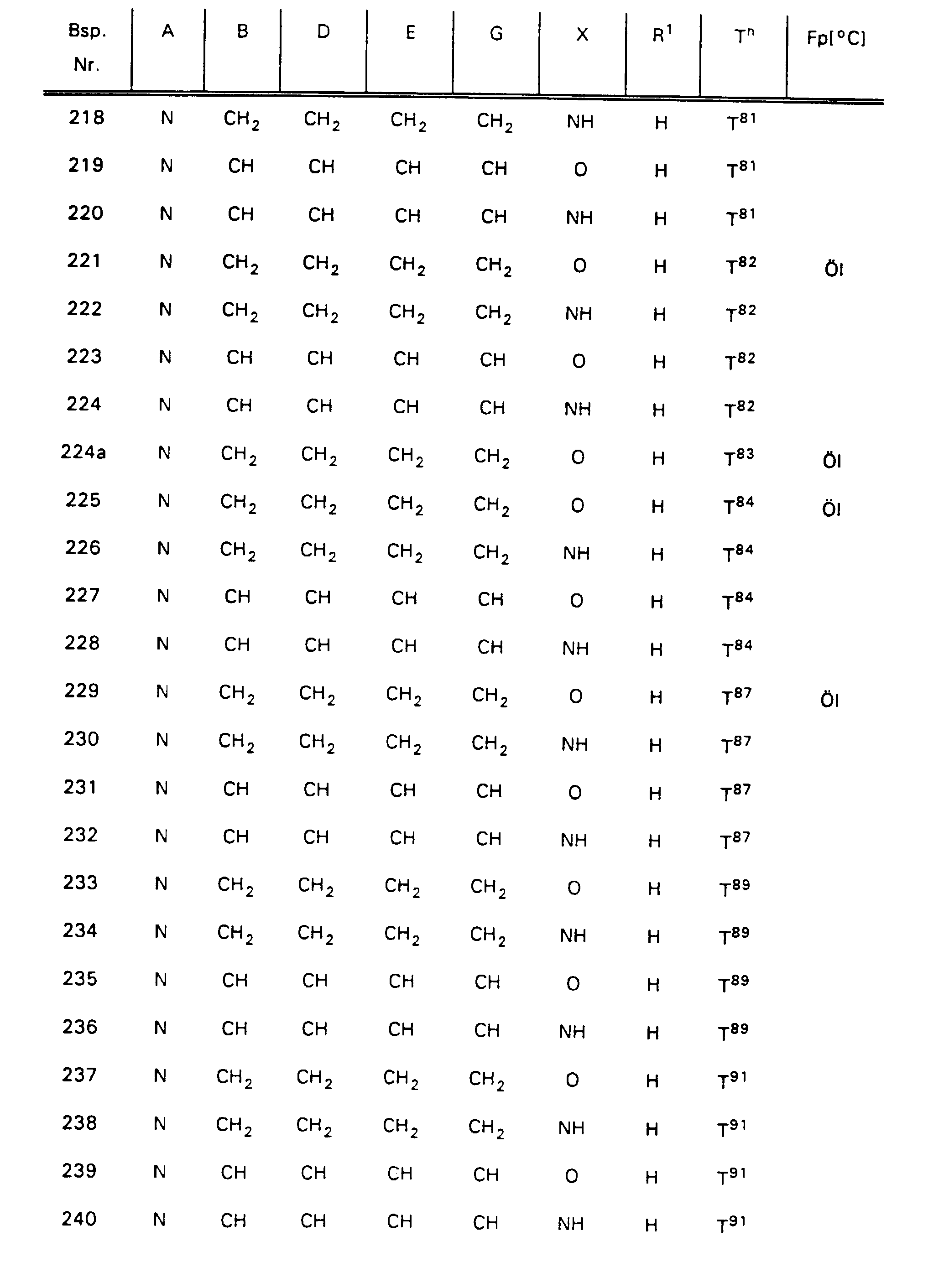

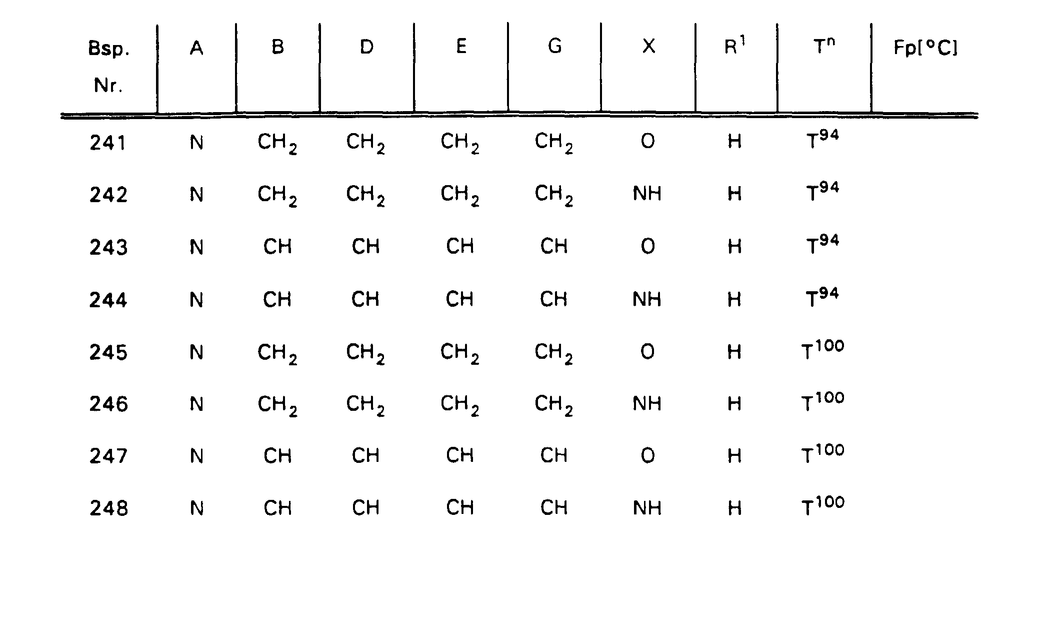

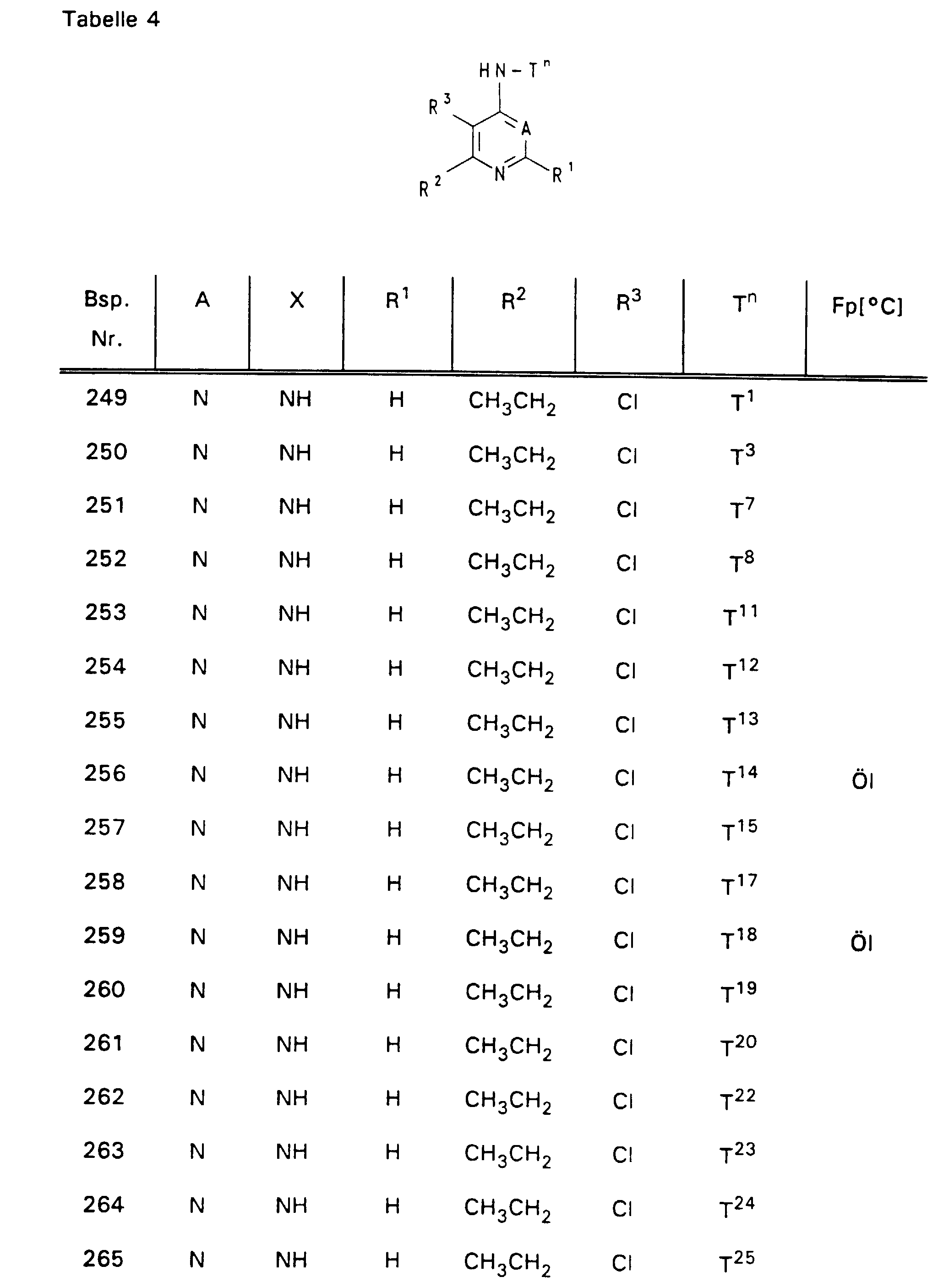

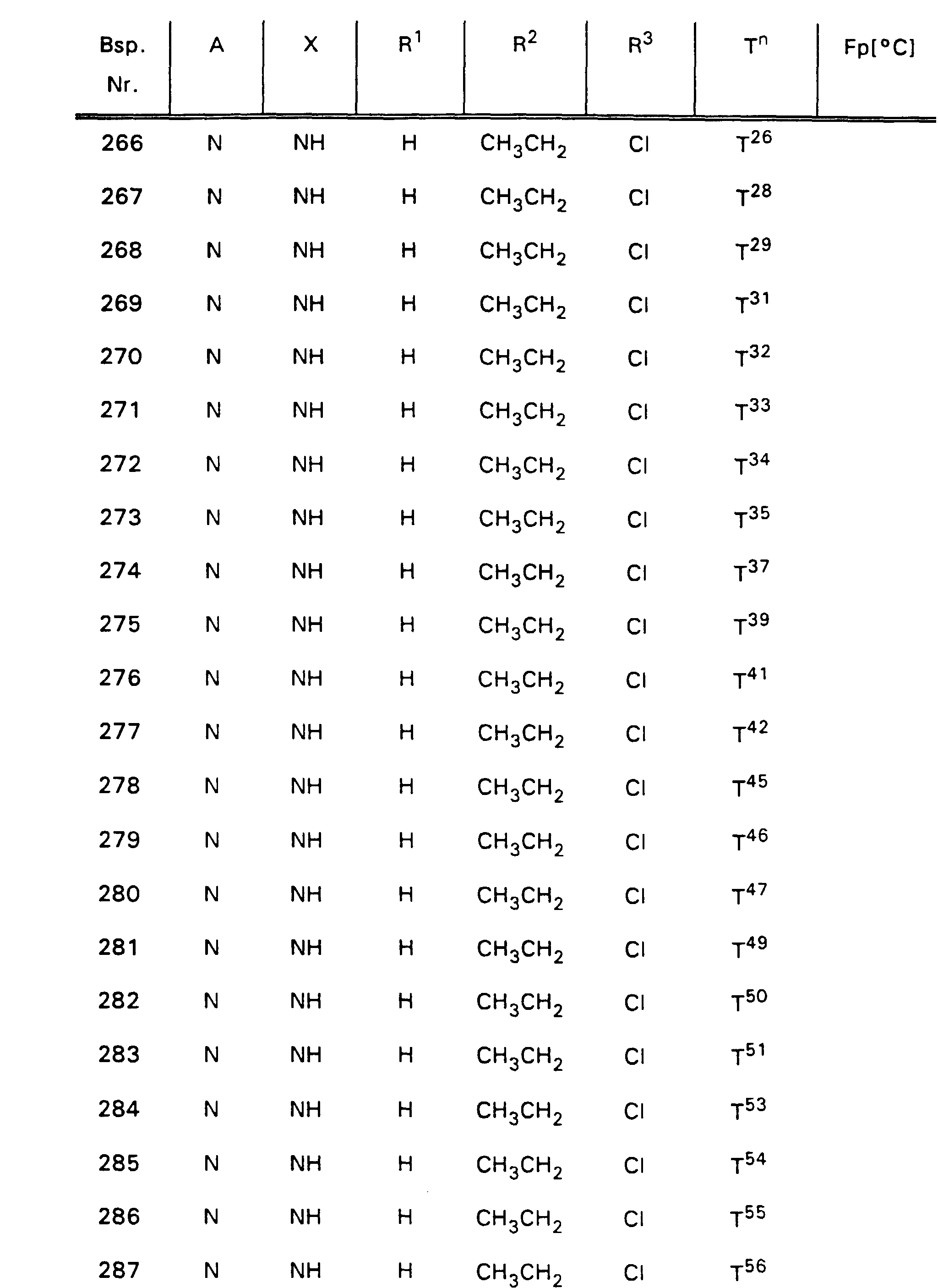

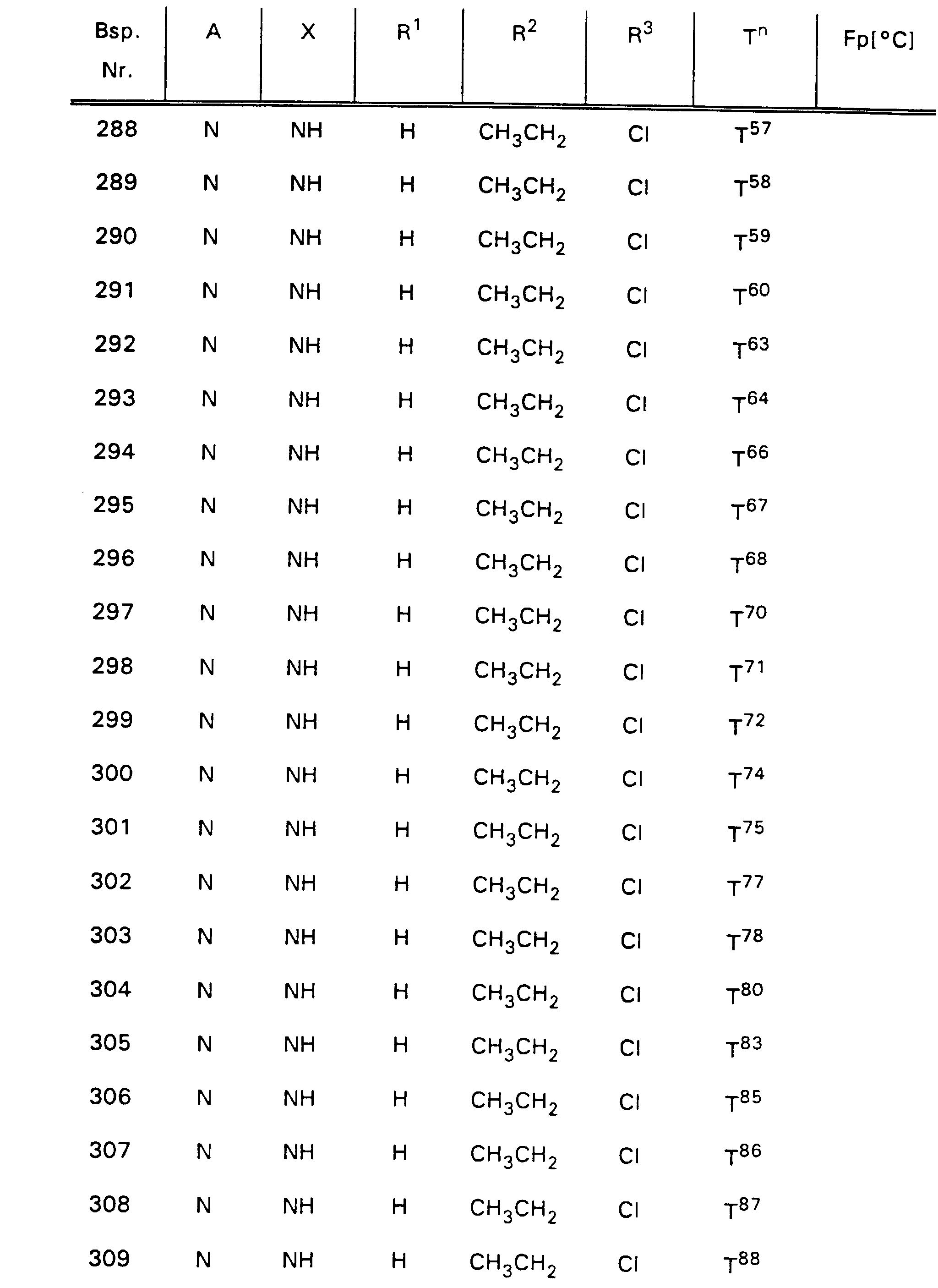

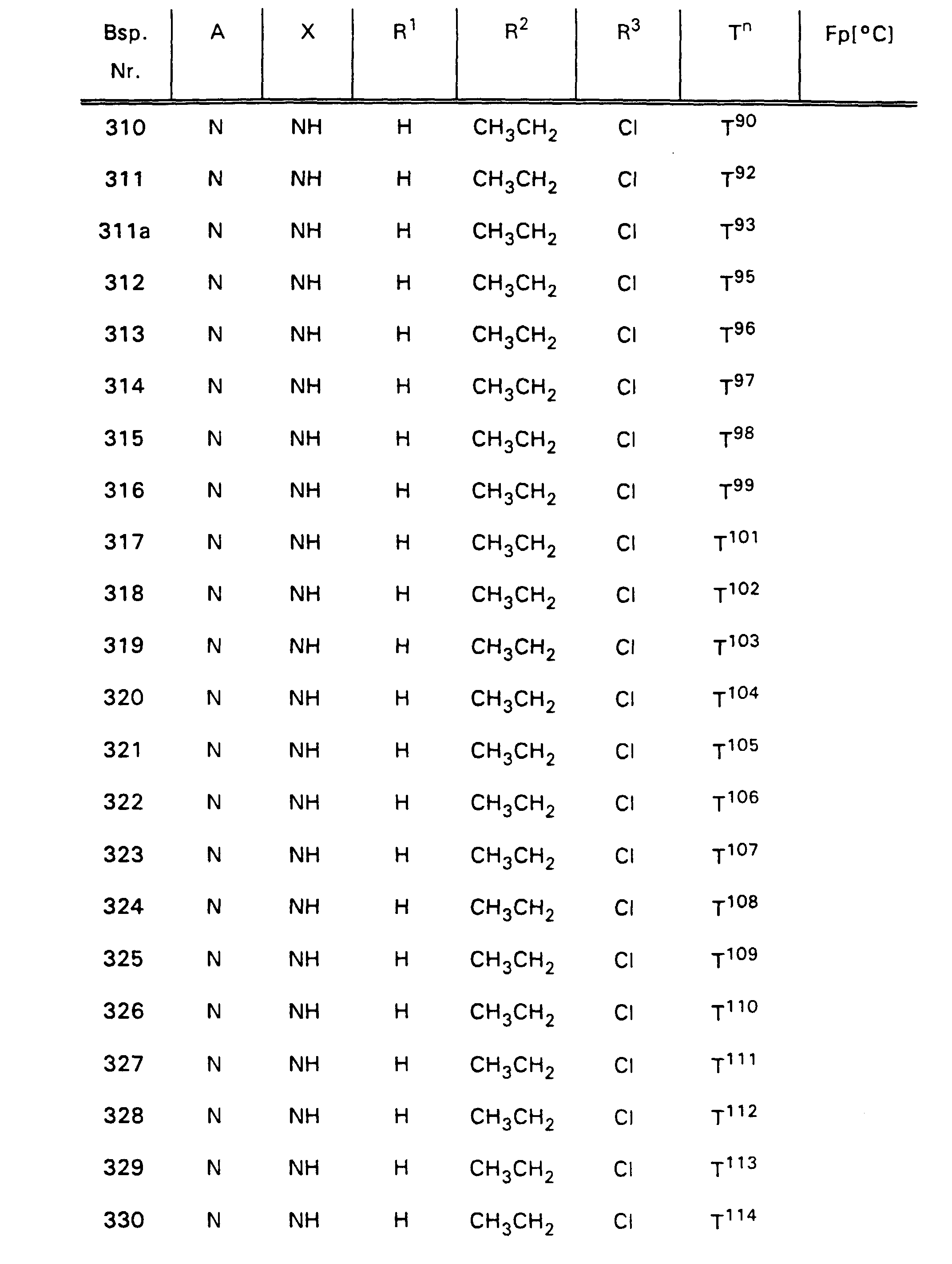

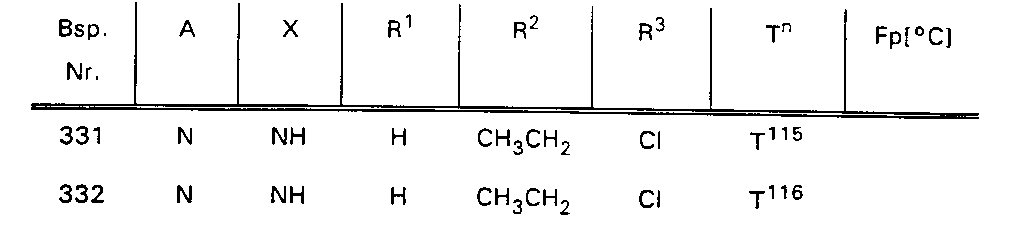

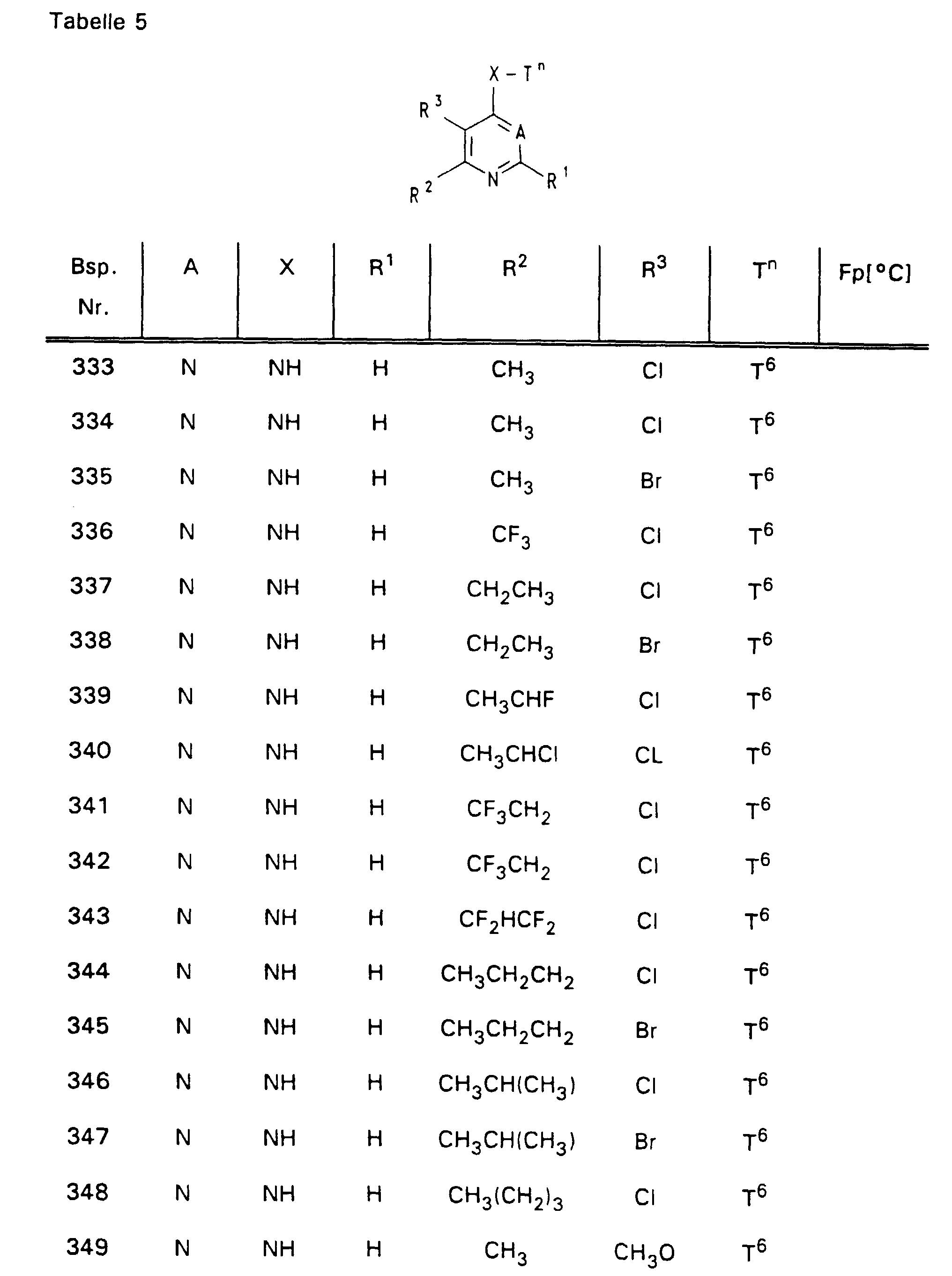

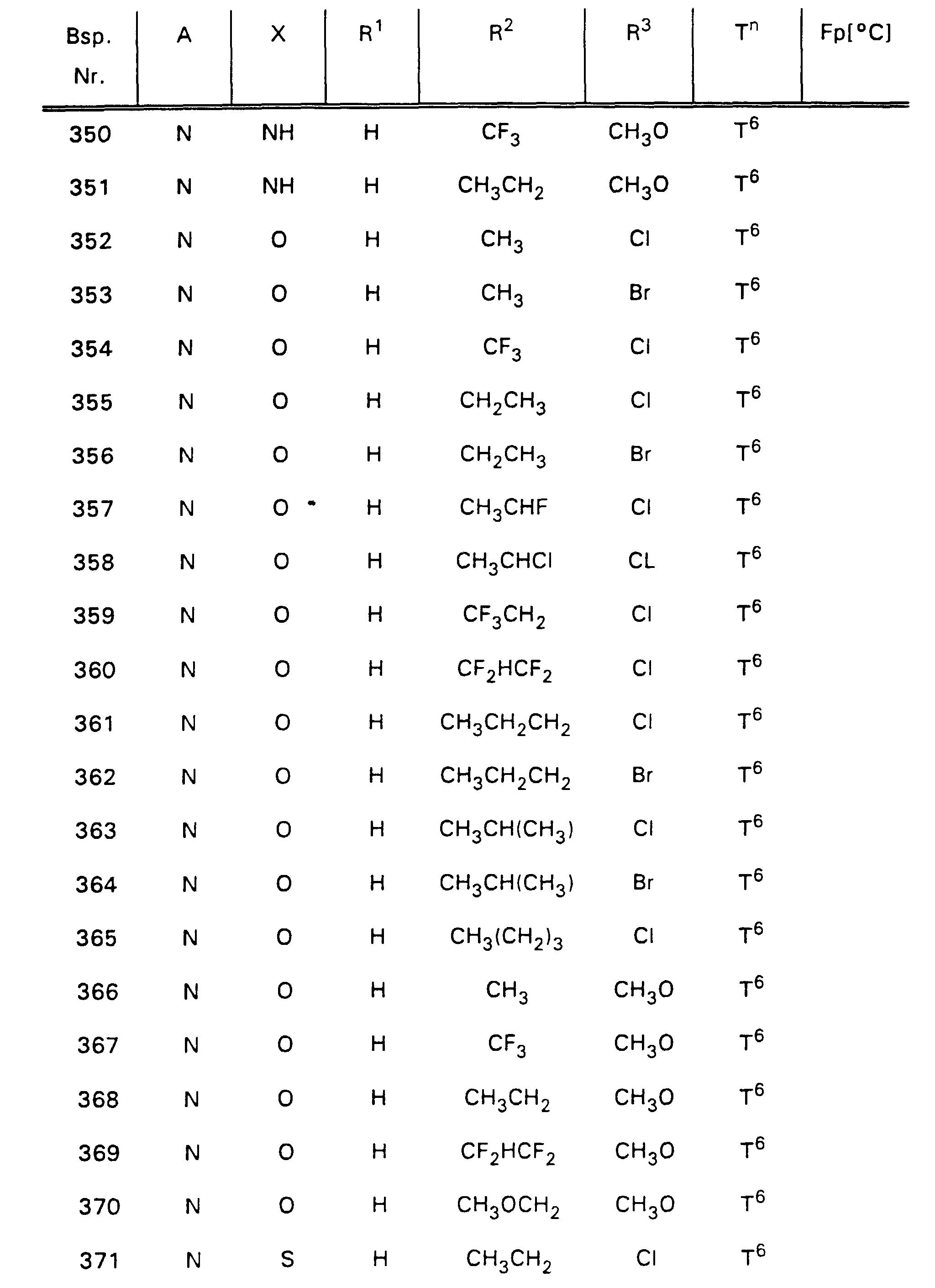

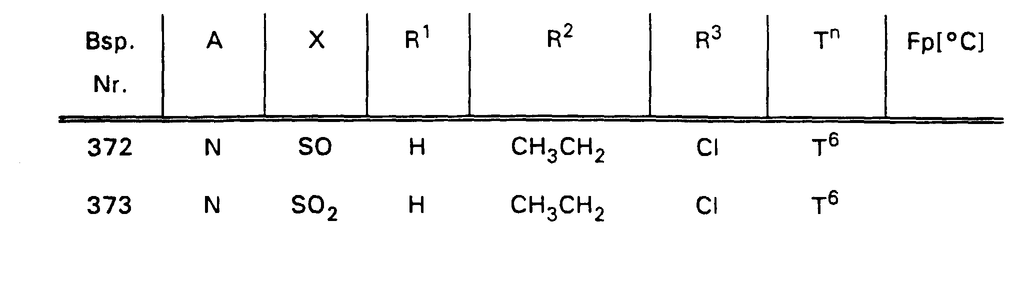

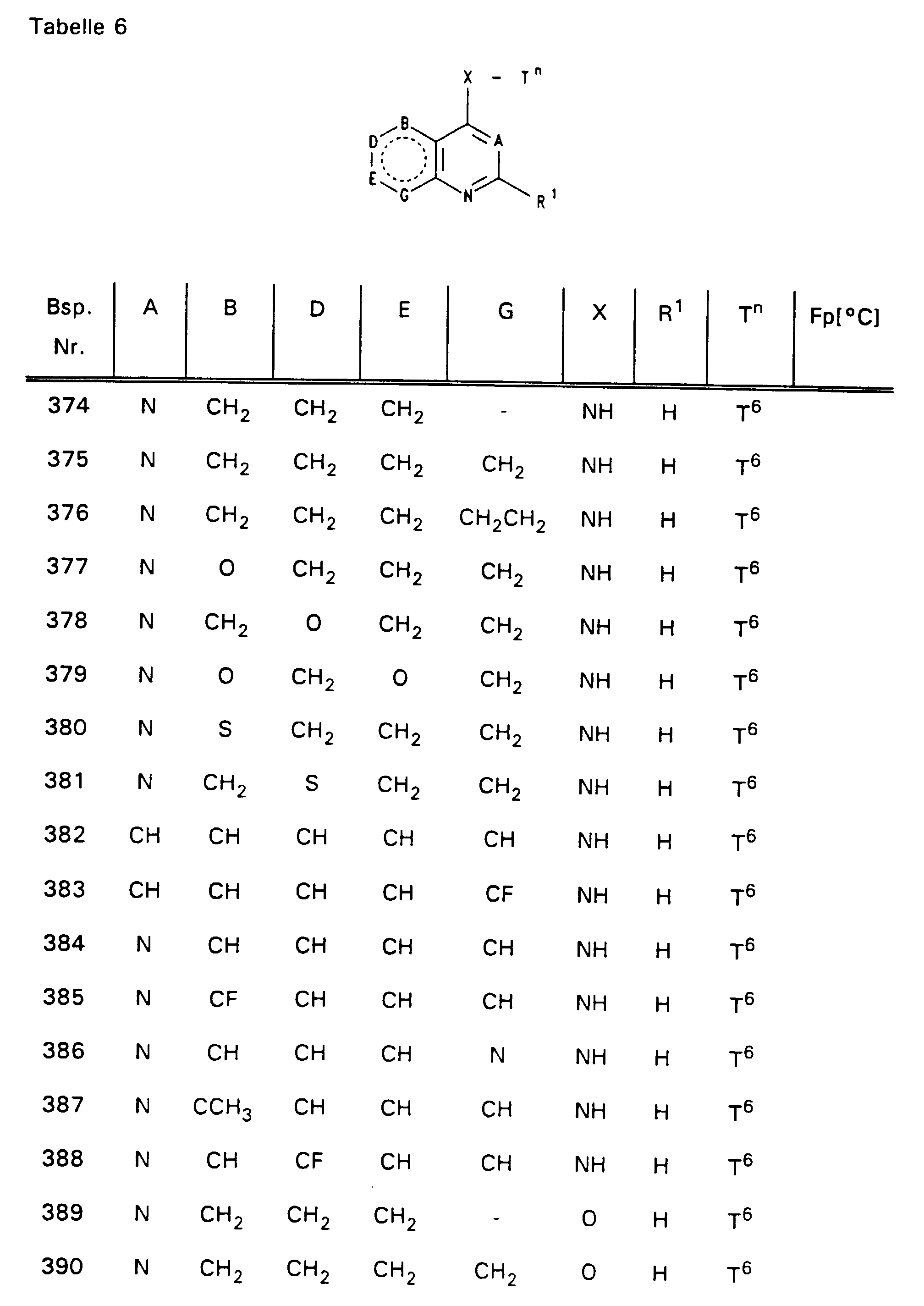

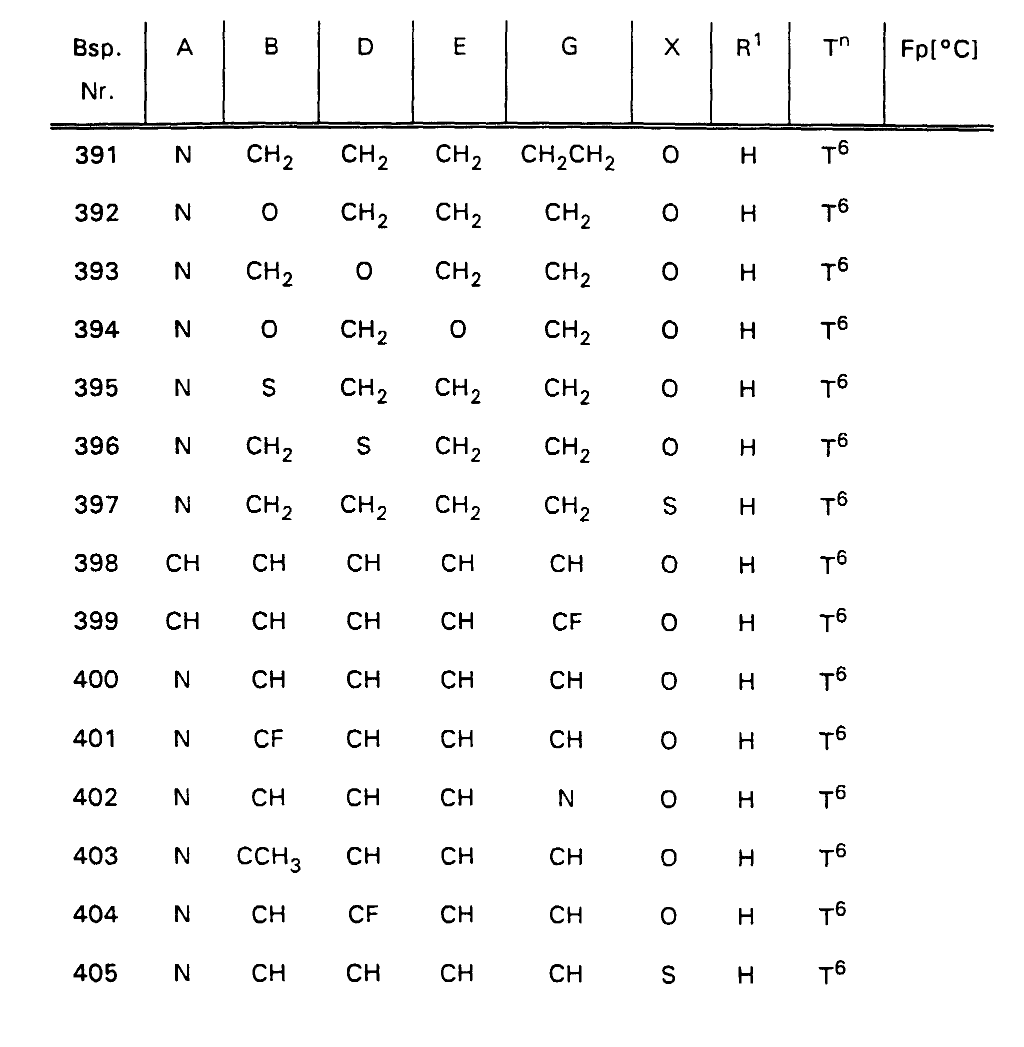

### B Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.
b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleylmethyltaurinsaures Natrium als Netz- und Dispergiermittel mischt und in einer Stiftmühle mahlt.
c) Ein in Wasser leicht dispergierbares Dispersionskonzentrat stellt man her, indem man 40 Gew.-Teile Wirkstoff mit 7 Gew.-Teilen eines Sulfobernsteinsäurehalbesters, 2 Gew.-Teilen eines LigninsulfonsäureNatriumsalzes und 51 Gew.-Teilen Wasser mischt und in einer Reibkugelmühle auf eine Feinheit von unter 5 Mikron vermahlt.
d) Ein emulgierbares Konzentrat läßt sich herstellen aus 15 Gew.-Teilen Wirkstoff, 75 Gew.-Teilen Cyclohexan als Lösungsmittel und 10 Gew.-Teilen oxethyliertem Nonylphenol (10 AeO) als Emulgator.
e) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel b) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten Trägermaterials ca. 95 % des fertigen Granulats.

### C. Biologische Beispiele

### Beispiel 1: Erysiphe graminis

Gerstenpflanzen wurden im 3-Blattstadium mit Konidien des Gerstenmehltaus (Erysiphye graminis f. sp. hordei) stark inokuliert und in einem Gewächshaus bei 20°C und einer relativen Luftfeuchte von 90 - 95 % aufgestellt. 24 Stunden nach Inokulation wurden die Pflanzen mit den nachfolgend aufgeführten Verbindungen in den angegebenen Wirkstoffkonzentrationen gleichmäßig benetzt. Nach einer Inkubationszeit von 10 Tagen wurden die Pflanzen auf Befall mit Gerstenmehltau untersucht. Der Befallsgrad wurde in % befallener Blattfläche im Vergleich zu unbehandelten, zu 100 % infizierten Kontrollpflanzen ausgedrückt.

Bei 250 mg Wirkstoff/l Spritzbrühe zeichen die Substanzen der folgenden Tabellenbeispiele eine vollständige Befallsunterdrückung:
197, 193, 205, 225, 201, 99 und 103.

### Beispiel 2: Leptosphaeria nodorum

Weizenpflanzen der Sorte "Jubilar" wurden im 2-Blatt-Stadium mit wäßrigen Suspensionen der beanspruchten Verbindungen tropfnaß behandelt. Nach dem Antrocknen des Spritzbelages wurden die Pflanzen mit einer wäßrigen Pyknosporen-Suspension von Leptosphaeria nodorum inokuliert und mehrere Stunden bei 100 % re. Luftfeuchte in einer Klimakammer inkubiert. Bis zur Symptomausprägung wurden die Pflanzen im Gewächshaus bei ca. 90 % rel. Luftfeuchte weiterkultiviert.

Ca. 1 Woche nach Inokulation wurde der Befallsgrad in % befallener Blattfläche im Vergleich zu unbehandelten, zu 100 % infizierten Kontrollpflanzen bonitiert.

Bei 250 mg Wirkstoff/l Spritzbrühe zeigen die Substanzen der folgenden Tabellenbeispiele eine vollständige Befallsunterdrückung:
193, 201.

### Beispiel 3:

Mit Bohnenspinnmilben (Tetranychus urticae, Vollpopulation) stark befallene Bohnenpflanzen (Phaseolus v.) wurden mit der wäßrigen Verdünnung eines Spritzpulverkonzentrates, das 250 ppm des jeweiligen Wirkstoffes enthielt, gespritzt.

Die Mortalität der Milben wurde nach 7 Tagen kontrolliert. 100 % Abtötung wurde mit den Verbindungen der Tabellenbeispiele 193, 197, 201, 205, 225, 217, 221, 103, 99 erreicht.

### Beispiel 4:

Mit Schwarzer Bohnenblattlaus (Aphis fabae) stark besetzte Ackerbohne (Vicia faba) werden mit wäßrigen Verdünnungen von Spritzpulverkonzentraten mit 250 ppm Wirkstoffgehalt bis zum Stadium des beginnenden Abtropfens besprüht. Die Mortalität der Blattläuse wird nach 3 Tagen bestimmt. Eine 100 %ige Abtötung kann mit den Verbindungen der Tabellenbeispiele 193, 197, 103, 99, 71, 35 erreicht werden.

### Beispiel 5

Mit Weißer Fliege (Trialeurodes vaporariorum) stark besetzte Bohnenpflanzen wurden mit wäßrigen Suspensionen von Spritzpulverkonzentraten (250 ppm Wirkstoffgehalt) bis zum beginnenden Abtropfen gespritzt. Nach Aufstellung der Pflanzen im Gewächshaus erfolgte nach 14 Tagen die mikroskopische Kontrolle mit dem Ergebnis jeweils 100 %ige Mortalität bei den Präparaten mit den Wirkstoffen der Tabellenbeispiele 193, 197, 201, 225, 217, 221.

### Beispiel 6

Der Boden und der Deckel einer Petrischale werden auf der Innenseite mit je 3 ml und einer wäßrigen Verdünnung eines Spritzpulverkonzentrates, das 250 ppm des jeweiligen Wirkstoffes enthielt, beschichtet. Nach Antrocknen des Belages wurden 24 h alte Stubenfliegen (Musca domestica) in die Petrischalen gesetzt und diese mit den behandelten Deckeln verschlossen. Nach 3 Stunden bei Raumtemperatur (20°C) wurde die Mortalität der Fliegen überprüft. 100 % Abtötung wurde mit den Verbindungen der Tabellenbeispiele 193, 197, 221 erreicht.

### Beispiel 7

Rundes Filterpapier wurde mit je 1 ml der wäßrigen Verdünnung eines Spritzpulverkonzentrates, das 250 ppm des jeweiligen Wirkstoffes enthielt behandelt und bis zum Abtrocknen offen gelagert. Danach wurde das Filterpapier in den Boden einer Petrischale gelegt und mit je 1 ml Wasser (dest.) beträufelt. Anschließend wurden 10 Larven (L3) von Diabrotica undecimpunctata auf das Filterpapier gesetzt, die Petrischale verschlossen und bei 28°C im Dunkeln 48 h aufbewahrt. Danach wurde die Mortalität der Larven bestimmt. 100 % Abtötung wurde mit der Verbindung des Tabellenbeispiels 193 erzielt.

### Beispiel 8

Reissaatgut wurde unter Feuchtbedingungen angekeimt und in Schalen ca. 10 cm hoch angetrieben. Je 3 Reispflanzen wurden in Glasröhrchen, die mit nasser Watte gefüllt waren, gepflanzt und die Blätter der Reispflanzen in eine wäßrige Verdünnung eines Spritzpulverkonzentrates, das 250 ppm des jeweiligen Wirkstoffes enthielt, getaucht. Nach Antrocknen des Belages wurden die Pflanzen mit dem Röhrchen auf den Boden einer Schale gelegt, in die Schale je 10 Tiere der braunrückigen Reiszikade (Nilaparvata lugens, L3) gesetzt, die

Schale verschlossen und bei 25°C aufbewahrt. Die Mortalität der Zikaden wurde nach 3 Tagen kontrolliert. 100 % Abtötung wurde mit der Verbindung des Tabellenbeispiels 197 erzielt.

### Beispiel 9

Larven (L4) der Schabe, Blaberus craniifer wurden in Methanol gelöste Wirkstoffe injiziert.

Nach Applikation der Verbindungen der Tabellenbeispiele 17, 19, 217 (2 x 10⁻⁴ g a.i./Tier) konnte nach 48 Stunden eine 100 %ige Mortalität festgestellt werden.

## Patentansprüche

1. Verbindungen der Formel I 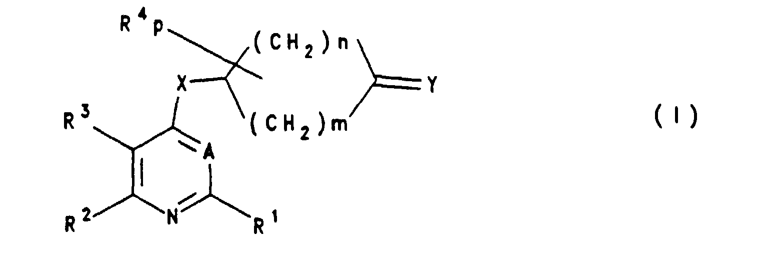 und deren Säureadditionssalze, worin
A die Bedeutung N oder CH hat,
R¹ Wasserstoff, Halogen, (C₁-C₄)-Alkyl oder (C₃-C₆)-Cycloalkyl bedeutet,
R² Wasserstoff, Halogen, (C₁-C₄)-Alkyl, (C₁-C₄)-Halogenalkyl, (C₁-C₄)-Alkoxy, (C₁-C₄)-Halogenalkoxy, (C₁-C₄)-Alkoxy-(C₁-C₄)-alkyl, (C₁-C₄)-Halogenalkoxy-(C₁-C₄)-halogenalkyl, (C₁-C₄)-Alkylthio, (C₁-C₄)-Alkylthio-(C₁-C₄)-alkyl, (C₁-C₄)-Alkylamino oder Di-(C₁-C₄)-alkylamino bedeutet,
R³ Wasserstoff, Halogen, (C₁-C₄)-Alkyl, (C₁-C₄)-Halogenalkyl, (C₁-C₄)-Alkoxy, (C₁-C₄)-Halogenalkoxy, (C₁-C₄)-Alkylthio, Amino, (C₁-C₄)-Alkylamino oder Di-(C₁-C₄)-alkylamino bedeutet oder
R² und R³ zusammen mit den Kohlenstoffatomen, an die sie gebunden sind, einen ungesättigten 5- oder 6-gliedrigen isocyclischen Ring bilden, der auch ein oder mehrere, vorzugsweise ein oder zwei Stickstoffatome enthalten kann und der, falls es sich um einen 5-Ring handelt, an Stelle von CH₂ ein Sauerstoff- oder Schwefelatom enthalten kann und der gegebenenfalls durch 1, 2 oder 3 gleiche oder verschiedene Reste substituiert ist und diese Reste (C₁-C₄)-Alkyl, (C₁-C₄)-Alkoxy, (C₁-C₄)-Halogenalkyl, (C₁-C₄)-Halogenalkoxy und/oder Halogen bedeuten,
oder
R² und R³ zusammen mit den Kohlenstoffatomen, an die sie gebunden sind, einen gesättigten 5-, 6- oder 7-gliedrigen isocyclischen Ring bilden, der an Stelle von CH₂ ein oder mehrere Sauerstoff- oder Schwefelatome enthalten kann und der gegebenenfalls durch ein bis vier gleiche oder verschiedene Reste aus der Reihe (C₁-C₄)-Alkyl und Halogen substituiert ist,
X Sauerstoff, NH und S(O)_{q} bedeutet, wobei q 0, 1 oder 2 sein kann,
R⁴ Halogen, (C₁-C₄)-Alkyl, (C₃-C₇)-Cycloalkyl, (C₁-C₄)-Halogenalkyl, (C₁-C₄)-Alkoxy, (C₁-C₄)-Halogenalkoxy oder gegebenenfalls mit einem, zwei oder drei gleichen oder verschiedenen Substituenten aus der Reihe Halogen, (C₁-C₄)-Alkyl, (C₁-C₄)-Alkoxy, (C₁-C₄)-Halogenalkyl und (C₁-C₄)-Halogenalkoxy substituiertes phenyl bedeutet;
p eine ganze Zahl von 0 bis 4,
n eine ganze Zahl von 0 bis 2,
m eine ganze Zahl von 1 bis 3 sein kann,
Y die Bedeutungen 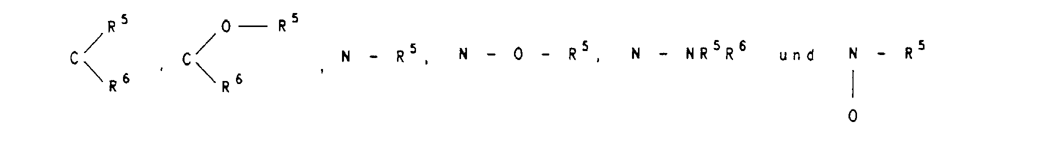 haben kann,
R⁵ Wasserstoff, Halogen, (C₁-C₁₂)-Alkyl, (C₃-C₈)-Cycloalkyl, (C₃-C₈)-Cycloalkyl-(C₁-C₄)-alkyl, [(C₁-C₄)-Alkoxy]ₜ-(C₁-C₄)-alkyl, wobei t eine ganze Zahl von 1 bis 3 sein kann, (C₁-C₁₂)-Halogenalkyl, 2-(Tetrahydro-2H-pyran-2-yloxy)-(C₁-C₄)-alkyl, [(C₁-C₄)Halogenalkoxy]ₜ-(C₁-C₄)-halogenalkyl, [(C₁-C₄)Halogenalkoxy]ₜ-(C₁-C₄)-alkenyl, (C₁-C₈)-Alkylcarbonyl, (C₁-C₈)-Alkoxycarbonyl, (C₁-C₈)-Alkylcarbonyl-(C₁-C₄)-alkyl, (C₁-C₈)-Halogenalkylcarbonyl, (C₁-C₈)-Halogenalkoxycarbonyl, (C₃-C₈)-Cycloalkylcarbonyl, (C₃-C₈)-Cycloalkoxycarbonyl, gegebenenfalls mit einem, zwei oder drei gleichen oder verschiedenen Substituenten aus der Reihe Halogen, (C₁-C₄)-Alkyl, (C₁-C₄)-Alkoxy, (C₁-C₄)-Halogenalkyl und (C₁-C₄)-Halogenalkoxy substituiertes Benzoyl, gegebenenfalls mit einem, zwei oder drei gleichen oder verschiedenen Substituenten aus der Reihe Halogen, (C₁-C₄)-Alkyl, (C₁-C₄)-Alkoxy, (C₁-C₄)-Halogenalkyl und (C₁-C₄)-Halogenalkoxy substituiertes Benzyloxycarbonyl, (C₂-C₈)-Alkenyl, (C₂-C₈)-Alkinyl, Tri-(C₁-C₈)-alkylsilyl, vorzugsweise Dimethyl-(C₁-C₈)alkylsilyl oder Triethylsilyl, Di-(C₁-C₈)-alkyl-(C₃-C₈)-cycloalkylsilyl, vorzugsweise Dimethylcyclohexylsilyl, Di-(C₁-C₈)-alkyl-(phenyl-(C₁-C₄₎-alkyl)-silyl, vorzugsweise Dimethyl-(phenyl-(C₁-C₄)-alkyl)-silyl, Di-(C₁-C₈)-alkyl-(C₁-C₄)-halogenalkylsilyl, Dimethylphenylsilyl, Heteroaryl, Phenyl, Phenyl-(C₂-C₄)-alkyl, Benzyl, Benzyloxy-(C₁-C₄)-alkyl bedeutet, wobei Phenyl oder Heteroaryl in den sechs letztgenannten Resten unsubstituiert oder ein oder mehrfach, vorzugsweise bis zu dreifach, substituiert sein können und diese Substituenten gleich oder verschieden sind und jeweils Halogen,(C₁-C₈)-Alkyl, (C₃-C₈)-Cycloalkyl, (C₁-C₄)-Halogenalkyl, (C₁-C₄)-Dialkylamino, (C₁-C₄)-Alkylthio, (C₁-C₈)Alkoxy, (C₁-C₈)-Alkoxycarbonyl, (C₁-C₈)-Alkanoyloxy,(C₁-C₄)-Halogenalkoxy, (C₁-C₄)-Alkoxy-(C₁-C₄)-alkoxy, [(C₁-C₄)-Alkyl-O]ₜ-(C₂-C₄)-alkyl, 2-(Tetrahydro-2H-pyran-2-yloxy)-ethoxy, (C₂-C₈)-Alkenyl, (C₂-C₈)-Alkinyl, Phenoxy, Benzyloxy, welches im Phenylrest gegebenenfalls einen oder mehrere, vorzugsweise bis zu drei, gleiche oder verschiedene Substituenten aus der Reihe (C₁-C₄)-Alkyl, (C₁-C₄)-Halogenalkyl, (C₁-C₄)-Alkoxy, (C₁-C₄)-Halogenalkoxy und Halogen trägt,
R⁶ Wasserstoff, Halogen, (C₁-C₈)-Alkyl, (C₃-C₈)-Cycloalkyl, (C₁-C₈)-Halogenalkyl, Phenyl und Benzyl bedeutet, wobei die Phenylringe wie oben bei R⁵ beschrieben substituiert sein können, oder
R⁵ und R⁶ zusammen einen 3 - 7-gliedrigen Ring bilden können, der gegebenenfalls mit einem, zwei oder drei gleichen oder verschiedenen Resten aus der Reihe Halogen, (C₁-C₄).Alkyl, (C₁-C₄)-Alkoxy, (C₁-C₄)-Halogenalkyl und (C₁-C₄)-Halogenalkoxy substituiert sein kann und in dem ein Kohlenstoffatom durch O, S, NR⁷ ersetzt sein kann, wobei R⁷ die gleiche Bedeutung wie R⁴ hat, aber nicht Halogen sein darf.

2. Verbindungen der Formel I gemäß Anspruch 1 oder deren Salze, worin
A die in Anspruch 1 angegebene Bedeutung hat,
R¹ Wasserstoff oder Methyl ist,
R² Halogen, (C₁-C₄)-Alkyl, (C₁-C₄)-Halogenalkyl, (C₁-C₄)-Alkoxy oder (C₁-C₄)-Alkoxy-(C₁-C₄)-alkyl bedeutet,
R³ Wasserstoff, Halogen, (C₁-C₃)-Alkyl, Methoxy oder Ethoxy bedeutet
oder
R² und R³ zusammen mit den Kohlenstoffatomen, an die sie gebunden sind, einen ungesättigten 5- oder 6-gliedrigen Ring bilden, der ein bis zwei Stickstoffatome, ein Sauerstoff- oder Schwefelatom enthalten kann, oder
R² und R³ zusammen mit den Kohlenstoffatomen, an die sie gebunden sind, einen gesättigten, gegebenenfalls substituierten, 5- oder 6-gliedrigen Ring bilden, der ein Schwefelatom oder 1 bis 2 Sauerstoffatome enthalten kann, und
R⁴, R⁵, R⁶ und
R⁷ sowie
X, Y, m, n, p die in Anspruch 1 angegebenen Bedeutungen haben.

3. Verbindungen der Formel I gemäß Anspruch 1 oder 2 oder deren Salze, worin
A die im Anspruch 1 angegebene Bedeutung hat,
R¹ Wasserstoff bedeutet,
R² (C₁-C₄)-Alkyl oder Methoxymethyl bedeutet,
R³ Methyl, Ethyl, Methoxy, Chlor oder Brom bedeutet, oder
R² und R³ zusammen mit den Kohlenstoffatomen, an die sie gebunden sind, das Chinolin- oder Chinazolin-System bilden, das mit Fluor, Chlor, Brom oder Methyl substituiert sein kann oder
R² und R³ zusammen mit den Kohlestoffatomen, an die sie gebunden sind, ein ungesättigten 6-Ring bilden, der ein Stickstoffatom enthält, oder
R² und R³ zusammen mit den Kohlenstoffatomen, an die sie gebunden sind, einen gesättigten 6-gliedrigen Ring bilden, der ein oder zwei Sauerstoffatome enthalten kann,
m, n, p und X die in Anspruch 1 angegebenen Bedeutungen haben,
Y 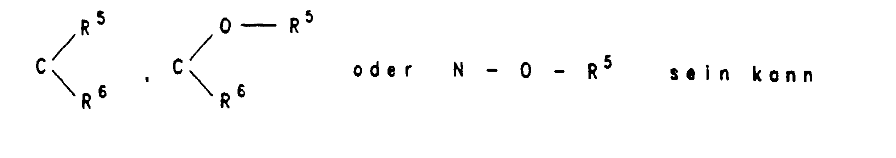 und
R⁵, R⁶ und R⁷ ebenfalls die in Anspruch 1 definierten Bedeutungen haben.

4. Verbindungen der Formel I gemäß einem der Ansprüche 1 bis 3 oder deren Salze, worin
A und R¹ bis R⁷ die in den vorangehenden Ansprüchen angegebenen Bedeutungen haben, und
m und n 2 sind,
p 0 ist, und
X die Bedeutung NH oder Sauerstoffatom hat.

5. Verbindungen der Formel I gemäß einem der Ansprüche 1 bis 4 oder deren Salze, worin
A die in Anspruch 1 angegebene Bedeutung hat,
R¹ Wasserstoff bedeutet,
R² (C₁-C₄)-Alkyl oder Methoxymethyl bedeutet,
R³ Chlor, Brom oder Methoxy bedeutet oder
R² und R³ zusammen mit den Kohlenstoffatomen, an die sie gebunden sind, das Chinolin oder Chinazolin-System bilden, das mit Fluor, Chlor, Brom oder Methyl substituiert sein kann oder
R² und R³ zusammen mit dem Pyrimidin-Ring das 5,6,7,8-Tetrahydrochinazolin-System oder das 5,6-Dihydro-7H-pyrano [2,3-d]pyrimidin-System bilden,
m und n die Zahl zwei bedeuten,
p gleich null ist,
X die Bedeutung NH oder Sauerstoffatom hat,
Y 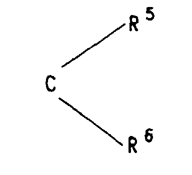 oder N - O - R⁵ bedeutet, und
R⁵, R⁶ und R⁷ die in den vorangehenden Ansprüchen definierten Bedeutungen haben.

6. Verbindungen der Formel I gemäß einem der Ansprüche 1 bis 5 oder deren Salze, worin
A die in Anspruch 1 angegebene Bedeutung hat,
R¹ Wasserstoff bedeutet,
R² Methoxymethyl und R³ Methoxy oder
R² (C₁-C₄)-Alkyl und R³ Chlor oder Brom bedeuten oder
R² und R³ zusammen mit dem Kohlenstoffatom, an das sie gebunden sind, ein Chinolin- oder Chinazolin-System bilden, das mit Fluor, Chlor oder Methyl substituiert ist oder ein 5,6,7,8-Tetrahydrochinazolin-, oder das 5,6-Dihydro-7H-pyrano[2,3-d]pyrimidin-System bilden,
m, n, p, X sowie Y die in Anspruch 1 genannten Bedeutungen haben,
R⁵ (C₂-C₈)-Alkyl, Cyclopentyl, Cyclohexyl, (C₁-C₈)-Alkylcarbonyl, (C₁-C₈)-Alkoxycarbonyl, Phenyl, Benzyl, Benzoyl bedeutet, wobei die letztgenannten drei Reste unsubstituiert oder mit einem oder zwei Substituierten versehen sein können die gleich oder verschieden sein können und diese Fluor, Chlor, Brom, (C₁-C₄)-Alkyl, (C₁-C₄)-Alkoxy, Triflourmethyl, (C₁-C₂)-Halogenalkoxy, Cyclohexyl, 2-Ethoxyethoxy, Methylthio oder Dimethylamino bedeuten und
R⁶ H, Halogen oder (C₁-C₃)-Alkyl sein kann.

7. Verfahren zur Herstellung von Verbindungen der Formel I gemäß einem der Ansprüche 1 bis 6, dadurch gekennzeichnet, daß man
a) eine Verbindung der Formel II 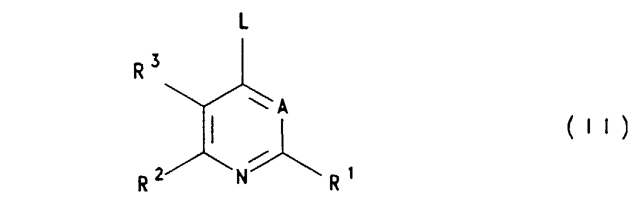 worin A, R¹, R² und R³ die unter Formel I angegebenen Bedeutungen haben, umsetzt mit einer Verbindung der Formel III 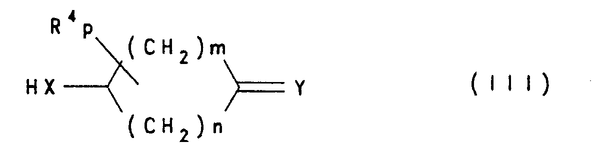 worin X, R⁴, n, m und Y die unter Formel I angegebenen Bedeutungen haben, oder
b) zur Herstellung einer Verbindung der Formel I, worin Y für 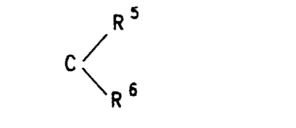 steht, eine Verbindung der Formel V 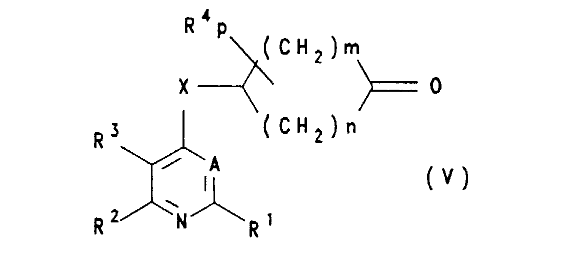 worin R¹, R², R³, R⁴, A, X, m, n und p die unter Formel 1 angegebenen Bedeutungen haben, in Gegenwart einer Base umsetzt mit einer Verbindung der Formel P⁺Ph₃CHR⁵R⁶Z⁻, worin Ph einen aromatischen Rest und Z⁻ ein Anion bedeutet und R⁵ und R⁶ wie unter Formel I definiert sind, oder
c) zur Herstellung einer Verbindung, worin Y nicht für 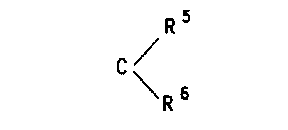 steht, eine Verbindung der unter b) definierten Formel V umsetzt mit H₂NOH oder einer Verbindung der Formel H-Q, worin Q für HNR⁵, HN-OR⁵, HN-NR⁵R⁶ oder N(OH)R⁵ steht und R⁵, R⁶ wie unter Formel I definiert sind,
nach a) oder c) erhaltene Verbindungen der Formel I, worin Y für N-OH steht, gegebenenfalls in Gegenwart einer Base umsetzt, mit einer Verbindung der Formel L-R⁵, worin L eine Abgangsgruppe bedeutet und R⁵ wie unter Formel I definiert ist, aber nicht Wasserstoff bedeutet,
und erhaltene Verbindungen gegebenenfalls in ihr Salz überführt.

8. Mittel, enthaltend mindestens eine Verbindung gemäß einem der Ansprüche 1 bis 6 und mindestens ein Formulierungshilfsmittel.

9. Fungizides Mittel gemäß Anspruch 8, enthaltend eine fungizid wirksame Menge mindestens einer Verbindung gemäß einem der Ansprüche 1 bis 6 zusammen mit den für diese Anwendung üblichen Zusatz- oder Hilfsstoffen.

10. Insektizides, akarizides oder nematizides Mittel gemäß Anspruch 8, enthaltend eine wirksame Menge mindestens einer Verbindung gemäß einem der Ansprüche 1 bis 6 zusammen mit den für diese Anwendungen üblichen Zusatz- oder Hilfststoffen.

11. Pflanzenschutzmittel, enthaltend eine fungizid, insektizid, akarizid oder nematizid wirksame Menge mindestens einer Verbindung gemäß einem der Ansprüche 1 bis 6 und mindestens einem weiteren Wirkstoff, vorzugsweise aus der Reihe der Fungizide, Insektizide, Lockstoffe, Sterilantien, Akarizide, Nematizide und Herbizide zusammen mit den für diese Anwendung üblichen Hilfs- und Zusatzstoffen.

12. Mittel zur Anwendung im Holzschutz oder als Konservierungsmittel in Dichtmassen, in Anstrichfarben, in Kühlschmiermitteln für die Metallbearbeitung oder in Bohr- und Schneidölen, enthaltend eine wirksame Menge mindestens einer Verbindung gemäß einem der Ansprüche 1 bis 6 zusammen mit den für diese Anwendungen üblichen Hilfs- und Zusatzstoffen.

13. Verbindung gemäß einem der Ansprüche 1 bis 6 oder Mittel gemäß Anspruch 8, zur Anwendung als Tierarzneimittel, vorzugsweise bei der Bekämpfung von Endo- oder Ektoparasiten.

14. Verfahren zur Herstellung eines Mittels gemäß einem der Ansprüchje 8 bis 13, dadurch gekennzeichnet, daß man den Wirkstoff und die weiteren Zusätze zusammen gibt und in eine geeignete Anwendungsform bringt.

15. Verwendung einer Verbindung gemäß einem der Ansprüche 1 bis 6 oder eines Mittels gemäß einem der Ansprüche 8, 9, 11 und 12 als Fungizid.

16. Verwendung einer Verbindung der Formel I gemäß einem der Ansprüche 1 bis 6 oder eines Mittels gemäß einem der Ansprüche 8, 9 und 12 als Holzschutzmittel oder als Konservierungsmittel in Dichtmassen, in Anstrichfarben, in Kühlschmiermitteln für die Metallbearbeitung oder in Bohrund Schneidölen.

17. Verfahren zur Bekämpfung von phytopathogenen Pilzen, dadurch gekennzeichnet, daß man auf diese oder die von ihnen befallenen Pflanzen, Flächen oder Substrate oder auf Saatgut eine fungizid wirksame Menge einer Verbindung gemäß einem der Ansprüche 1 bis 6 oder eines Mittels gemäß einem der Ansprüche 8, 9, 11 und 12 appliziert.

18. Verfahren zur Bekämpfung von Schadinsekten, Acarina und Nematoden, bei welchem man auf diese oder die von ihnen befallenen Pflanzen, Flächen oder Substrate eine wirksame Menge einer Verbindung gemäß einem der Ansprüche 1 bis 6 oder eines Mittels gemäß einem der Ansprüche 8, 10 und 11 appliziert.

19. Verwendung von Verbindungen gemäß einem der Ansprüche 1 bis 6 oder eines Mittels gemäß einem der Ansprüche 8, 10 und 11 zur Bekämpfung von Schadinsekten, Acarina und Nematoden.

20. Saatgut, gebeizt oder beschichtet mit einer wirksamen Menge einer Verbindung der Formel I gemäß einem der Ansprüche 1 bis 6 oder eines Mittels gemäß einem der Ansprüche 8 bis 11.

21. Verbindungen der Formel V, 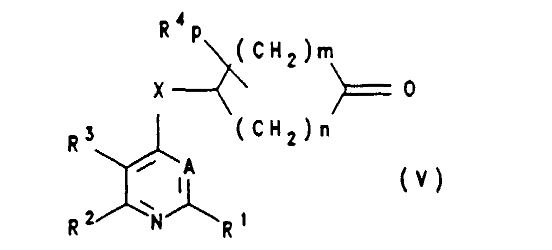 in welcher R¹, R², R³, R⁴, A, X, m, n und p wie in Anspruch 1 definiert sind.

22. Verbindungen der Formel II, 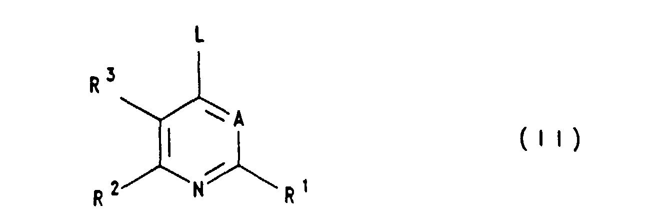 in welcher
R² und R³ zusammen mit den Kohlenstoffatomen, an die sie gebunden sind, einen gesättigten 5-, 6- oder 7-gliedrigen isocyclischen Ring bilden, der an Stelle von CH₂ ein oder mehrere Sauerstoff- oder Schwefelatome enthalten kann und der gegebenenfalls mit einem bis vier gleichen oder verschiedenen Resten aus der Reihe (C₁-C₄)-Alkyl und Halogen substituiert ist und
L eine Abgangsgruppe ist, und R¹ und A wie in Anspruch 1 definiert sind.

## Claims

1. A compound of the formula I 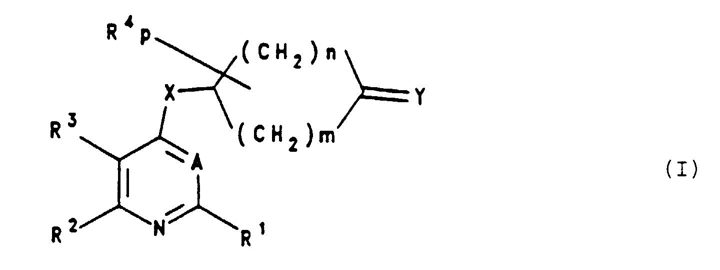 or an acid addition salt thereof, in which
A is N or CH,
R¹ is hydrogen, halogen, (C₁-C₄)-alkyl or (C₃-C₆)-cycloalkyl,
R² is hydrogen, halogen, (C₁-C₄) -alkyl, (C₁-C₄)-haloalkyl, (C₁-C₄)-alkoxy, (C₁-C₄)-haloalkoxy, (C₁-C₄) -alkoxy- (C₁-C₄) -alkyl, (C₁-C₄) -haloalkoxy- (C₁-C₄)-haloalkyl, (C₁-C₄) -alkylthio, (C₁-C₄)-alkylthio- (C₁-C₄)-alkyl, (C₁-C₄) -alkylamino or di-(C₁-C₄)-alkylamino,
R³ is hydrogen, halogen, (C₁-C₄) -alkyl, (C₁-C₄)-haloalkyl, (C₁-C₄) -alkoxy, (C₁-C₄) -haloalkyoxy, (C₁-C₄)-alkylthio,amino, (C₁-C₄)-alkylamino or di- (C₁-C₄)-alkylamino, or
R² and R³ together with the carbon atoms to which they are bonded form an unsaturated 5- or 6-membered isocyclic ring which can also contain one or more, preferably one or two, nitrogen atoms and which, in the event that it is a 5-membered ring, can contain an oxygen or sulfur atom instead of CH₂ and which is optionally substituted by 1, 2 or 3 identical or different radicals, these radicals being (C₁-C₄) -alkyl, (C₁-C₄) -alkoxy, (C₁-C₄) -haloalkyl, (C₁-C₄) -haloalkoxy and/or halogen,
or
R² and R³ together with the carbon atoms to which they are bonded form a saturated 5-, 6- or 7-membered isocyclic ring which can contain one or more oxygen or sulfur atoms instead of CH₂ and which is optionally substituted by one to four identical or different radicals selected from the series consisting of (C₁-C₄)-alkyl and halogen,
X is oxygen, NH and S(O)_{q}, it being possible for q to be 0, 1 or 2,
R⁴ is halogen, (C₁-C₄)-alkyl, (C₃-C₇)-cycloalkyl, (C₁-C₄)-haloalkyl, (C₁-C₄) -alkoxy, (C₁-C₄) -haloalkoxy or phenyl which is optionally substituted by one, two or three identical or different substituents selected from the series consisting of halogen, (C₁-C₄) -alkyl, (C₁-C₄) -alkoxy, (C₁-C₄) -haloalkyl and (C₁-C₄) -haloalkoxy;
p can be an integer from 0 to 4,
n can be an integer from 0 to 2,
m can be an integer from 1 to 3,
Y can be 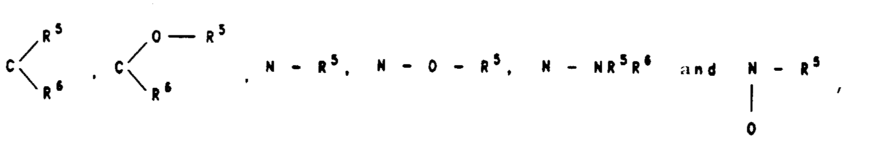
R⁵ is hydrogen, halogen, (C₁-C₁₂)-alkyl, (C₃-C₈)-cycloalkyl, (C₃-C₈)-cycloalkyl-(C₁-C₄)-alkyl,[(C₁-C₄) -alkoxy]ₜ- (C₁-C₄)-alkyl, it being possible for t to be an integer from 1 to 3, (C₁-C₁₂)-haloalkyl, 2-(tetrahydro-2H-pyran-2-yloxy) - (C₁-C₄)-alkyl, [(C₁-C₄) haloalkoxy]ₜ- (C₁-C₄) -haloalkyl, [(C₁-C₄) haloalkoxy]ₜ- (C₁-C₄) -alkenyl, (C₁-C₈)-alkylcarbonyl, (C₁-C₈) -alkoxycarbonyl, (C₁-C₈)-alkylcarbonyl- (C₁-C₄)-alkyl, (C₁-C₈)-haloalkylcarbonyl, (C₁-C₈) -haloalkoxycarbonyl, (C₃-C₈) -cycloalkylcarbonyl, (C₃-C₈)-cycloalkoxycarbonyl, benzoyl which is optionally substituted by one, two or three identical or different substituents selected from the series consisting of halogen, (C₁-C₄)alkyl, (C₁-C₄)-alkoxy, (C₁-C₄)-haloalkyl and (C₁-C₄) - haloalkoxy, benzyloxycarbonyl which is optionally substituted by one, two or three identical or different substituents selected from the series consisting of halogen, (C₁-C₄)-alkyl, (C₁-C₄)-alkoxy, (C₁-C₄)-haloalkyl and (C₁-C₄) haloalkoxy, (C₂-C₈)-alkenyl, (C₂-C₈) -alkynyl, tri- (C₁-C₈) -alkylsilyl, preferably dimethyl - (C₁-C₈) alkylsilyl or triethylsilyl, di-(C₁-C₈)-alkyl-(C₃-C₈)-cycloalkylsilyl , preferably dimethylcyclohexylsilyl, di- (C₁-C₈) -alkyl(phenyl-(C₁-C₄)-alkyl)-silyl, preferably dimethyl- (phenyl- (C₁-C₄) -alkyl) -silyl, di- (C₁-C₈)-alkyl-(C₁-C₄)-haloalkylsilyl, dimethylphenylsilyl, heteroaryl, phenyl, phenyl-(C₂-C₄) -alkyl, benzyl, benzyloxy- (C₁-C₄)-alkyl, it being possible for phenyl or heteroaryl in the last-mentioned six radicals to be unsubstituted or mono- or polysubstituted, preferably up to trisubstituted, and these substituents, which are identical or different, are in each case halogen, (C₁-C₈)-alkyl, (C₃-C₈)-cycloalkyl, (C₁-C₄)-haloalkyl, (C₁-C₄) -dialkylamino, (C₁-C₄)-alkylthio, (C₁-C₈)-alkoxy, (C₁-C₈)-alkoxycarbonyl, (C₁-C₈)-alkanoyloxy, (C₁-C₄) -haloalkoxy, (C₁-C₄)-alkoxy-(C₁-C₄)-alkoxy, [(C₁-C₄) -alkyl-O]ₜ- (C₂-C₄)-alkyl, 2-(tetrahydro-2H-pyran-2-yloxy) -ethoxy, (C₂-C₈)-alkenyl, (C₂-C₈)-alkynyl, phenoxy or benzyloxy which has optionally one or more, preferably up to three, identical or different substituents selected from the series consisting of (C₁-C₄)-alkyl, (C₁-C₄)-haloalkyl, (C₁-C₄)-alkoxy, (C₁-C₄)-haloalkoxy and halogen in the phenyl radical,
R⁶ is hydrogen, halogen, (C₁-C₈) -alkyl, (C₃-C₈)-cycloalkyl, (C₁-C₈)-haloalkyl, phenyl and benzyl, it being possible for the phenyl rings to be substituted as described above under R⁵, or
R⁵ and R⁶ together can form a 3- to 7-membered ring which can optionally be substituted by one, two or three identical or different radicals selected from the series consisting of halogen, (C₁-C₄)-alkyl, (C₁-C₄) -alkoxy, (C₁-C₄) -haloalkyl and (C₁-C₄)-haloalkoxy and in which a carbon atom can be replaced by 0, S, NR⁷ where R⁷ is as defined for R⁴ but must not be halogen.

2. A compound of the formula I as claimed in claim 1 or a salt thereof, in which
A is as defined in claim 1,
R¹ is hydrogen or methyl,
R² is halogen, (C₁-C₄)-alkyl, (C₁-C₄)-haloalkyl, (C₁-C₄)-alkoxy or (C₁-C₄) -alkoxy- (C₁-C₄) -alkyl,
R³ is hydrogen, halogen, (C₁-C₃)-alkyl, methoxy or ethoxy,
or
R² and R³ together with the carbon atoms to which they are bonded form an unsaturated 5- or 6-membered ring which can contain one to two nitrogen atoms, an oxygen atom or a sulfur atom, or
R² and R³ together with the carbon atoms to which they are bonded form a saturated, optionally substituted 5- or 6-membered ring which can contain a sulfur atom or 1 to 2 oxygen atoms, and
R⁴, R⁵, R⁶ and
R⁷ and
X, Y, m, n, p are as defined in claim 1.

3. A compound of the formula I as claimed in claim 1 or 2 or a salt thereof, in which
A is as defined in claim 1,
R¹ is hydrogen,
R² is (C₁-C₄)-alkyl or methoxymethyl,
R³ is methyl, ethyl, methoxy, chlorine or bromine, or
R² and R³ together with the carbon atoms to which they are bonded form the quinoline or quinazoline system which can be substituted by fluorine, chlorine, bromine or methyl, or
R² and R³ together with the carbon atoms to which they are bonded form an unsaturated 6-membered ring which contains a nitrogen atom, or
R² and R³ together with the carbon atoms to which they are bonded form a saturated 6-membered ring which can contain one or two oxygen atoms,
m, n, p and X are as defined in claim 1,
Y can be 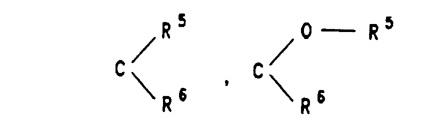 or N- O - R⁵ and
R⁵, R⁶ and R⁷ are also as defined in claim 1.

4. A compound of the formula I as claimed in any of claims 1 to 3 or a salt thereof, in which
A and R¹ to R⁷ are as defined in the preceding claims, and
m and n are 2,
p is 0, and
X is NH or an oxygen atom.

5. A compound of the formula I as claimed in any of claims 1 to 4 or a salt thereof, in which
A is as defined in claim 1,
R¹ is hydrogen,
R² is (C₁-C₄)-alkyl or methoxymethyl,
R³ is chlorine, bromine or methoxy, or
R² and R³ together with the carbon atoms to which they are bonded form the quinoline or quinazoline system which can be substituted by fluorine, chlorine, bromine or methyl, or
R² and R³ together with the pyrimidine ring form the 5,6,7,8-tetrahydroquinazoline system or the 5,6-dihydro-7H-pyrano [2,3-d] pyrimidine system,
m and n are the number two,
p is zero,
X is NH or an oxygen atom,
Y is 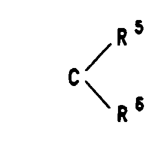 or N - O - R⁵, and
R⁵, R⁶ and R⁷ are as defined in the preceding claims.

6. A compound of the formula I as claimed in any of claims 1 to 5 or a salt thereof, in which
A is as defined in claim 1,
R¹ is hydrogen,
R² is methoxymethyl and R³ is methoxy, or
R² is (C₁-C₄) -alkyl and R³ is chlorine or bromine, or
R² and R³ together with the carbon atom to which they are bonded form a quinoline or quinazoline system which is substituted by fluorine, chlorine or methyl, or a 5,6,7,8-tetrahydroquinazoline system or the 5,6-dihydro-7H-pyrano [2,3-d]pyrimidine system,
m, n, p, X and Y are as defined in claim 1,
R⁵ is (C₂-C₈)-alkyl, cyclopentyl, cyclohexyl, (C₁-C₈) -alkylcarbonyl, (C₁-C₈) -alkoxycarbonyl, phenyl, benzyl or benzoyl, it being possible for the last-mentioned three radicals to be unsubstituted or to be provided with one or two substituents which can be identical or different and are fluorine, chlorine, bromine, (C₁-C₄)-alkyl, (C₁-C₄) -alkoxy, trifluoromethyl, (C₁-C₂)-haloalkoxy, cyclohexyl, 2-ethoxyethoxy, methylthio or dimethylamino, and
R⁶ can be H, halogen or (C₁-C₃)-alkyl.

7. A process for the preparation of compounds of the formula I as claimed in any of claims 1 to 6, which comprises
a) reacting a compound of the formula II 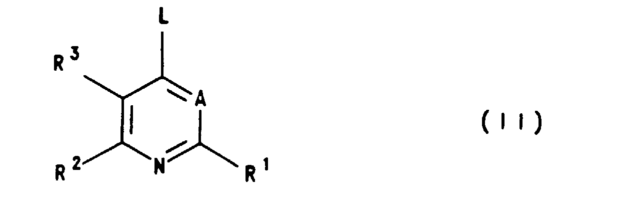 in which A, R¹, R² and R³ are as defined under formula I with a compound of the formula III 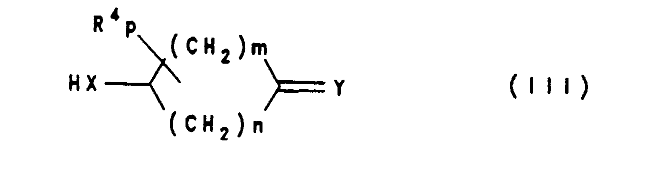 in which X, R⁴, n, m and Y are as defined under formula I, or
b) to prepare a compound of the formula I in which Y is 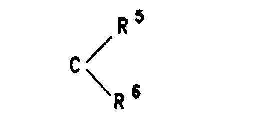 reacting a compound of the formula V 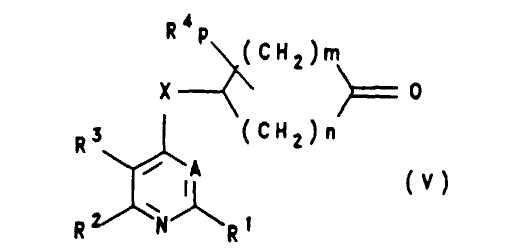 in which R¹, R², R³, R⁴, A, X, m, n and p are as defined under formula I with a compound of the formula P⁺Ph₃CHR⁵R⁶Z⁻ in which Ph is an aromatic radical, Z⁻ is an anion and R⁵ and R⁶ are as defined under formula I, in the presence of a base, or
c) to prepare a compound in which Y is not 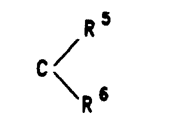 reacting a compound of the formula V defined under b) with H₂NOH or with a compound of the formula H-Q in which Q is HNR⁵, HN-OR⁵, HN-NR⁵R⁶ or N(OH)R⁵ and R⁵ and R⁶ are as defined under formula I,
reacting compounds of the formula I in which Y is N-OH and which have been obtained by a) or c) with a compound of the formula L-R⁵ in which L is a leaving group and R⁵ is as defined under formula I, but is not hydrogen, if appropriate in the presence of a base,
and, if appropriate, converting compounds obtained into a salt thereof.

8. A composition comprising at least one compound as claimed in any of claims 1 to 6 and at least one formulation auxiliary.

9. A fungicidal composition as claimed in claim 8, comprising a fungicidally effective amount of at least one compound as claimed in any of claims 1 to 6 together with the additives or auxiliaries which are customary for this application.

10. An insecticidal, acaricidal or nematicidal composition as claimed in claim 8, comprising an effective amount of at least one compound as claimed in any of claims 1 to 6 together with the additives or auxiliaries which are customary for these applications.

11. A crop protection composition, comprising a fungicidally, insectidically, acaricidally or nematicidally effective amount of at least one compound as claimed in any of claims 1 to 6 and at least one further active ingredient, preferably from the series of the fungicides, insecticides, attractants, sterilants, acaricides, nematicides and herbicides, together with the auxiliaries and additives which are customary for this application.

12. A composition for use in the preservation of wood or as a preservative in sealants, in paints, in cooling lubricants for metalworking or in drilling and cutting oils, comprising an effective amount of at least one compound as claimed in any of claims 1 to 6 together with the auxiliaries and additives which are customary for these applications.

13. A compound as claimed in any of claims 1 to 6 or a composition as claimed in claim 8 for use as a veterinary pharmaceutical, preferably for controlling endoparasites or ectoparasites.

14. A process for the preparation of a composition as claimed in any of claims 8 to 13, which comprises combining the active substance and the other additives and formulating the mixture to give a suitable use form.

15. The use of a compound as claimed in any of claims 1 to 6 or of a composition as claimed in any of claims 8, 9, 11 and 12 as a fungicide.

16. The use of a compound of the formula I as claimed in any of claims 1 to 6 or of a composition as claimed in any of claims 8, 9 and 12 as a wood preservative or as a preservative in sealants, in paints, in cooling lubricants for metalworking or in drilling and cutting oils.

17. A method of controlling phytopathogenic fungi, which comprises applying a fungicidally effective amount of a compound as claimed in any of claims 1 to 6 or of a composition as claimed in any of claims 8, 9, 11 and 12 to these phytopathogenic fungi or to the plants, areas or substrates attacked by them, or to seed.

18. A method of controlling harmful insects, Acarina and nematodes, in which an effective amount of a compound as claimed in any of claims 1 to 6 or of a composition as claimed in any of claims 8, 10 and 11 is applied to these harmful insects, Acarina and nematodes or to the plants, areas or substrates attacked by them.

19. The use of compounds as claimed in any of claims 1 to 6 or of a composition as claimed in any of claims 8, 10 and 11 for controlling harmful insects, Acarina and nematodes.

20. Seed, dressed or coated with an effective amount of a compound of the formula I as claimed in any of claims 1 to 6 or of a composition as claimed in any of claims 8 to 11.

21. A compound of the formula V 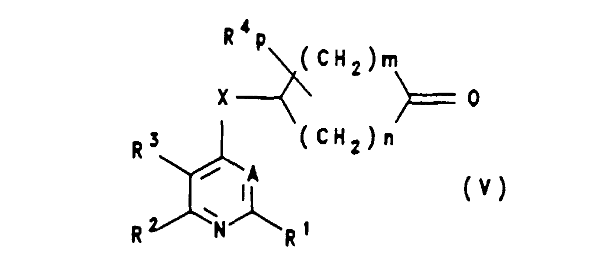 in which R¹, R², R³, R⁴, A, X, m, n and p are as defined in claim 1.

22. A compound of the formula II 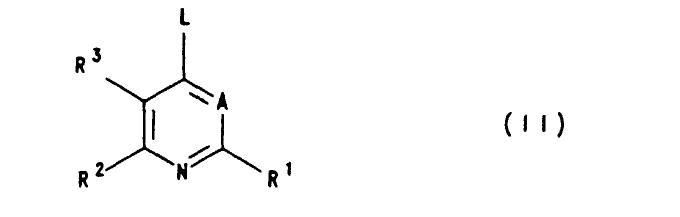 in which
R² and R³ together with the carbon atoms to which they are bonded form a saturated 5-, 6- or 7-membered isocyclic ring which can contain one or more oxygen or sulfur atoms instead of CH₂ and which is optionally substituted by one to four identical or different radicals selected from the series consisting of (C₁-C₄)-alkyl and halogen, and
L is a leaving group, and R¹ and A are as defined in claim 1.

## Revendications

1. Composés de formule I 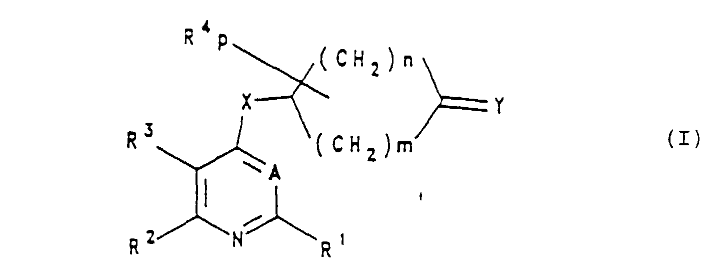 et leurs sels d'addition avec un acide, dans lesquels
A représente un atome d'azote ou un groupe CH,
R¹ représente un atome d'hydrogène, un atome d'halogène, un groupe alkyle en C₁-C₄ ou cycloalkyle en C₃-C₆,
R² représente un atome d'hydrogène, un atome d'halogène, un groupe alkyle en C₁-C₄, halogénoalkyle en C₁-C₄, alkoxy en C₁-C₄, halogénoalkoxy en C₁-C₄, (alkoxy en C₁-C₄)-(alkyle en C₁-C₄), (halogénoalkoxy en C₁-C₄)-(halogénoalkyle en C₁-C₄), alkylthio en C₁-C₄, (alkylthio en C₁-C₄)-(alkyle en C₁-C₄), alkylamino en C₁-C₄ ou di-(alkyl en C₁-C₄)-amino,
R³ représente un atome d'hydrogène, un atome d'halogène, un groupe alkyle en C₁-C₄, halogénoalkyle en C₁-C₄, alkoxy en C₁-C₄, halogénoalkoxy en C₁-C₄, alkylthio en C₁-C₄, amino, alkylamino en C₁-C₄ ou di-(alkyle en C₁-C₄)-amino ou
R² et R³ forment, ensemble avec les atomes de carbone auxquels ils sont liés, un noyau isocyclique insaturé à 5 ou 6 chaînons, qui peut également contenir un ou plusieurs, de préférence un ou deux atomes d'azote et qui, s'il s'agit d'un cycle à 5 chaînons, peut contenir à la place de CH₂ un atome d'oxygène ou un atome de soufre, et qui peut être éventuellement substitué par 1, 2 ou 3 résidus identiques ou différents et ces résidus représentent un groupe alkyle en C₁-C₄, alkoxy en C₁-C₄, halogénoalkyle en C₁-C₄, halogénoalkoxy en C₁-C₄ et/ou un atome d'halogène,
ou
R² et R³ forment, ensemble avec les atomes de carbone auxquels ils sont liés, un noyau isocyclique saturé à 5, 6 ou 7 chaînons, qui peut contenir à la place de CH₂ un ou plusieurs atomes d'oxygène ou de soufre, et qui est éventuellement substitué par 1 à 4 résidus identiques ou différents choisis entre un groupe alkyle en C₁-C₄ et un atome d'halogène,
X représente un atome d'oxygène, un groupe NH et S(O)_{q}, dans lequel q peut être 0, 1 ou 2,
R⁴ représente un atome d'halogène, un groupe alkyle en C₁-C₄, cycloalkyle en C₃-C₇, halogénoalkyle en C₁-C₄, alkoxy en C₁-C₄, halogénoalkoxy en C₁-C₄ ou phényle éventuellement substitué par un, deux ou trois substituants identiques ou différents choisis parmi un atome d'halogène, un groupe alkyle en C₁-C₄, alkoxy en C₁-C₄, halogénoalkyle en C₁-C₄ et halogénoalkoxy en C₁-C₄;
p peut être un nombre entier de 0 à 4,
n un nombre entier de 0 à 2 et
m un nombre entier de 1 à 3,
Y peut représenter 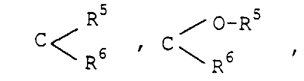 N - R⁵, N -O -R⁵, N - NR⁵R⁶ et 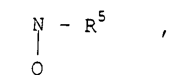
R⁵ représente un atome d'hydrogène, un atome d'halogène, un groupe alkyle en C₁ - C₁₂ , cycloalkyle en C₃-C₈, (cycloalkyl en C₃-C₈)-(alkyle en C₁-C₄), [alkoxy en C₁-C₄]ₜ-(alkyle en C₁-C₄), formule dans laquelle t peut être un nombre entier de 1 à 3, halogénoalkyle en C₁-C₁₂, 2- (tétrahydro-2H-pyran-2-yloxy) -alkyle en C₁-C₄, [halogénoalkoxy en C₁-C₄]ₜ-halogénoalkyle en C₁-C₄, [halogénoalkoxy en C₁-C₄]ₜ-alcényle en C₁-C₄, alkylcarbonyle en C₁-C₈, alkoxycarbonyle en C₁-C₈, (alkyl en C₁-C₈)carbonyl-(alkyle en C₁-C₄) , halogénoalkylcarbonyle en C₁-C₈, halogénoalkoxycarbonyle en C₁-C₈, (cycloalkyl en C₃-C₈)carbonyle (cycloalkoxy en C₃-C₈)carbonyle, benzoyle éventuellement substitué avec un, deux ou trois substituants identiques ou différents choisis parmi un atome d'halogène,'un groupe alkyle en C₁- C₄, alkoxy en C₁- C₄, représente un groupe benzyloxycarbonyle éventuellement substitué avec un, deux ou trois substituants identiques ou différents choisis parmi un atome d'halogène, un groupe alkyle en C₁ - C₄, alkoxy en C₁ - C₄, représente un groupe alcényle en C₂-C₈, alcynyle en C₂-C₈, tri(alkyl en C₁-C₈)silyle, de préférence di(méthylalkyl en C₁-C₈)silyle ou triéthylsilyle, di(alkyl en C₁-C₈)-(cycloalkyl en C₃-C₈)silyle, de préférence diméthylcyclohexylsilyle, di(alkyl en C₁-C₈)-(phénylalkyle en C₁-C₄)-silyle, de préférence diméthyl(phénylalkyl en C₁-C₄) silyle, di-(alkyl en C₁-C₈)-(halogénoalkyl en C₁-C₄)-silyle, diméthylphénylsilyle, hétéroaryle, phényle, phénylalkyle en C₂-C₄, benzyle, benzyl-oxyalkyle en C₁ - C₄, formules dans lesquelles les groupes phényle ou hétéroaryle dans les six derniers groupes cités peuvent être non substitués ou substitués une ou plusieurs fois, de préférence jusqu'à trois fois, et ces substituants sont identiques ou différents et représentent respectivement un atome d'halogène, un groupe alkyle en C₁-C₈, cycloalkyle en C₃-C₈, halogénoalkyle en C₁-C₄, (dialkyl en C₁-C₄)amino, (alkyl en C₁-C₄)thio, alkoxy en C₁-C₈, (alkoxy en C₁-C₈)carbonyle, alcanoyloxy en C₁-C₈, halogénoalkoxy en C₁-C₄, (alkoxy en C₁-C₄)-(alkoxy en C₁-C₄), [(alkyl en C₁-C₄)-O]ₜ-(alkyle en C₁-C₄), 2-(tétrahydro-2H-pyran-2-yloxy)-éthoxy, alcényle en C₂-C₈, alcynyle en C₂-C₈, phénoxy, benzyloxy, qui porte éventuellement dans le radical phényle un ou plusieurs, de préférence jusqu'à trois, substituants identiques ou différents choisis parmi un groupe alkyle en C₁-C₄, halogénoalkyle en C₁- C₄, alkoxy en C₁-C₄, halogénoalkoxy en C₁-C₄ et un atome d'halogène,
R⁶ représente un atome d'hydrogène, d'halogène, un groupe alkyle en C₁-C₈, cycloalkyle en C₃-C₈, halogénoalkyle en C₁-C₈, phényle et benzyle, dans lesquels les noyaux phényle peuvent être substitués comme décrit ci-dessus pour R⁵, ou
R⁵ et R⁶ peuvent former ensemble un cycle à 3 - 7 chaînons, qui peut être éventuellement substitué par un, deux ou trois résidus identiques ou différents choisis parmi un atome d'halogène, un groupe alkyle en C₁- C₄, alkoxy en C₁ - C₄, halogénoalkyle en C₁-C₄ et halogénoalkoxy en C₁-C₄ et dans lequel un atome de carbone peut être remplacé par O, S, NR⁷, R⁷ ayant la même signification que R⁴ , mais ne devant pas être un atome d'halogène.

2. Composés de formule I selon la revendication 1 ou leurs sels, dans lesquels
A a la signification donnée dans la revendication I,
R¹ est un atome d'hydrogène ou un groupe méthyle,
R² représente un atome d'halogène, un groupe alkyle en C₁- C₄, halogénoalkyle en C₁-C₄, alkoxy en C₁-C₄ ou alkoxy en C₁-C₄-alkyle en C₁-C₄,
R³ représente un atome d'hydrogène, d'halogène, un groupe alkyle en C₁-C₃, méthoxy ou éthoxy
ou
R² et R³ forment, ensemble avec les atomes de carbone auxquels ils sont liés, un cycle insaturé à 5 ou 6 chaînons, qui peut contenir un jusqu'à deux atomes d'azote, un atome d'oxygène ou de soufre, ou
R² et R³ forment, ensemble avec les atomes de carbone auxquels ils sont liés, un cycle saturé, éventuellement substitué, à 5 ou 6 chaînons, qui peut contenir un atome de soufre ou 1 à 2 atomes d'oxygène, et
R⁴, R⁵, R⁶ et
R⁷ ainsi que
X, Y, m, n, p ont les significations données à la revendication 1.

3. Composés de formule I selon la revendication 1 ou 2 ou leurs sels,
dans lesquels
A a la signification donnée à la revendication 1,
R¹ représente un atome d'hydrogène,
R² représente un groupe alkyle en C₁-C₄ ou méthoxyméthyle,
R³ représente un groupe méthyle, éthyle, méthoxy, un atome de chlore ou de brome, ou
R² et R³ forment, ensemble avec les atomes de carbone auxquels ils sont liés, le système quinoléine ou quinazoline, qui peut être substitué avec du fluor, du chlore, du brome ou un groupe méthyle ou
R² et R³ forment, ensemble avec les atomes de carbone auxquels ils sont liés, un cycle insaturé à 6 chaînons, qui contient un atome d'azote, ou
R² et R³ forment, ensemble avec les atomes de carbone auxquels ils sont liés, un cycle saturé à 6 chaînons, qui peut contenir un ou deux atomes d'oxygène,
m, n, p et X ont les significations données ci-dessus,
Y peut être 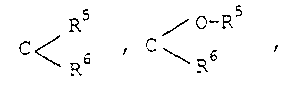 ou N - O -R⁵
et
R⁵, R⁶ et R⁷ ont également les significations définies à la revendication 1.

4. Composés de formule I ou leurs sels selon l'une des revendications 1 à 3, dans lesquels
A et R¹ à R⁷ ont les significations indiquées dans les revendications précédentes, et
m et n sont égaux à 2,
p est 0, et
X représente un groupe NH ou un atome d'oxygène.

5. Composés de formule I ou leurs sels selon l'une des revendications 1 à 4, dans lesquels
A a la signification donnée à la revendication 1,
R¹ représente un atome d'hydrogène,
R² représente un groupe alkyle en C₁-C₄ ou méthoxyméthyle,
R³ représente un atome de chlore, de brome ou un groupe méthoxy ou
R² et R³ forment, ensemble avec les atomes de carbone auxquels ils sont liés, le système quinoléine ou quinazoline, qui peut être substitué avec du fluor, du chlore, du brome ou un groupe méthyle ou
R² et R³ forment ensemble avec le cycle pyrimidine le système 5,6,7,8-tétrahydroquinazoline ou le système 5,6-dihydro-7H-pyrano[2,3-d]pyrimidine,
m et n représentent le nombre deux, et
p est égal à zéro,
X représente un groupe NH ou un atome d'oxygène,
Y représente 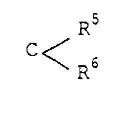 ou N - O - R⁵,
R⁵, R⁶ et R⁷ ont les significations données dans les revendications précédentes.

6. Composés de formule I ou leurs sels selon l'une des revendications 1 à 5, dans lesquels
A a la signification donnée à la revendication 1,
R¹ représente un atome d'hydrogène,
R² représente un groupe méthoxyméthyle et R³ un groupe méthoxy ou
R² représente un groupe alkyle en C₁-C₄ et R³ un atome de chlore ou de brome ou
R² et R³ forment, ensemble avec les atomes de carbone auxquels ils sont liés, un système quinoléine ou quinazoline, qui est substitué avec du fluor, du chlore ou un groupe méthyle ou un système 5,6,7,8-tétrahydroquinazoline ou le système 5,6-dihydro-7H-pyrano[2,3-d]pyrimidine,
m, n, p, X ainsi que Y ont les significations indiquées à la revendication 1,
R⁵ représente un groupe alkyle en C₂-C₈, cyclopentyle, cyclohexyle, alkylcarbonyle en C₁-C₈, alkoxycarbonyle en C₁-C₈, phényle, benzyle, benzoyle, les trois derniers groupes cités pouvant être non substitués ou substitués avec un ou deux substituants qui peuvent être identiques ou différents et qui représentent un atome de fluor, de chlore, de brome, un groupe alkyle en C₁-C₄, alkoxy en C₁- C₄, trifluorométhyle, halogénoalkoxy en C₁-C₂, cyclohexyle, 2-éthoxyéthoxy, méthylthio ou diméthylamino et
R⁶ peut être un atome d'hydrogène, d'halogène ou un groupe alkyle en C₁-C₃.

7. Procédé de préparation de composés de formule I selon l'une des revendications 1 à 6, caractérisé en ce que
a) l'on met à réagir un composé de formule Il 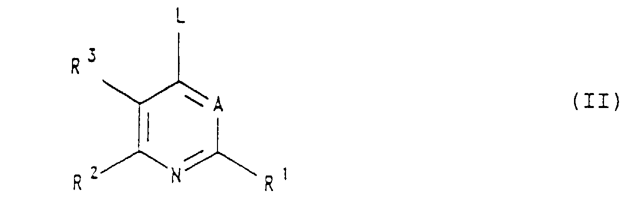 dans laquelle A, R¹, R² et R³ ont les significations données à la formule I, avec un composé de formule III 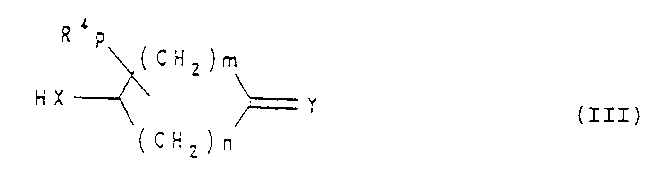 dans laquelle X, R⁴, n, m et Y ont les significations données à la formule I, ou
b) pour la préparation d'un composé de formule I, dans lequel Y représente 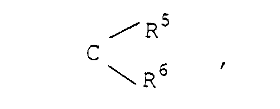 on met à réagir un composé de formule V 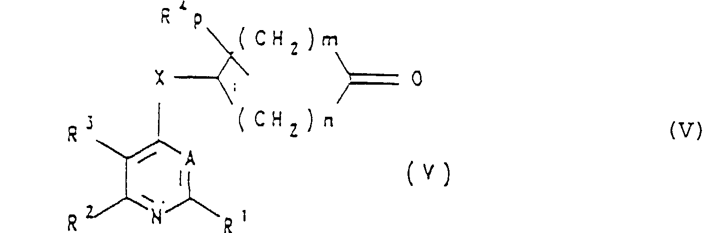 dans laquelle R¹, R², R³, R⁴, A, X, m, n et p ont les significations données à la formule I, en présence d'une base, avec un composé de formule P⁺Ph₃CHR⁵R⁶Z⁻, dans laquelle Ph représente un groupe aromatique et Z un anion et R⁵ et R⁶ sont comme définis dans la formule I, ou
c) pour la préparation d'un composé, dans lequel Y ne représente pas 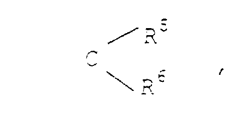 on met à réagir un composé de formule V définie en b) avec H₂NOH ou un composé de formule H-Q, dans laquelle Q représente HNR⁵, HN-OR⁵, HN-NR⁵R⁶ ou N(OH)R⁵ et où R⁵, R⁶ sont définis comme pour la formule I,
on met à réagir des composés de formule I obtenus selon a) ou c), dans lesquels Y représente N-OH, éventuellement en présence d'une base, avec un composé de formule L-R⁵, dans laquelle L représente un groupe partant et R⁵ est défini comme à la formule I, mais ne représente pas un atome d'hydrogène,
et on transforme éventuellement en leurs sels les composés obtenus.

8. Produit contenant au moins un composé selon l'une des revendications 1 à 6 et au moins un adjuvant de formulation.

9. Agent fongicide selon la revendication 8, contenant une quantité fongicide efficace d'au moins un composé selon l'une des revendications 1 à 6 en même temps que les additifs ou adjuvants usuels pour cette application.

10. Agent insecticide, acaricide ou nématocide selon la revendication 8, contenant une quantité efficace d'au moins un composé selon l'une des revendications 1 à 6 conjointement avec les additifs ou adjuvants usuels pour ces applications.

11. Agent phytosanitaire, contenant une quantité efficace fongicide, insecticide, acaricide ou nématocide au moins un composé selon l'une des revendications 1 à 6 et d'au moins une autre matière active, de préférence choisie parmi les fongicides, insecticides, appâts, stérilisants, acaricides, nématocides et herbicides conjointement avec les additifs ou adjuvants usuels pour cette application.

12. Agent à utiliser dans la protection du bois ou comme agent de conservation dans des matières d'étenchéification, dans des peintures, dans des agents réfrigérants lubrifiants pour l'usinage de métaux ou dans les huiles de perçage et de coupe, contenant une quantité efficace d'au moins un composé selon l'une des revendications 1 à 6 conjointement avec les additifs et adjuvants usuels pour ces applications.

13. Composé selon l'une des revendications 1 à 6 ou produit selon la revendication 8, à utiliser comme médicament vétérinaire, de préférence pour combattre les endo- ou les ectoparasites.

14. Procédé de préparation d'un produit selon l'une des revendications 8 à 13, caractérisé en ce que l'on groupe la matière active,et les autres additifs et en ce qu'on les met sous une forme d'utilisation appropriée.

15. Utilisation d'un composé selon l'une des revendications 1 à 6 ou d'un produit selon l'une des revendications 8, 9, 11 et 12 comme fongicide.

16. Utilisation d'un composé de formule I selon l'une des revendications 1 à 6 ou d'un produit selon l'une des revendications 8, 9 et 12 comme agent de protection du bois ou comme agent de conservation dans des matières d'étenchéification, dans des peintures, dans des agents réfrigérants lubrifiants pour l'usinage de métaux ou dans des huiles de perçage et de coupe.

17. Procédé pour combattre les champignons phytopathogènes, caractérisé en ce que, l'on applique sur ceux-ci ou sur les plantes, sur les surfaces ou les substrats ou sur les semences infectés par eux, une quantité fongicide efficace d'un composé selon l'une des revendications 1 à 6 ou d'un produit selon l'une des revendications 8, 9, 11 et 12.

18. Procédé pour combattre les insectes nuisibles, les acarides et les nématodes, dans lequel on applique sur ceux-ci ou sur les plantes, surfaces ou substrats infectés par eux, une quantité efficace d'un composé selon l'une des revendications 1 à 6 ou d'un produit selon l'une des revendications 8, 10 et 11.

19. Utilisation de composés selon l'une des revendications 1 à 6 ou d'un produit selon l'une des revendications 8, 10 et 11 pour combattre les insectes nuisibles, les acarides et les nématodes.

20. Semences, désinfectées ou revêtues avec une quantité efficace d'un composé de formule I selon l'une des revendications 1 à 6 ou d'un produit selon l'une des revendications 8 à 11.

21. Composés de formule V, 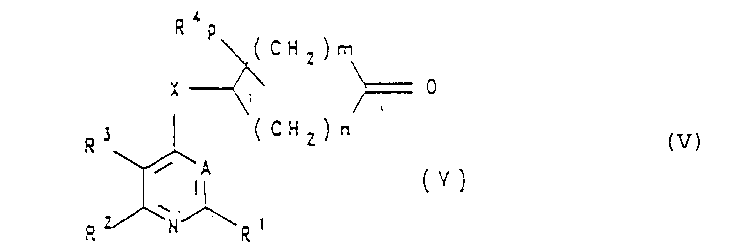 dans laquelle R¹, R², R³, R⁴, A, m, n et p sont définis comme à la revendication 1.

22. Composés de formule II 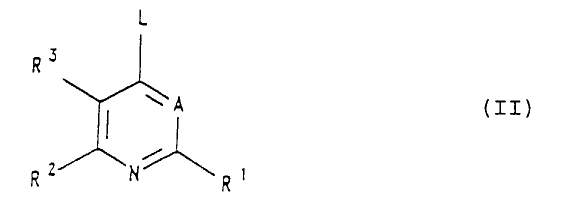 dans laquelle
R² et R³ forment, ensemble avec les atomes de carbone auxquels ils sont liés, un noyau isocyclique saturé à 5, 6 ou 7 chaînons, qui peut contenir à la place de CH₂ un ou plusieurs atomes d'oxygène ou de soufre, et qui est substitué éventuellement avec 1 à 4 résidus identiques ou différents choisis entre un groupe alkyle en C₁-C₄ et un atome d'halogène et
L est un groupe partant, et R¹ et A sont définis comme à la revendication 1.
